# EUROPEAN PATENT APPLICATION

(11) **EP 4 378 943 A1**
(43) Date of publication of application: **05.06.2024**
(21) Application number: 22848533.0
(22) Date of filing: 26.07.2022
(51) Int. Cl.: C07D 487/04, A61K 31/5377, A61P 35/00

(54) **8-OXO-3-AZABICYCLO[3.2.1]OCTANE COMPOUND OR SALT THEREOF, AND PREPARATION METHOD THEREFOR AND USE THEREOF**

(30) Priority: 27.07.2021 CN 202110860428; 01.07.2022 CN 202210779302
(71) Applicant: Littdd Medicines Ltd, Hangzhou, Zhejiang 310051 (CN)
(72) Inventor: CHENG, Yaobang, Shanghai 201203 (CN); HUANG, Yafei, Shanghai 201203 (CN); ZHOU, Juan, Shanghai 201203 (CN); WANG, Yonghui, Shanghai 201203 (CN); CHEN, Wyatt Wei, Shanghai 201203 (CN)
(74) Representative: Sagittarius IP
(86) International application number: PCT/CN2022/107932
(87) International publication number: WO 2023/005928

(57) **Abstract**

Provided are a group of 8-oxa-3-azabicyclo[3.2.1]octane compounds of formula (I) used as ATR inhibitors, and a preparation method thereof, a pharmaceutical compositions containing same, and the use thereof in the treatment or prevention of ATR-related diseases.

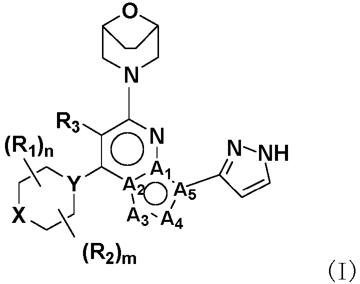

## Description

### Cross Reference

This application claims the priority to and the benefit of the Chinese patent application No. 202110860428.3 with the filing date on July 27, 2021, and the Chinese patent application No. 202210779302.8 with the filing date on July 1, 2022.

### Technical Field

This disclosure relates to the field of medicinal chemistry, in particular relates to a group of 8-oxa-3-azabicyclo[3.2.1]octane compounds useful as ATR inhibitors, preparation methods thereof, pharmaceutical compositions comprising them, and their uses for treating or preventing ATR-associated diseases.

### Background technique

Human cells are continuously exposed to DNA damage events due to environmental factors such as ultraviolet radiation, X-rays and endogenous factors such as reactive oxygen species. In cancer cells, driven by oncogene drivers or exogenously induced by DNA damaging drugs or Ionizing Radiation (IR), there is a relatively high level of DNA replication stress (a hallmark of cancer), with the sustained replication stress leading to DNA breakage, causing tumor cells to experience higher DNA damage. The DNA damage and replication stress constitute a main source of genomic instability.

DNA damage, if high enough and not repaired, will produce high toxicity to cells and lead to cell death. To ensure the integrity of the eukaryotic genome, a set of biological pathways collectively referred to as DDR (DNA Damage Response) have evolved to identify, signal and repair DNA damage. ATR (ataxia telangiectasia mutation and Rad3-related kinase), ATM (ataxia telangiectasia mutated kinase) and DNA-PK (DNA-dependent protein kinase) are key components of DDR that function to repair DNA damage by responding to diverse DNA damage, of which ATM and DNA-PK mainly respond to DNA double-strand breaks, and ATR mainly responds to replication stress.

ATR is a member of the phosphatidylinositol kinase associated kinase (PIKK) protein family, and its main target is CHK1. When ATR is activated by a variety of DNA damage, especially replication stress, ATR transmits DNA damage signals through CHK1 phosphorylation, arresting the cell cycle in S phase or G2/M, repairing the damage, relieving replication stress of cells, and thus restarting replication after the stress is removed.

Compared with healthy proliferating cells, tumor cells have higher DNA damage and replication stress, and are more dependent on DNA repair to replicate and survive, and to maintain cell division. Therefore, the repair function of tumor cells can be inhibited through inhibiting ATR, resulting in the increased DNA damage and replication stress as can not be repaired, and ultimately leading to tumor cell death, meanwhile not or less affecting healthy proliferating cells, which constitutes the basis for ATR inhibitors to be used in cancer treatment, and also makes ATR inhibition has been considered as an important approach to cancer treatment in recent years.

Standard cancer therapies such as radiatherapy or chemotherapy exert therapeutic effects by inducing DNA damage, which is particularly toxic to proliferating cells, and the presence of DNA damage repair mechanisms limits the efficacy of these therapies, resulting in resistance to chemotherapy or radiotherapy drugs. By inhibiting ATR, increasing replication stress and increasing DNA damage, the sensitivity of tumor cells to these DNA damage-induced therapies can be enhanced, helping overcome the drug resistance of radiotherapy or chemotherapy drugs due to damage repair, and ATR inhibition can be used for treating tumor patients with genemutations or chemotherapy resistance, and to reduce doses of chemotherapy or radiotherapy, thereby reducing the toxicity to blood and gastrointestinal organ systems.

Therefore, for cancer cells with increased replication stress or other cancer cells with impaired or defective activity of DNA damage repair pathway, ATR inhibitors can be used to increase replication stress and induce death of tumor cells. Indeed, studies have revealed the synthetic lethality of ATR inhibitors against p53-mutated tumors or tumors that have lost ATM function, and are synergistic when combined with a variety of replication stress/DNA damage-inducing chemotherapeutic agents, such as platinum, ionizing radiation and PARP inhibitors.

In addition, ATR inhibition can also prevent the occurrence of cancer, because ATR is also an important member of DNA damage checkpoints, and ATR inhibition will limit the amplification of protocarcinoma cells cells caused by oncogene activation.

In recent years, some ATR inhibitors have been developed (such as WO2017202748, CN111848605A, WO2020087170, WO2020049017). However, it is still very challenging to develop new potent ATR inhibitors. The high degree of identity of PIKK kinase family with related lipid kinases such as phosphatidylinositol 3-kinase (PI3KS) and mTOR increases the risk of inhibiting other kinases, which may increase toxicity or offset the therapeutic effects of ATR inhibition; in addition, the application of some ATR inhibitors is also limited by physicalchemical properties, pharmacokinetic properties and drug-drug interactions.

Therefore, there is still a need in the art for new selective ATR inhibitors with enhanced ATR inhibitory activity, especially with improved physicochemical properties, improved metabolic stability, improved pharmacokinetic properties (oral available), and/or minimized CYP450 inhibition.

### Brief Description of the Invention

The present inventors have identified through research that the compounds of the present disclosure exhibit satisfactory ATR inhibitory activity, and show good performance in in vivo and/or in vitro pharmacokinetic experiments, indicating improved druggability and improved bioavailability. Therefore, the compounds of the present disclosure can not only achieve the purpose of preventing or treating ATR-associated diseases, but also the prepared medicine is expected to have improved absorption, improved therapeutic efficacy at the same dose, or provide the same therapeutic efficacy at a lower dose and/or reduce possible side effects. Thus, the present disclosure also provides the use of the compounds of the present disclosure in the preparation of a medicament for the prevention or treatment of ATR-associated diseases, pharmaceutical compositions comprising the compounds, and methods of preventing and/or treating ATR-associated diseases by administering the compounds.

Accordingly, in one aspect of the present disclosure, provided herein is a compound of formula (I), a stereoisomer, a tautomer, a stable isotopic variant, a pharmaceutically acceptable salt or a solvate thereof: 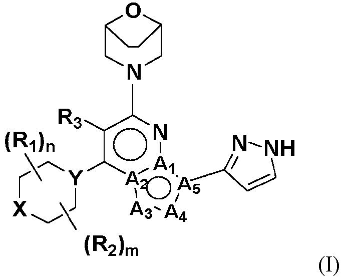 wherein the definition of each group is as described in the detailed description of the invention.

In another aspect of the present disclosure, provided herein is a compound of formula (I), a stereoisomer, a tautomer, a stable isotopic variant, a pharmaceutically acceptable salt or a solvate thereof having ATR inhibitory activity, for use as a medicament, especially for use as an ATR inhibitor for the prevention and/or treatment of ATR-associated diseases.

In another aspect of the present disclosure, provided herein is a pharmaceutical composition comprising a compound of the present disclosure and a pharmaceutically acceptable excipient. In a specific aspect, the pharmaceutical composition can further comprise other therapeutically active ingredient(s) suitable for use in combination with the compound of the present disclosure. In a specific aspect, provided herein is a pharmaceutical combination product, such as a kit, comprising a compound of the present disclosure and a further active agent.

In another aspect of the present disclosure, provided herein is use of a compound of the present disclosure or a pharmaceutical composition comprising the same for the prevention or the treatment of ATR-associated diseases in mammals, especially in humans.

In another aspect of the present disclosure, provided herein is a method of inhibiting ATR in vivo or in vitro, the method comprising contacting said ATR with an effective amount of a compound of the present disclosure.

In another aspect of the present disclosure, provided herein is a method of preventing or treating ATR-associated diseases in an indivual, such as a mammal, particularly in a human, comprising administering an effective amount of a compound of the present disclosure or a pharmaceutical composition comprising the same as disclosed herein.

In another aspect of the present disclosure, provided herein is use of a compound of the present disclosure or a pharmaceutical composition comprising the same as described above in the manufacture of a medicament for the prevention or treatment of ATR-associated diseases.

In additional aspects, provided herein is a method for synthesizing the compound of the present disclosure, wherein representative synthetic schemes and routes are described hereinafter.

Other objects and advantages of the present disclosure would be obvious to those skilled in the art upon reading the subsequent detailed descriptions.

### DETAILED DESCRIPTION OF THE INVENTION

### Definitions

Unless specified otherwise, the terms used in the description and claims herein have the following meanings. Specific terms or phrases without specified definitions, should not be recognised as indefinite or unclear, but should be understood in accordance with the ordinary meaning in the art and in combination with the context herein.

Many groups defined herein are optionally substituted, and the list of substituents provided in this definition section is only illustrative, not exhaustive, and is not intended to limit substituents defined elsewhere in this description and claims.

The term "treatment" or "treating" as used herein refers to administering one or more compounds of the present disclosure as described herein to a subject, e.g, a mammal, e.g, a human, suffering from, or having symptoms of the disease, for curing, relieving, alleviating or affecting the disease or symptoms of the disease. In specific embodiments of the present disclosure, the disease is an ATR-associated disease as defined hereinafter, especially a tumor or a cancer.

The term "prevention" or "preventing" as used herein is well known in the art, and refers to administering one or more compounds of the present disclosure to a subject, e.g, a mammal, e.g, a human, suspected of suffering from or being susceptible to an ATR-associated disease as definded herein, especially a cancer or a tumor, so as to reduce the risk of suffering from the defined diseases. The term "prevention" or "preventing" comprises the use of a compound of the present disclosure before any clinical and/or pathological symptoms are diagnosed or determined.

The terms "inhibit" and "reduce" or any variant of these terms, as used herein, refer to the ability of a biologically active agent to reduce the signaling activity of a target of interest through interacting directly or indirectly with the target, and refer to any measurable reduction or complete inhibition of the activity of the target. For example, it may be an activity (e.g., ATR activity) reduction of about or at least about 5%, 10%, 15%, 20%, 25%, 30%, 35%, 40%, 45%, 50% %, 55%, 60%, 65%, 70%, 75%, 80%, 85%, 90%, 95%, 99% or more, or any range derivable therein, compared to normal conditions.

The term "selective inhibition" as used herein refers to the ability of a biologically active agent to preferentially reduce signaling activity of a target of interest over off-target signaling activity by interacting directly or indirectly with the target. In terms of the compound of the present disclosure, it can selectively inhibit the activity of ATR, over other kinase such as PIKK kinase family and associated lipid kinase e.g. phosphatidylinositol 3-kinase (PI3KS) and mTOR which have high identity, thereby reducing the toxicity or offset of the inhibitory effect of ATR caused by simultaneously acting on other kinases. For example, as compared to that for another specific kinase, the present disclosure has at least 5%, 10%, 15%, 20%, 25%, 30%, 35%, 40%, 45%, 50%, 55%, 60%, 65%, 70%, 75%, 80%, 85%, 90%, 95%, 99% or more, or any derivable range of better inhibition activity, or has at least 1-, 2-, 3-, 4-, 5-, 10-, 25-, 50-, 100-, 250-, or 500-fold better activity for ATR as compared to that for another specific kinase.

The term "cancer" or "tumor" as used herein refers to neoplastic cell growth and proliferation, either malignant or benign, and all precancerous cells and cancerous cells and tissues. For the compounds, methods, pharmaceutical compositions, pharmaceutical combinations and uses of the present disclosure, the cancer or tumor includes but is not limited to colon cancer, pancreatic cancer, breast cancer, ovarian cancer, prostate cancer, squamous cell carcinoma, basal cell carcinoma, adenocarcinoma, sweat gland cancer, sebaceous gland cancer, lung cancer, leukemia, bladder cancer, stomach cancer, cervical cancer, testicular cancer, skin cancer, rectal cancer, thyroid cancer, kidney cancer, uterine cancer, pemphigus cancer, liver cancer, acoustic neuroma, oligodendroglioma, (spinal) meningioma, neuroblastoma, eye cancer.

Accordingly, the "anti-cancer effect" or "anti-tumor effect" as described herein includes, but is not limited to, effects on response rate, time to disease progression, and survival rate. The anti-tumor effects of the compounds of the present disclosure and the medical uses and methods thereof include, but are not limited to, inhibiting tumor growth, delaying tumor growth, regressing tumors, shrinking tumors, prolonging tumor regrowth after cessation of treatment, slowing down disease progression, and also include preventing tumorigenesis.

The term "therapeutically effective amount" as used herein refers to an amount that, when administered to an individual to treat a disease, is sufficient to reduce or completely alleviate symptoms or other deleterious effects of a disorder; reverse, completely halt or slow the progression of the disorder; or reduce the risk of exacerbation of the disorder. The "effective amount" will vary depending on the compound, the disease and its severity, and the age, weight, etc., of the individual to be treated.

The term "subject" or "individual" as used herein includes a human or non-human animal. Exemplary human individuals include human individuals with a disease (referred to as patients) (e.g., a disease described herein) or normal individuals. The "non-human animals" in the present disclosure include all vertebrates such as non-mammals (e.g., birds, amphibians, reptiles) and mammals such as non-human primates, livestock and/or domesticated animals (e.g., sheep, dogs, cats, cows, pigs, etc.).

The term "ATR-associated disease" or "ATR-related disease" as used herein refers to a disease in which ATR activity contributes to the occurrence and progression of the disease, or a disease in which inhibition of ATR will reduce the incidence, reduce or eliminate disease symptoms. In respect to the present disclosure, "ATR-associated disease" preferably refers to an ATR-mediated disease, more preferably a cancer or tumor. As described herein, ATR kinase inhibitors should be of therapeutic or prophylactic value for the diseases such as: hematological malignancies, e.g. leukemia (including chronic lymphocytic leukemia, acute lymphocytic leukemia, acute myelogenous leukemia, and chronic myelogenous leukemia), multiple myeloma, lymphoid malignancies (e.g. lymphoma, Hodgkin's disease, non-Hodgkin's lymphoma), myelodysplastic syndromes, and solid tumors such as carcinomas and sarcomas and their metastases, e.g. breast cancer, lung cancer (non-small cell lung cancer, small cell lung cancer, squamous cell carcinoma, bronchioloalveolar carcinoma), central nervous system tumors (e.g. glioma, dysembryonic dysplastic neuroepithelial tumor, glioblastoma multiforme, mixed Glioma, medulloblastoma, retinoblastoma, neuroblastoma, germ cell tumor and teratoma), gastrointestinal cancers (e.g. gastric cancer, esophageal cancer, liver cancer, bile duct cancer, colorectal cancer, carcinoma of small intestine, pancreatic cancer), skin cancer, melanoma, thyroid cancer, bone cancer, head and neck cancer, salivary gland cancer, prostate cancer, testicular cancer, ovarian cancer, cervical cancer, uterine cancer, endometrial cancer, vulvar cancer, bladder cancer, renal cancer, squamous cell carcinoma, sarcomas (e.g. osteosarcoma, chondrosarcoma, leiomyosarcoma, soft tissue sarcoma, Ewing's sarcoma, gastrointestinal tissue carcinoma, gastrointestinal stromal tumor, Kaposi's sarcoma), and pediatric cancers (e.g. rhabdomyosarcoma and neuroblastoma).

The compounds of the present disclosure are especially useful for the treatment of patients with lung cancer, prostate cancer, melanoma, ovarian cancer, breast cancer, endometrial cancer, renal cancer, gastric cancer, sarcoma, head and neck cancer, central nervous system tumors and their metastases, and patients with acute myeloid leukemia.

The term "pharmaceutical composition" or "pharmaceutical preparation" as used herein refers to a composition comprising one or more compounds of formula (I) of the present disclosure or a stereoisomer, tautomer, stable isotope derivative, pharmaceutically acceptable salt or solvate thereof and a pharmaceutically acceptable excipient or carrier generally accepted in the art, and may be in solid, semi-solid, liquid or gaseous form.

The term "pharmaceutical combination" as used herein refers to that a compound of the present disclosure may be used in combination with other active agents for purposes of the present disclosure. The other active agent may be one or more additional compounds of the present disclosure, or may be a second or additional (e.g, the third) compound which is compatible with the compound of the present disclosure, i.e., does not adversely affect each other, or has complementary activities to the compound of the present disclosure. Such active agents are suitably present in combination in amounts that are effective for the intended purpose. The other active agents may be co-administered together with the compound of the present disclosure in a unitary pharmaceutical composition, or administered separately in separate discrete units, e.g., in the form of a kit, which when administered separately may be simultaneous or sequential. Such sequential administration may be close or remote in time.

The term "pharmaceutically acceptable" as used herein refers to such molecular entities and compositions that have been approved by regulatory agencies in various countries or could be approved by the same, or listed in a generally recognized pharmacopeia for use in animals, and more particularly in humans, or when administered to animals such as humans in appropriate amounts do not produce disadvangeous, allergic or other adverse reactions.

The term "pharmaceutically acceptable excipient or carrier" as used herein refers to one or more compatible solid or liquid filler or gelling substances, which are pharmacologically inactive, compatible with the other ingredients in the composition and should be acceptable for administration to warm-blooded animals, such as humans, for use as a carrier or vehicle for the compounds of the present disclosure in administration forms, examples of which include, but are not limited to, cellulose and its derivatives (e.g., carboxymethyl cellulose sodium, cellulose acetate, etc.), gelatin, talc, solid lubricants (e.g., magnesium stearate), calcium sulfate, vegetable oils, polyols (e.g., propylene glycol, glycerol, mannitol, sorbitol, etc.), emulsifiers (e.g., Tweens), wetting agents (e.g., sodium lauryl sulfate), colorants, flavors, stabilizers, antioxidants, preservatives, etc.

The term "pharmaceutically acceptable salt" as used herein refers to a salt of a compound of the present disclosure herein which is pharmaceutically acceptable and which possesses the desired pharmacological activities of the parent compound. Specifically, such salts are non-toxic and can be inorganic acid addition salts or organic acid addition salts or base addition salts. Specifically, the salts include: (1) acid addition salts formed with inorganic acids such as hydrochloric acid, hydrobromic acid, sulfuric acid, nitric acid, phosphoric acid, etc.; or acid addition salts formed with organic acids, such as acetic acid, propionic acid, caproic acid, glycolic acid, pyruvic acid, lactic acid, malonic acid, succinic acid, malic acid, maleic acid, fumaric acid, tartaric acid, citric acid, benzoic acid, cinnamic acid, mandel acid, methanesulfonic acid, ethanesulfonic acid, benzenesulfonic acid, 2-naphthalenesulfonic acid, 4-toluenesulfonic acid, camphorsulfonic acid, glucoheptanoic acid, 3-phenylpropionic acid, trimethylacetic acid, tert-butylacetic acid, lauryl sulfuric acid, gluconic acid, glutamic acid, hydroxynaphthoic acid, salicylic acid, stearic acid, muconic acid, etc.; or (2) salts formed when the acidic proton present in the parent compound is either replaced by a metal ion such as an alkali metal ion, an alkaline earth metal ion or an aluminum ion, or coordinates with an organic base such as ethanolamine, diethanolamine, triethanolamine, *N*-methylglucamine, etc. Those skilled in the art understand the general principles and techniques for preparing pharmaceutically acceptable salts, for example those described in Berge et al., Pharm ScL, 66, 1-19. (1977).

The term "stereoisomer" as used herein refers to an isomer formed from at least one asymmetric center. In compounds having one or more (e.g., 1, 2, 3 or 4) asymmetric centers, it can give rise to racemic mixtures, single enantiomers, diastereomeric mixtures and individual diastereomers. Certain molecules may also exist as geometric isomers (cis/trans). The compounds of the disclosure may exist as mixtures of two or more different structural forms in rapid equilibrium (often referred to as tautomers). Representative examples of tautomers include keto-enol tautomers, phenol-ketone tautomers, nitroso-oxime tautomers etc. It is to be understood that the scope of this disclosure covers all such isomers or mixtures thereof in any ratio, e.g., 60%, 65%, 70%, 75%, 80%, 85%, 90%, 95%, 96%, 97%, 98%, 99%.

The term "solvate" as used herein refers to a solvent addition form comprising a stoichiometric or non-stoichiometric amount of a solvent, including any solvated form of the compounds of the present disclosure, including, for example, solvates with water, such as hydrates, or solvates with organic solvents, e.g. methanol, ethanol or acetonitrile, i.e. as methanolate, ethanolate or acetonitrile, respectively; or in the form of any polymorph. It should be understood that such solvates of the compounds of the present disclosure also include solvates of pharmaceutically acceptable salts of the compounds of the present disclosure.

The term "prodrug" as used herein refers to a compound that has a cleavable group and can be convereted by solvolysis or under physiological conditions to a compound of the present disclosure which is pharmaceutically active in vivo. Prodrugs include acid derivatives well known in the art, such as esters prepared by reacting the parent acid compound with a suitable alcohol, or amides prepared by reacting the parent acid compound with a substituted or unsubstituted amine, or anhydrides or mixed anhydrides. Simple aliphatic or aromatic esters, amides, and anhydrides derived from the pendant acid groups of the compounds of the disclosure are particularly suitable prodrugs. Particular such prodrugs are C₁₋₈ alkyl, C₂₋₈ alkenyl, optionally substituted C₆₋₁₀ aryl and (C₆₋₁₀ aryl)-(C₁₋₄ alkyl) esters of the compounds described herein.

The term "isotopic variant" as used herein refers to a compound containing unnatural proportions of isotopes at one or more atoms constituting the compound. The compounds of the present disclosure may contain unnatural proportions of atomic isotopes at one or more atoms constituting the compounds, thereby forming isotopic variants of the compounds which whether radioactive or not are intended to be encompassed within the scope of the present disclosure. Examples of isotopes that can be incorporated into the compounds of the disclosure and pharmaceutically acceptable salts thereof, include, but are not limited to, isotopes of hydrogen (e.g. 2H, 3H); isotopes of carbon (e.g. 11C, 13C, and 14C); isotopes of chlorine (e.g. 36Cl); isotopes of fluorine (e.g. 18F); isotopes of iodine (e.g. 1231 and 1251); isotopes of nitrogen (e.g. 13N and 15N); isotopes of oxygen (e.g. 150, 170 and 180); isotopes of phosphorus (e.g. 32P); and isotopes of sulfur (e.g. 35S). It should be understood that isotopic variants of the compounds of the present disclosure may generally be prepared by conventional methods using appropriate isotopic variation of suitable reagents.

The 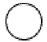 as used herein in the structure of compounds of general formula (I) of the present disclosure indicates an aromatic ring, i.e. the A₁ to A₅ are selected such that the rings formed satisfy the valence-bond theory of aromatic ring, and are feasible and stable in chemistry.

The " 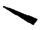 " or " 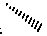 " as used herein in structures of compounds or in structural fragments indicates the existence of stereoisomers, and indicates the absolute configuration of the asymmetric center, which is usually represented by R or S in the naming of the compounds or intermediates provided in the present disclosure. When present in a racemic mixture, the solid and broken wedge symbols define relative stereochemistry rather than absolute stereochemistry.

The " 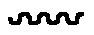 " as used herein in a structural fragment indicates that the bond it crosses is the bond that connects the structural fragment to the rest of a molecule.

The term "halo" or "halogen" as used herein refers to fluorine (F), chlorine (Cl), bromine (Br) or iodine (I). The prefered halogen is fluorine or chlorine.

The term "halogen-substituted" or "substituted by halogen" in groups as used herein is intended to include monohalogenated or polyhalogenated groups, wherein one or more (e.g. 2, 3, 4, 5 or 6) identical or different halogen substituents one or more (e.g. 2, 3, 4, 5 or 6) hydrogens in a group.

The term "cyano" as used herein refers to a group -CN.

The term "hydroxyl" as used herein refers to -OH.

The term "oxo" as used herein refers to =O.

The term "alkyl" as used herein refers to a straight or branched chain saturated hydrocarbon group consisting of carbon atoms and hydrogen atoms. Specifically, the alkyl group has 1-10, such as 1 to 6, 1 to 4, 1 to 3, or 1 to 2 carbon atoms. For example, the term "C₁-C₆ alkyl" as used herein refers to a straight or branched chain saturated hydrocarbon group having 1 to 6 carbon atoms, examples are methyl, ethyl, propyl (including n-propyl and isopropyl), butyl (including n-butyl, isobutyl, sec-butyl or tert-butyl), pentyl (including n-pentyl, isopentyl, neopentyl), n-hexyl, 2-methylpentyl, etc.. Particular alkyls have 1 to 3 carbon atoms.

The term "alkoxy" as used herein refers to a -O-alkyl group, wherein the alkyl has the meaning as defined herein. Specifically, this term refers to -O-C₁₋₆ alkyl group, more specifically -O-C₁₋₃ alkyl. Representative examples of suitable alkoxy include, but are not limited to, methoxy, ethoxy, propoxy (including n-propoxy, isopropoxy), butoxy (including n-butoxy, isobutoxy, tert-butoxy), pentyloxy (including n-pentyloxy, isopentyloxy, neopentyloxy), hexyloxy (including n-hexyloxy, isohexyloxy), etc. Particular alkoxys have 1 to 3 carbon atoms.

The term "alkylene" used herein alone or in combination with other groups refers to a saturated straight-chain or branched-chain divalent hydrocarbon group. For example, the term "C₁₋₃ alkylene" refers to an alkylene group having 1 to 3 carbon atoms, such as methylene, ethylene, propylene, 1-methylethylene, 2-methylethylene ethyl, etc.

The term "cycloalkyl" as used herein refers to a monocyclic, fused polycyclic, bridged polycyclic, or spirocyclic non-aromatic saturated cyclic hydrocarbon ring structure having specified number of ring atoms. The cycloalkyl may have 3 to 12 carbon atoms (i.e. C₃-C₁₂cycloalkyl), such as C₃₋₁₀cycloalkyl, C₃₋₈cycloalkyl, C₃₋₆cycloalkyl, C₅₋₆cycloalkyl. Suitable examples of cycloalkyl include, but are not limited to, monocyclic structure, e.g., cyclopropyl, cyclobutyl, cyclopentyl, cyclohexyl, cycloheptyl or cyclooctyl; or polycyclic (such as bicyclic) structure, including spiro, fused or bridged systems, such as bicyclo[1.1.1]pentyl, bicyclo[2.2.1]heptyl, spiro[3.4]octyl, bicyclo[3.1.1]hexyl, bicyclo[3.1.1] heptyl or bicyclo[3.2.1] octyl,etc..

The term "aryl" as used herein refers to a monovalent aromatic hydrocarbon group derived by removing one hydrogen atom from a single carbon atom in an aromatic ring system. Specifically, an aryl refers to a monocyclic or fused polycyclic aromatic ring structure having specified number of ring atoms. Specifically, the term includes groups containing 6 to 14, e.g. 6 to 10, preferably 6 ring members. Particular aryl groups include phenyl and naphthyl, most particularly phenyl.

The term "substitute" or "substituted" as used herein refers to that one or more (e.g, 1, 2, 3, or 4) hydrogens on the designated atom are replaced by the designated group, provided that it does not exceed normal valences of the designated atom under the current situation and form stable compounds, and combinations of substituents and variables are permissible only when such combinations form stable compounds.

The term "optionally substituted" as used herein, unless otherwise indicated, means that a group may be unsubstituted or substituted by one or more (e.g 0, 1, 2, 3, 4 or 5 or more) substituents as listed for the group, wherein the substituents may be the same or different.

Unless otherwise specified, Cₙ₋ₙ₊ₘ or Cₙ-Cₙ₊ₘ in the definitions of the compounds of the present disclosure includes each case of n to n+m carbon atoms, e.g., C₁₋₆ includes C₁, C₂, C₃, C₄, C₅ and C₆, also includes any range of n to n+m, e.g., C₀₋₆ includes C₁, C₂, C₃, C₄, C₅, C₆, C₀₋₁, C₀₋₂, C₀₋₃, C₀₋₄, C₀₋₅, C₁₋₂, C₁₋₃, C₁₋₄, C₂₋₃, etc. Similarly, n membered to n+m membered in the definitions of the compounds of the present disclosure indicates that the number of ring atoms can be any one between n to n+m, also includes any range of n to n+m membered.

As used in this description and the following claims, the words "comprising", "including" and "containing" mean "including but not limited to", and do not exclude, for example, other additives, ingredients, integers or steps. It should be understood that these terms include technical solutions that "consist of the components, steps or conditions" or "consist essentially of the components, steps or conditions".

It is to be understood that the dosages referred to when describing the compounds of the present disclosure, pharmaceutical compositions, pharmaceutical combinations comprising the same, and related uses and methods, are based on the weight of the free form but not on any salt, hydrate or solvate thereof etc., unless otherwise defined in the description.

### Compounds of the Present Disclosure

The terms "inventive/disclosed compound(s)" and "compound(s) of the present invention/disclosure" and the like as used throughout the present description, unless otherwise indicated, emcompass compounds of formula (I) and stereoisomers, tautomers, stable isotopic variants, pharmaceutically acceptable salts or solvates and prodrugs thereof as definded in each embodiment and specific or preferable embodiments thereof as descibed herein. The stereoisomers, tautomers, stable isotopic variants, pharmaceutically acceptable salts or solvates and prodrugs are as described in the definitions section above. Preferably, a compound of the present disclosure is a compound of formula (I) in free form or a pharmaceutically acceptable salt or solvate thereof; most preferably is a compound of formula (I) in free form or a pharmaceutically acceptable salt thereof.

Certain compounds of the present disclosure may exist in polymorphic or amorphous forms, which also fall within the scope of the present disclosure. When in solid crystalline form, the compounds of formula (I) may be in the form of a co-crystal with another chemical entity, and this description includes all such co-crystals.

When a chiral center is present, the compound of the present disclosure may exist as individual enantiomers or as a mixture of enantiomers, and those skilled in the art will be able to determine stable and feasible isomeric forms of the compounds of the present disclosure. According to one embodiment, there is provided a compound of formula (I), or a pharmaceutically acceptable salt thereof, which is a single enantiomer with an enantiomeric excess (%ee) of >95, >98%, or >99%. Preferably, a single enantiomer is present with >99% enantiomeric excess (%ee).

The compounds of the present disclosure also encompass possible N-oxides, and those skilled in the art will be able to determine stable and feasible N-oxides of the compounds of the present disclosure. The compounds of the present disclosure also encompass metabolites of the compounds of the present disclosure, that is, substances formed in vivo by oxidation, reduction, hydrolysis, amidation, esterification, etc. when the compounds of the present disclosure are administered, which can be identified by techniques known in the art.

Specifically, in one aspect, the present disclosure provides a compound of formula (I), a stereoisomer, a tautomer, a stable isotopic variant, a pharmaceutically acceptable salt or a solvate thereof: 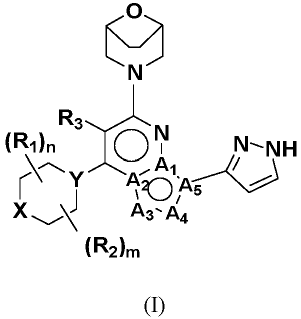 wherein
A₁, A₂ and A₅ are each independently C or N;
A₃ and A₄ are each independently CR₄, N or NR₅;
X is O, C(R₆)₂ or NR₇;
Y is N or CR₈;
R₁, R₂ and R₃ are each independently H, -OH, oxo, halogen, CN, -C₁₋₆alkyl or -O-C₁₋₆alkyl, wherein the -C₁₋₆alkyl is optionally substituted with one or more halogen or hydroxyl; or R₁ and R₂ are linked together to form a C₁₋₃ alkylene bridge;
R₄ is H, oxo, halogen or -C₁₋₆alkyl, wherein the -C₁₋₆alkyl is optionally substituted with one or more halogen or hydroxyl;
R₅ is H or -C₁₋₆alkyl, wherein the -C₁₋₆alkyl is optionally substituted with one or more halogen;
Each R₆ is independently H, halogen, CN, -OH, -NH₂, -NH-C₁₋₆alkyl, -N(C₁₋₆alkyl)₂, -C₁₋₆alkyl, -O-C₁₋₆alkyl, -C(O)-C₁₋₆alkyl, -C(O)-C₃₋₆cycloalkyl, -SO₂-C₁₋₆alkyl, -SO₂-C₃₋₆cycloalkyl, -SO-C₁₋₆alkyl, -SO-C₃₋₆cycloalkyl, -C₆₋₁₀aryl or -C₃₋₆cycloalkyl, wherein the -C₁₋₆alkyl, the -C₆₋₁₀aryl or the -C₃₋₆cycloalkyl is optionally substituted with one or more halogen, hydroxyl, -O-C₁₋₆alkyl, -C₁₋₆alkyl, or -C₁₋₆alkyl substituted with hydroxyl or halogen;
R₇ is H, -C₁₋₆alkyl, -C(O)-C₁₋₆alkyl, -C(O)-C₃₋₆cycloalkyl, -SO₂-C₁₋₆alkyl, -SO₂-C₃₋₆cycloalkyl, -SO-C₁₋₆alkyl or -SO-C₃₋₆cycloalkyl, wherein the -C₁₋₆alkyl or the -C₃₋₆cycloalkyl is optionally substituted with one or more halogen, hydroxyl, -O-C₁₋₆alkyl, -C₁₋₆alkyl, or -C₁₋₆alkyl substituted with halogen or hydroxyl;
R₈ is H, -OH or halogen;
n and m are each independently an integer of 0 to 4.

In one embodiment of the compound of formula (I), at least two of A₁, A₂, A₃, A₄ and A₅ are N or NR₅, and the others are C or CR₄; preferably two of them are N or NR₅, and the others are C or CR₄.

In one embodiment of the compound of formula (I), the heteroaryl moiety containing A₁~A₅ and consisting of a six membered ring fused to a five membered ring has a structure selected from: 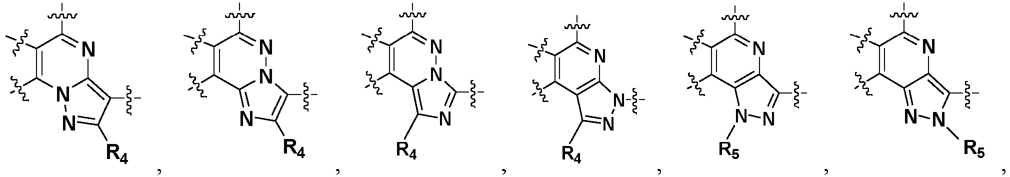 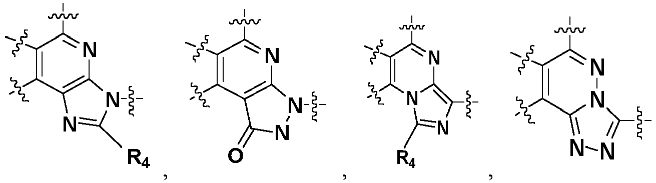 and

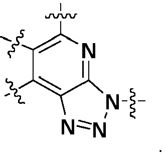

In one embodiment of the compound of formula (I), the heteroaryl moiety containing A₁~A₅ and consisting of a six membered ring fused to a five membered ring is selected from: 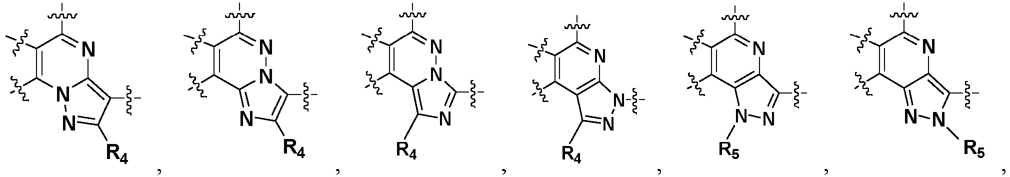 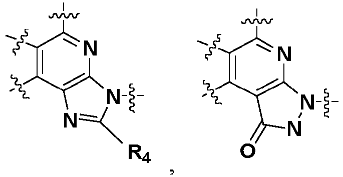 and 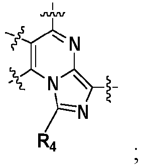 preferably selected from 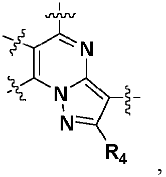 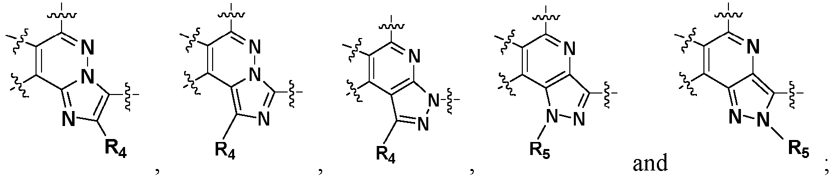 more preferably

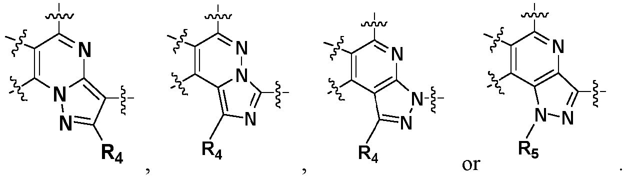

In one embodiment of the compound of formula (I), the six membered confused five membered heteroaryl moiety containing A1-A5 preferably is

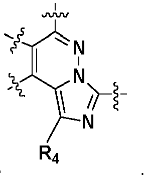

In one embodiment of the compound of formula (I), the heteroaryl moiety containing A₁~A₅ and consisting of a six membered ring fused to a five membered ring preferably is

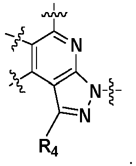

In one embodiment of the compound of formula (I), the heteroaryl moiety containing A₁~A₅ and consisting of a six membered ring fused to a five membered ring preferably is

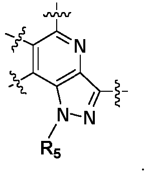

In one embodiment of the compound of formula (I), the heteroaryl moiety containing A₁~A₅ and consisting of a six membered ring fused to a five membered ring preferably is

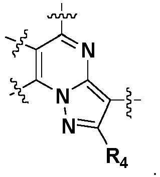

In one embodiment of the compound of formula (I), R₄ is H.

In one embodiment of the compound of formula (I), R₄ is oxo or halogen.

In one embodiment of the compound of formula (I), R₄ is -C₁₋₆alkyl, optionally substituted with one or more halogen or hydroxyl; such as, but is not limited to methyl, ethyl, propyl (including n-propyl and isopropyl), butyl (including n-butyl, isobutyl, sec-butyl or tert-butyl), pentyl (including n-pentyl, isopentyl, neopentyl), n-hexyl, 2- methylpentyl, -CH₂F, -CHF₂, -CF₃, -C₂F₅, -CH₂CF₃, -CH₂Cl, -CH₂CH₂CF₃, -CH(CF₃)₂, -CH₂OH or -CH₂CH₂OH. Preferably, R₄ is -C₁₋₃alkyl, such as methyl, ethyl, propyl, isopropyl, most preferably methyl.

In the aforementioned embodiments of the compound of formula (I), R₄ is preferably H.

In one embodiment of the compound of formula (I), R₅ is H.

In one embodiment of the compound of formula (I), R₅ is -C₁₋₆alkyl, optionally substituted with one or more halogen; such as, but is not limited to methyl, ethyl, propyl (including n-propyl and isopropyl), butyl (including n-butyl, isobutyl, sec-butyl or tert-butyl), pentyl (including n-pentyl, isopentyl, neopentyl), n-hexyl, 2- methylpentyl, -CH₂F, -CHF₂, -CF₃, -C₂F₅, -CH₂CF₃, -CH₂Cl,-CH₂CH₂CF₃ or -CH(CF₃)₂.

In the aforementioned embodiments of the compound of formula (I), R₅ is preferably -C₁₋₃alkyl, such as methyl, ethyl, propyl, isopropyl, most preferably methyl.

In one embodiment of the compound of formula (I), X is O.

In one embodiment of the compound of formula (I), X is C(R₆)₂, wherein each R₆ is H.

In one embodiment of the compound of formula (I), X is C(R₆)₂, wherein one of R₆ is H, and the other is selected from halogen, CN, -OH, -C₁₋₆alkyl, -O-C₁₋₆alkyl, -C(O)-C₁₋₆alkyl, -C(O)-C₃₋₆cycloalkyl, -SO₂-C₁₋₆alkyl, -SO₂-C₃₋₆cycloalkyl, -SO-C₁₋₆alkyl, -SO-C₃₋₆cycloalkyl, -C₆₋₁₀aryl or - C₃₋₆cycloalkyl, wherein the -C₁₋₆alkyl, -C₆₋₁₀aryl or -C₃₋₆cycloalkyl is optionally substituted with one or more halogen, hydroxyl, -O-C₁₋₆alkyl, -C₁₋₆alkyl or -C₁₋₆alkyl substituted with hydroxyl or halogen.

In one embodiment of the compound of formula (I), X is C(R₆)₂, wherein one of R₆ is H, and the other is selected from halogen, such as F, Cl, Br, I.

In one embodiment of the compound of formula (I), X is C(R₆)₂, wherein one of R₆ is H, and the other is OH, CN, NH₂, -NH-C₁₋₆alkyl, -N(C₁₋₆alkyl)₂, such as but is not limited to OH, CN, -NH₂, - NH-CH₃, -NH-CH₂-CH₃, -N(CH₃)₂, -N(CH₂-CH₃)₂, -N(CH₃)(CH₂-CH₃).

In one embodiment of the compound of formula (I), X is C(R₆)₂, wherein one of R₆ is H, and the other is -C₁₋₆alkyl, optionally substituted with one or more halogen, hydroxyl or -O-C₁₋₆alkyl, such as but is not limited to methyl, ethyl, propyl (including n-propyl and isopropyl), butyl (including n-butyl, isobutyl, sec-butyl or tert-butyl), pentyl (including n-pentyl, isopentyl, neopentyl), n-hexyl, 2-methylpentyl, -CH₂F, -CHF₂, -CF₃, -C₂F₅, -CH₂CF₃, -CH₂Cl, -CH₂CH₂CF₃, -CH(CF₃)₂, -CH₂OH, - CH₂CH₂OH, -CH₂OCH₃ and -CH₂OCH₂CH₃.

In one embodiment of the compound of formula (I), X is C(R₆)₂, wherein one of R₆ is H, and the other is -O-C₁₋₆alkyl, wherein the -C₁₋₆alkyl is optionally substituted with one or more halogen, hydroxyl or -O-C₁₋₆alkyl, such as but is not limited to -OCH₃, -OCH₂CH₃, -OCF₃, -OCH₂CF₃, - OCH₂CH₂OH, -OCH₂CH₂OCH₃.

In one embodiment of the compound of formula (I), X is C(R₆)₂, wherein one of R₆ is H, and the other is -C(O)-C₁₋₆alkyl, -C(O)-C₃₋₆cycloalkyl, -SO₂-C₁₋₆alkyl, - SO₂-C₃₋₆cycloalkyl, -SO-C₁₋₆alkyl, - SO-C₃₋₆cycloalkyl, -C₆₋₁₀aryl or -C₃₋₆cycloalkyl, such as but is not limited to -C(O)-CH₃, -C(O)-CH₂CH₃, C(O)-CH₂CH₂CH₃, -C(O)-CH(CH₃)₂, -C(O)-cyclopropyl, -C(O)-cyclopentyl, -C(O)-cyclohexyl, -SO₂-CH₃, -SO₂-CH₂CH₃, SO₂-CH₂CH₂CH₃, SO₂-CH(CH₃)₂, -SO₂-cyclopropyl, -SO₂-cyclopentyl, -SO-CH₃, -SO-CH₂CH₃, SO-CH₂CH₂CH₃, SO-CH(CH₃)₂, -SO-cyclopropyl, -SO-cyclopentyl, phenyl, cyclopropyl, cyclobutyl, cyclopentyl or cyclohexyl, wherein the -C₁₋₆alkyl, the -C₆₋₁₀aryl or the C₃₋₆cycloalkyl is optionally substituted with one or more halogen, hydroxyl, -O-C₁₋₆alkyl, -C₁₋₆alkyl, or -C₁₋₆alkyl substituted with halogen or hydroxyl, the substituents are, e.g. but not limited to fluorine, chlorine, bromine, iodine, -OH, -CH₃, -CH₂CH₃, -CH₂CH₂CH₃, -CH(CH₃)₂, -O-CH₃, -O-CH₂CH₃, -O-CH₂CH₂CH₃, -OCH(CH₃)₂, -CF₃, -CH₂CF₃, -CH₂OH, -CH₂CH₂OH.

In one embodiment of the compound of formula (I), X is C(R₆)₂, wherein each R₆ is independently -C₁₋₆alkyl, halogen, CN, -OH, -NH₂, -NH-C₁₋₆alkyl, -N(C₁₋₆alkyl)₂, -O-C₁₋₆alkyl, wherein the -C₁₋₆alkyl is optionally substituted with one or more halogen, hydroxyl or -O-C₁₋₆alkyl;

Examples include but are not limited to, two R₆ both are halogen, both are said optionally (such as halogen) substituted -C₁₋₆alkyl, one is halogen and the other is said optionally (such as halogen) substituted -C₁₋₆alkyl, one is OH or -O-C₁₋₆alkyl and the other is said optionally (such as halogen) substituted -C₁₋₆alkyl, one is NH₂, -NH-C₁₋₆alkyl or -N(C₁₋₆alkyl)₂ and and the other is said optionally (such as halogen) substituted -C₁₋₆alkyl, one is CN and the other is said optionally (such as halogen) substituted -C₁₋₆alkyl;

Examples of X include but are not limited to CF₂, CCl₂, CBr₂, CFCl, C(CH₃)₂, C(CH₂CH₃)₂, C(CH₃)(CH₂CH₃), C(CF₃)(CF₃), C(CH₃)(CF₃), C(CH₃)(CH₂OH), C(CH₃)(CH₂OCH₃), C(CH₃)(F), C(CH₃)(OCH₃), C(CH₃)(OH), C(CH₃)(NH₂), -C(CH₃)(NHCH₃)-.

In one embodiment of the compound of formula (I), X is C(R₆)₂, wherein each R₆ is independently selected from H or halogen, for example both are H, or both are halogen, for example both are F.

In one embodiment of the compound of formula (I), X is NR₇, and R₇ is H.

In one embodiment of the compound of formula (I), X is NR₇, R₇ is -C₁₋₆alkyl optionally substituted with one or more halogen, hydroxyl or -O-C₁₋₆alkyl, such as but is not limited to methyl, ethyl, propyl (including n-propyl and isopropyl), butyl (including n-butyl, isobutyl, sec-butyl or tert-butyl), pentyl (including n-pentyl, isopentyl, neopentyl), n-hexyl, 2- methylpentyl, -CH₂F, -CHF₂, - CF₃, -C₂F₅, -CH₂CF₃, -CH₂Cl,-CH₂CH₂CF₃, -CH(CF₃)₂, -CH₂OH, -CH₂CH₂OH, -CH₂OCH₃ and - CH₂OCH₂CH₃; preferably R₇ is methyl, ethyl, propyl or isopropyl.

In one embodiment of the compound of formula (I), X is NR₇, R₇ is -C(O)-C₁₋₆alkyl, -C(O)-C₃₋₆cycloalkyl, -SO₂-C₁₋₆alkyl, - SO₂-C₃₋₆cycloalkyl, -SO-C₁₋₆alkyl or -SO-C₃₋₆cycloalkyl, such as but is not limited to -C(O)-CH₃, -C(O)-CH₂CH₃, C(O)-CH₂CH₂CH₃, -C(O)-CH(CH₃)₂, -C(O)-cyclopropyl, -C(O)-cyclobutyl, -C(O)-cyclopentyl, -C(O)-cyclohexyl, -SO₂-CH₃, -SO₂-CH₂CH₃, SO₂-CH₂CH₂CH₃, SO₂-CH(CH₃)₂, -SO₂-cyclopropyl, -SO₂-cyclobutyl, -SO₂-cyclopentyl, -SO₂-cyclohexyl, -SO-CH₃, -SO-CH₂CH₃, SO-CH₂CH₂CH₃, SO-CH(CH₃)₂, -SO-cyclopropyl, -SO-cyclobutyl, -SO-cyclopentyl or -SO-cyclohexyl, wherein each -C₁₋₆alkyl or -C₃₋₆cycloalkyl is optionally substituted with one or more halogen, hydroxyl, -O-C₁₋₆alkyl, -C₁₋₆alkyl, or -C₁₋₆alkyl substituted with halogen or hydroxyl, the substituents are e.g., but not limited to, fluorine, chlorine, bromine, iodine, -OH, -CH₃, -CH₂CH₃, -CH₂CH₂CH₃, -CH(CH₃)₂, -O-CH₃, -O-CH₂CH₃, -O-CH₂CH₂CH₃, -O CH(CH₃)₂, -CF₃, -CH₂CF₃, -CH₂OH, -CH₂CH₂OH, -CH₂OCH₃ and -CH₂OCH₂CH₃.

In one embodiment of the compound of formula (I), X is NR₇, R₇ is selected from H, -C₁₋₆alkyl and -C(O)-C₁₋₆alkyl, preferably -C₁₋₆alkyl, or preferably -C(O)-C₁₋₆alkyl, such as but not limited to methyl, ethyl, propyl, isopropyl, -C(O)-CH₃, -C(O)-CH₂CH₃, C(O)-CH₂CH₂CH₃ or -C(O)-CH(CH₃)₂.

In the aforementioned embodiments of the compound of formula (I), X is preferably -O-, -NH-, -N(C₁₋₆alkyl)-, -CH₂-, -C(halogen)₂-, such as -O-, -NH-, -N(CH₃)-, -CH₂-, -C(F)₂-.

In the aforementioned embodiments of the compound of formula (I), X is preferably -O-, -N(C₁₋₆alkyl)-, -N(CO-C₁₋₆alkyl)-, -CH₂-, -CH(C₁₋₆alkyl)-, -C(C₁₋₆alkyl)₂- or -C(halogen)₂-, such as -O-, - N(CH₃)-, -N(CO-CH₃)-, -CH₂-, -C(F)₂-.

In one embodiment of the compound of formula (I), Y is N.

In one embodiment of the compound of formula (I), Y is CRs, wherein R₈ is H.

In one embodiment of the compound of formula (I), Y is CR₈, wherein R₈ is OH.

In one embodiment of the compound of formula (I), Y is CRs, wherein R₈ is halogen, such as fluorine, chlorine, bromine, iodine, preferably F.

In the aforementioned embodiments of the compound of formula (I), Y is preferably N, or is CRs, wherein R₈ is OH; more preferably Y is N.

In one embodiment of the compound of formula (I), the six-membered ring containing X and Y 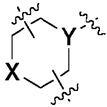 is selected from 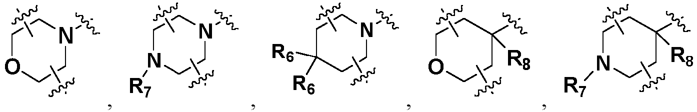 or 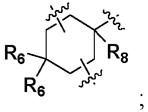 preferably the six-membered ring containing X and Y is selected from 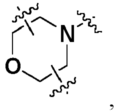 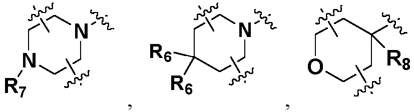 and 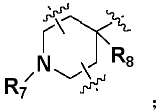 more preferably the six-membered ring containing X and Y is selected from 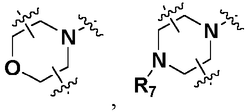 and 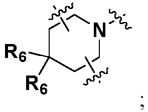 R₆, R₇ and R₈ have the definitions defined in each of the corresponding embodiments mentioned-above, preferably R₆ is selected from H, halogen and -C₁₋₆alkyl, R₇ is selected from -C₁₋₆alkyl and -CO-C₁₋₆alkyl.

In one embodiment of the compound of formula (I), R₁ and R₂ are each independently H.

In one embodiment of the compound of formula (I), R₁ and R₂ are each independently -OH, oxo, halogen, CN, -C₁₋₆alkyl or -O-C₁₋₆alkyl, wherein the -C₁₋₆alkyl is optionally substituted with one or more halogen or hydroxyl, such as but not limited to -OH, oxo, fluorine, chlorine, bromine, iodine, CN, -CH₃, -CH₂CH₃, -O-CH₃, -O-CH₂CH₃, -CF₃, -CH₂CF₃, -CH₂OH, -CH₂CH₂OH, preferably -CH₃, -CF₃ or -CH₂CH₃, most preferably -CH₃.

In one embodiment of the compound of formula (I), R₁ and R₂ are linked together to form a C₁₋₃ alkylene bridge, such as -CH₂-, -CH₂CH₂-, -CH₂CH₂CH₂-.

In one embodiment of the compound of formula (I), R₁ and R₂ are linked together to form a C₁₋₃ alkylene bridge, the form of bridge is e.g., but not limited to: 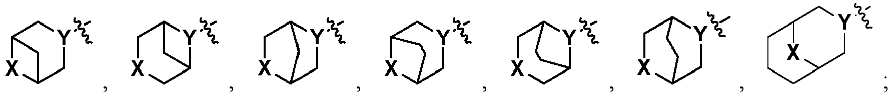 preferably 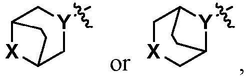 wherein preferably Y is N and X is O.

In aforementioned embodiments of the compound of the formula (I), preferably R₁ and R₂ are each independently H, -CH₃, -CH₂CH₃, or R₁ and R₂ are linked together to form a C₁₋₃ alkylene bridge, preferably C₂ ethylene bridge.

In one embodiment of the compound of formula (I), m and n are both 0.

In one embodiment of the compound of formula (I), one of m and n is 0, the other is 1, when R₁ or R₂ is not hydrogen and can be connected to an ortho position of Y or an ortho position of X on the ring, preferably connected to an ortho position of Y. For example, R₁ or R₂ is -C₁₋₆alkyl, such as but not limited to -CH₃, -CH₂CH₃ or -CH(CH₃)₂, preferably R₁ or R₂ is -CH₃, connected to an ortho position of Y or an ortho position of X, preferably connected to an ortho position of Y.

In one embodiment of the compound of formula (I), one of m and n is 0, the other is 2, when R₁ or R₂ is not hydrogen and can both be connected to an ortho position of Y, an ortho position of X, or respectively connected to an ortho-position of Y and an ortho-position of X on the ring, preferably both are connected to an ortho-position of Y. For example, R₁ or R₂ are each independently -C₁₋₆alkyl, such as but not limited to -CH₃, -CH₂CH₃ or -CH₂-CH₂-CH₃, preferably R₁ or R₂ is -CH₃, both are connected to an ortho-position of Y.

In one embodiment of the compound of formula (I), one of m and n is 0, the other is 3 or 4, when R₁ or R₂ is not hydrogen, and R₁ or R₂ is, e.g., not limited to, -CH₃, -CH₂CH₃ or -CH₂-CH₂-CH₃, for example the R₁ or R₂ is -C₁₋₆alkyl, preferably R₁ or R₂ is -CH₃.

In one embodiment of the compound of formula (I), m and n are both 1, when R₁ and R₂ are not hydrogen, each independently can be connected to an ortho position of Y or an ortho position of X on the ring, for example, both are connected ortho positions of Y, or both connected to ortho positions of X, or respectively connected to an ortho position of Y and an ortho position of X; preferably R₁ and R₂ are -C₁₋₆alkyl, more preferably R₁ and R₂ are -CH₃, both are connected to ortho-positions of Y, or both are connected to ortho-positions of X, or are respectively connected to an ortho-position of Y and an ortho-position of X.

In one embodiment of the compound of formula (I), m and are both 1, when R₁ and R₂ are not hydrogen, and R₁ and R₂ connected to an ortho position of Y and an ortho position of X, preferably both connected to, ortho positions of Y and ortho positions of X are linked together to form a C₁₋₃ alkylene bridge, such as -CH₂-, -CH₂CH₂-, -CH₂CH₂CH₂-.

In one embodiment of the compound of formula (I), m is 1 and n is 2, or m is 1 and n is 3,or m is 1 and n is 4, or m is 2 and n is 2, or m is 2 and n is 3, or m is 2 and n is 4, or m is 3 and n is 4, or m is 4 and n is 4, wherein for example R₁ and R₂ are -C₁₋₆alkyl, preferably R₁ and R₂ are -CH₃.

In one embodiment of the compound of formula (I), one of m and n is 0, the other is 1, and R₁ or R₂ is each independently -C₁₋₆alkyl optionally substituted with one or more halogen, connected to an ortho position of Y or an ortho position of X;
or one of m and n is 0, the other is 2, and R₁ or R₂ is each independently -C₁₋₆alkyl optionally substituted with one or more halogen, connected to an ortho-position of Y, an ortho position of X, or respectively connected to an ortho position of Y and an ortho position of X;
or m and n are both 1 and R₁ and R₂ are each independently -C₁₋₆alkyl optionally substituted with one or more halogen, both connected to ortho positions of Y on the ring, both connected to ortho positions of X, or respectively connected to an ortho position of Y and an ortho position of X.

In one embodiment of the compound of formula (I), preferably one of m and n is 0, the other is 1, and R₁ or R₂ is -C₁₋₆alkyl connected to an ortho position of Y or an ortho position of X.

In one embodiment of the compound of formula (I), one of m and n is 0, the other is 1, and R₁ or R₂ is -C₁₋₆alkyl optionally substituted with halogen and connected to an ortho position of Y; or one of m and n is 0, the other is 2, and R₁ or R₂ are each independently -C₁₋₆alkyl optionally substituted with halogen, both connected to an ortho position of Y.

In the aforementioned embodiments of the compound of formula (I), where chemically feasible, R₁ and/or R₂ can be in R or S configuration, preferably R configuration.

In one embodiment of the compound of formula (I), wherein the six-membered ring containing X and Y is e.g., but not limited to: 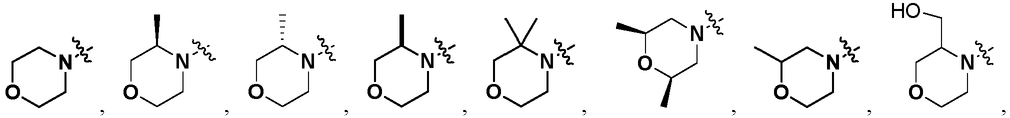 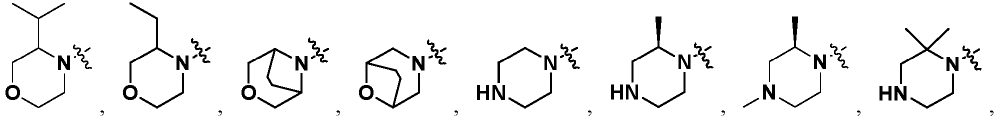 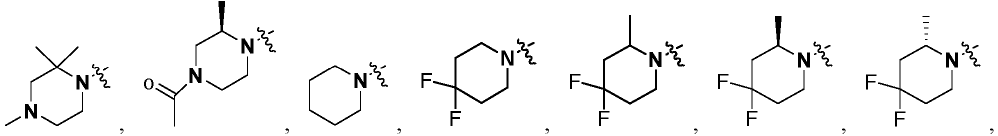 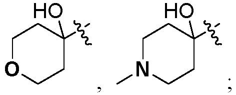 preferably 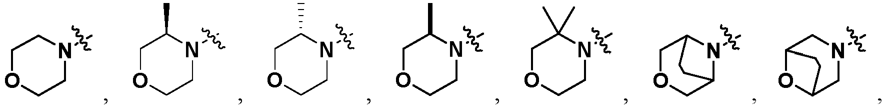 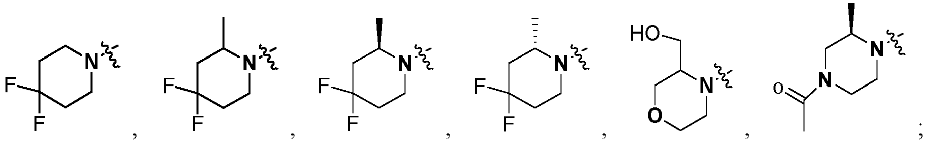 more preferably

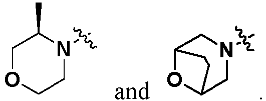

In one embodiment of the compound of formula (I), R₃ is H.

In one embodiment of the compound of formula (I), R₃ is halogen, such as fluorine, chlorine, bromine, iodine, preferably fluorine or chlorine.

It should be noted that the compound of the present disclosure covers each independent embodiment or each specific exemplified embodiment as mentioned above, and also covers the embodiments formed by any combination or sub-combination of each independent embodiment or each specific exemplified embodiment as mentioned above, and also covers the embodiments formed by any combination of any preferred or exemplified embodiment as mentioned above.

In one embodiment of the compound of formula (I), 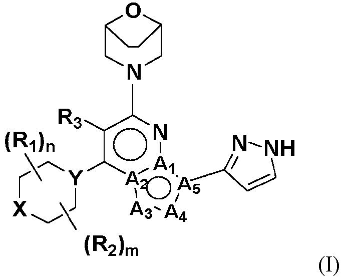 wherein,
the heteroaryl moiety containing A₁~A₅ and consisting of a six membered ring fused to a five membered ring is selected from: 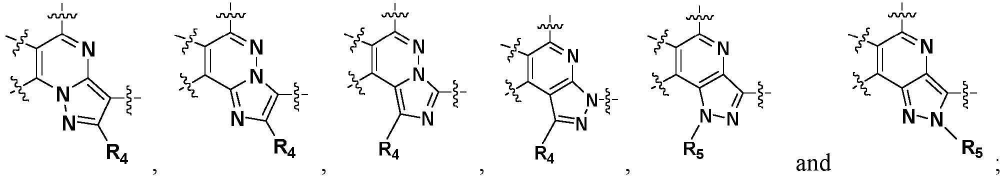
the six-membered ring containing X and Y is selected from 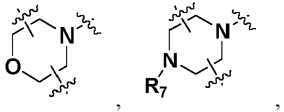 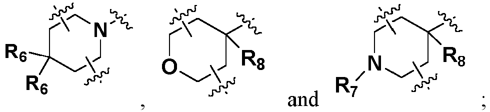
R₁ and R₂ are each independently H or -C₁₋₆alkyl, wherein the -C₁₋₆alkyl is optionally substituted with one or more halogen; or R₁ and R₂ are linked together to form a C₁₋₃ alkylene bridge;
R₃ is H or halogen;
R₄ is H;
R₅ is H or -C₁₋₆alkyl;
R₆ are each independently H, halogen, -C₁₋₆alkyl or -O-C₁₋₆alkyl, wherein the -C₁₋₆alkyl is optionally substituted with one or more halogen;
R₇ is H or -C₁₋₆alkyl, wherein the -C₁₋₆alkyl is optionally substituted with one or more halogen;
R₈ is H, -OH or halogen;
n and m are each independently an integer of 0 to 2.

In a preferred mode of the embodiment, the heteroaryl moiety containing A₁~A₅ and consisting of a six membered ring fused to a five membered ring is selected from

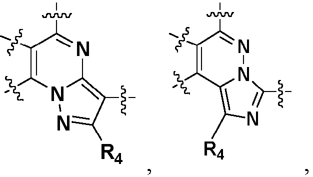

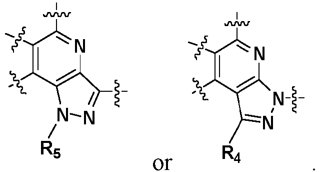

In a preferred mode of the embodiment, the six-membered ring containing X and Y is selected from 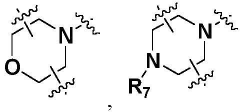 and

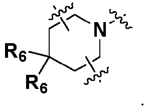

In a preferred mode of the embodiment, the six-membered ring containing X and Y is 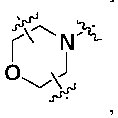 for example the six-membered ring containing X and Y is 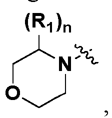 wherein R₁ is each independently -C₁₋₆alkyl, optionally substituted with halogen, n is selected from 0, 1 or 2; or R₁ and R₂ of the six-membered ring containing X and Y 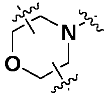 are linked together to form a C₁₋₃ alkylene bridge, such as 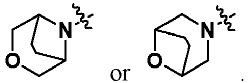 In a preferred mode of the embodiment, the six-membered ring containing X and Y is 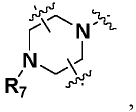 for example, the six-membered ring containing X and Y is 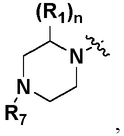 wherein R₁ is each independently -C₁₋₆alkyl optionally substituted with halogen, n is selected from 1 or 2, R₇ is selected from -C₁₋₆alkyl or -CO-C₁₋₆alkyl.

In a preferred mode of the embodiment, the six-membered ring containing X and Y is 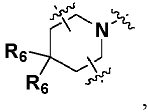 for examples the six-membered ring containing X and Y is 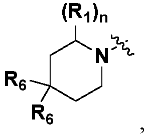 wherein R₁ is each independently -C₁₋₆alkyl optionally substituted with halogen, n is selected from 0 or 1, R₆ is selected from H, halogen or -C₁₋₆alkyl substituted with halogen.

In a preferred mode of the embodiment, the six-membered ring containing X and Y is selected from

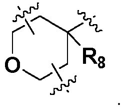

In a preferred mode of the embodiment, the six-membered ring containing X and Y is selected from

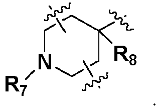

In the above-mentioned preferred modes of the embodiment, R₁ and R₂ are each independently H.

In the above-mentioned preferred modes of the embodiment, R₁ and R₂ are each independently - C₁₋₆alkyl optionally substituted with one or more halogen, such as -CH₃, -CH₂CH₃, -CH₂CH₂CH₃, - CH(CH₃)₂, -CF₃ or -CH₂CF₃, most preferably -CH₃.

In a preferred mode of the embodiment, R₁ and R₂ are linked together to form a C₁₋₃ alkylene bridge, preferably a C₂ alkylene bridge.

In the above-mentioned preferred modes of the embodiment, R₃ is H.

In the above-mentioned preferred modes of the embodiment, R₅ is -C₁₋₆alkyl, such as -CH₃, - CH₂CH₃, -CH₂CH₂CH₃, -CH(CH₃)₂, most preferably -CH₃.

In the above-mentioned preferred modes of the embodiment, R₆ are each independently H or halogen, preferably H or F.

In the above-mentioned preferred modes of the embodiment, R₇ is H, or -C₁₋₆alkyl, such as -CH₃, -CH₂CH₃, -CH₂CH₂CH₃, -CH(CH₃)₂, most preferably -CH₃.

In the above-mentioned preferred modes of the embodiment, R₈ is -OH.

In the above-mentioned preferred modes of the embodiment, one of n and m is 0, the other is 1, and R₁ or R₂ is connected to an ortho position of Y or an ortho position of X on the ring, preferably connected to an ortho position of Y; for example, R₁ or R₂ is -C₁₋₆alkyl, preferably R₁ or R₂ is -CH₃, connected to an ortho position of Y or an ortho position of X, preferably connected to an ortho position of Y.

In the above-mentioned preferred modes of the embodiment, one of n and m is 0, the other is 2, and R₁ or R₂ are both connected to an ortho-position of Y, an ortho-position of X, or respectively connected to an ortho-position of Y and an ortho-position of X, preferably both connected to an ortho-position of Y. For example, R₁ or R₂ is -C₁₋₆alkyl, preferably R₁ or R₂ is -CH₃, and both connected to an ortho position of Y.

In the above-mentioned preferred modes of the embodiment, n and m are both 1, and R₁ and R₂ are each independently connected to an ortho position of Y, or an ortho position of X, for example, both are connected to ortho positions of Y, or both are connected to ortho-positions of X, or respectively connected to an ortho-position of Y and an ortho-position of X, preferably both are connected to ortho positions of Y, preferably, R₁ and R₂ are -C₁₋₆alkyl, more preferably R₁ and R₂ are both -CH₃, both connected to ortho positions of Y, or both connected to ortho positions of X, or respectively connected to an ortho position of Y and an ortho position of X, preferably both connected to ortho positions of Y; or R₁ and R₂ connected to an ortho position of Y and an ortho position of X, preferably both connected to ortho positions of Y and ortho positions of X, are linked together to form a C₁₋₃ alkylene bridge, for example -CH₂-, -CH₂CH₂-, -CH₂CH₂CH₂-.

In the aforementioned preferred modes of the embodiment, when chemically feasible, R₁ and/or R₂ can be in R or S configuration, preferably R configuration.

It should be noted that the compounds of the present disclosure encompass the embodiments constituted by any combination of any of the above preferred or exemplary embodiments.

Specific embodiments of the compounds of the present disclosure include the following specific compounds, stereoisomers, tautomers, stable isotopic variants, pharmaceutically acceptable salts or solvates thereof,

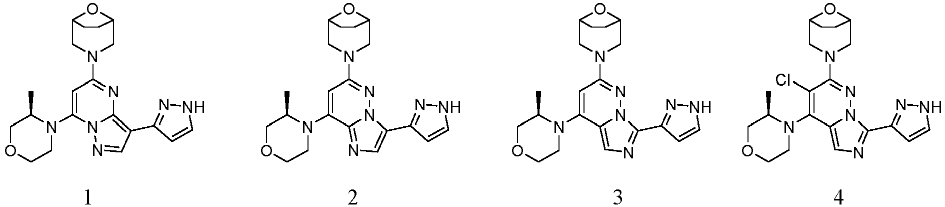

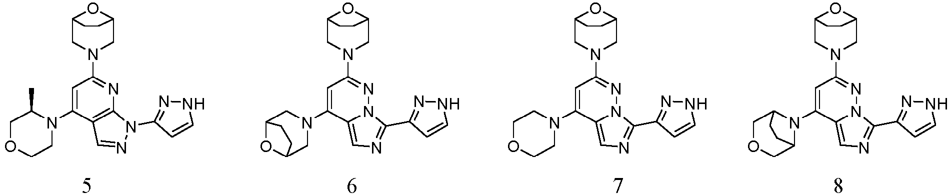

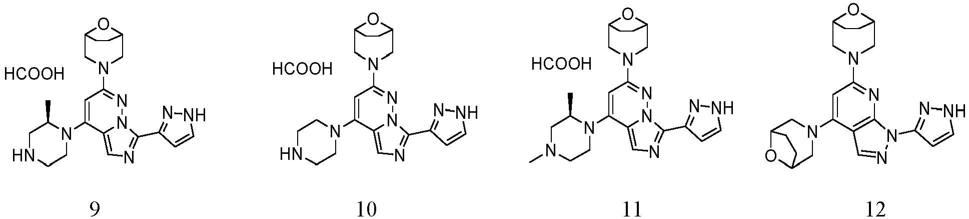

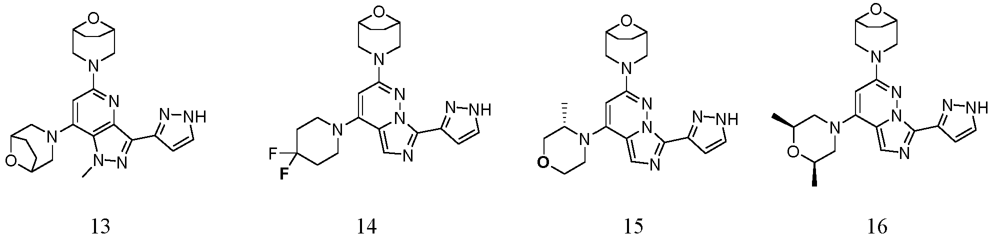

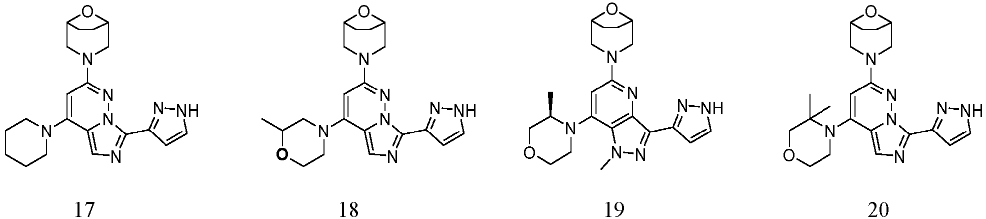

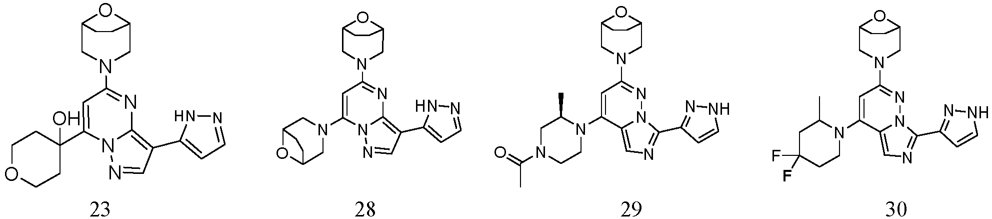

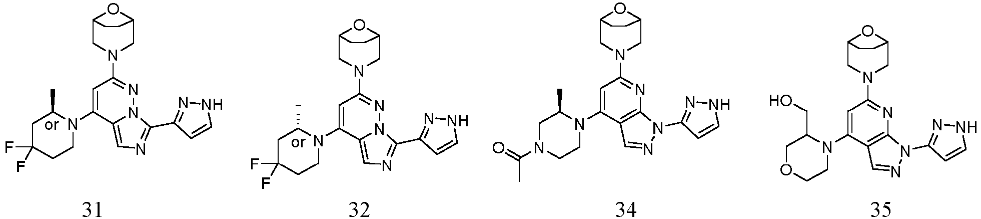

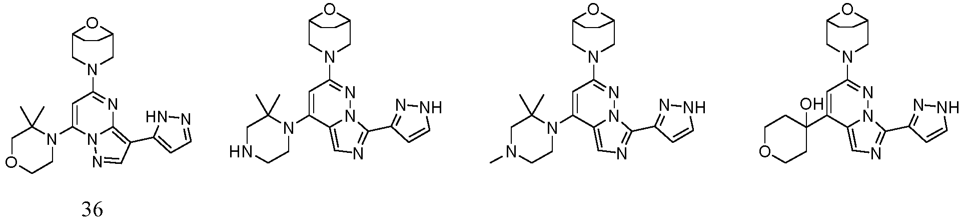

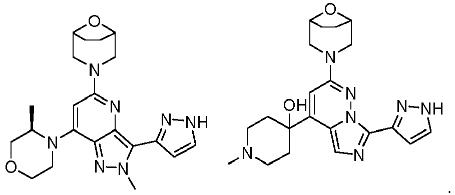

For the compounds of the present disclosure useful as a medicine, the method of prevention or treatment, the pharmaceutical composition, the combination or the use of the present disclosure described below, each preferred embodiment of the compound of formula (I) as defined herein is preferred, more preferably the specific compounds listed.

### Beneficial Effects of the Present Invention

As aforementioned, it is known that ATR kinase plays a role in tumorigenesis as well as in a variety of other diseases. We have surprisingly found that the above-mentioned compounds of formula (I) are capable of potently inhibiting ATR kinase and thus have value as anti-proliferative, anti-apoptotic and/or anti-invasive drugs in the suppression and/or treatment of solid and/or liquid tumor diseases. In particular, the compounds of the present disclosure are expected to be useful in the prevention or treatment of those tumors which are sensitive to inhibition of ATR. In addition, the compounds of the present disclosure are expected to be useful in the prevention or treatment of tumors mediated solely or partly by ATR.

Specifically, it has been found through research that the compounds of the present disclosure can effectively inhibit the activity of ATR kinase and tumor cell lines, and can achieve one or more of the following technical effects:
- High inhibitory activity on ATR kinase: the compounds show in the ATR kinase inhibition assay an IC50 in the range of 0.1 nM~1 µM, preferably in the range of 0.1 nM~0.5 µM, preferably in the range of 0.1 nM~0.1 µM, more preferably in the range of 0.1 nM~50 nM, 0.1nM~20nM, as demonstrated in the Activity Example 1; and/or
- High inhibitory activity on LOVO cell line proliferation, as demonstrated in the Activity Example 2; and/or
- Good pharmacokinetic properties, such as longer t_{1/2}, allowing for example greater dosing intervals, longer half-life, and better patient compliance, as demonstrated in the Activity Example 3; and/or
- Improved AUC0-t data, better druggability, higher bioavailability, as demonstrated in the Activity Example 4 hereinafter; and/or
- Good safety profile, e.g. membrane permeability, P450 (reduced risk of drug interactions), lower toxicity and/or fewer side effects; and/or
- Excellent physicochemical properties, such as solubility, physical and/or chemical stability, as demonstrated in the Activity Example 5.

Based on the above-mentioned beneficial effects of the compounds of the present disclosure, the present disclosure further provides technical solutions in each aspect as decribed hereinafter.

### Compounds of the Disclosure for Use in Therapy or as a Medicament

In one aspect, the present disclosure provides compounds of the present disclosure for use as medicaments, particularly as ATR inhibitors, more especially as anticancer or antineoplastic agents.

In another aspect, the present disclosure provides compounds of the present disclosure for use in the treatment and/or prevention of ATR-associated diseases.

In specific embodiments, the present disclosure provides compounds of the present disclosure for use in the treatment and/or prevention of diseases in which ATR contributes to the occurrence and progression of the diseases or in which inhibition of ATR will reduce the incidence, reduce or eliminate disease symptoms. The diseases, e.g. tumors or cancers, includw but are not limited to: hematological malignancies, e.g. leukemia (including chronic lymphocytic leukemia, acute lymphocytic leukemia, acute myelogenous leukemia, and chronic myelogenous leukemia), multiple myeloma, lymphoid malignancies (e.g. lymphoma, Hodgkin's disease, non-Hodgkin's lymphoma), myelodysplastic syndromes, and solid tumors such as carcinomas and sarcomas and their metastases, e.g. breast cancer, lung cancer (non-small cell lung cancer, small cell lung cancer, squamous cell carcinoma, bronchioloalveolar carcinoma), central nervous system tumors (e.g. glioma, dysembryonic dysplastic neuroepithelial tumor, glioblastoma multiforme, mixed Glioma, medulloblastoma, retinoblastoma, neuroblastoma, germ cell tumor and teratoma), gastrointestinal cancers (e.g. gastric cancer, esophageal cancer, liver cancer, bile duct cancer, colorectal cancer, carcinoma of small intestine, pancreatic cancer), skin cancer, melanoma, thyroid cancer, bone cancer, head and neck cancer, salivary gland cancer, prostate cancer, testicular cancer, ovarian cancer, cervical cancer, uterine cancer, endometrial cancer, vulvar cancer, bladder cancer, renal cancer, squamous cell carcinoma, sarcomas (e.g. osteosarcoma, chondrosarcoma, leiomyosarcoma, soft tissue sarcoma, Ewing's sarcoma, gastrointestinal tissue carcinoma, gastrointestinal stromal tumor, Kaposi's sarcoma), and pediatric cancers (e.g. rhabdomyosarcoma and neuroblastoma).

The present disclosure especially provides a compound of formula (I), a stereoisomer, a tautomer, a stable isotopic variant, a pharmaceutically acceptable salt or a solvate thereof, useful for the treatment of patients with lung cancer, prostate cancer, melanoma, ovarian cancer, breast cancer, endometrial cancer, renal cancer, gastric cancer, sarcoma, head and neck cancer, central nervous system tumors and their metastases, and patients with acute myeloid leukemia.

### Pharmaceutical Compositions and their Administration

In another aspect, in order to achieve the prevention or therapeutic purposes, the compounds of the present disclosure can be formulated into pharmaceutical compositions according to standard pharmaceutical practice. Meanwhile, based on the good pharmacokinetic properties, improved AUC0-last and good druggability of the compounds of the present disclosure, medicines with better pharmacokinetic properties and higher bioavailability can be prepared from the compounds of the present disclosure.

Accordingly, the present disclosure provides a pharmaceutical composition comprising the above-described compound of the present disclosure and a pharmaceutically acceptable excipient.

The selection of excipients for inclusion in a particular composition will depend on factors such as the mode of administration and the form of the composition provided. Suitable pharmaceutically acceptable excipients are well known to those skilled in the art and are described, for example, in Ansel, Howard C., et al., Ansel's Pharmaceutical Dosage Forms and Drug Delivery Systems. Philadelphia: Lippincott, Williams & Wilkins, 2004, including e.g., adjuvants, diluents (e.g., glucose, lactose or mannitol), carriers, pH adjusters, buffers, sweeteners, fillers, stabilizers, surfactants, wetting agents, lubricants, emulsifiers, suspending agents, preservatives, antioxidants, opacifiers, glidants, processing aids, colorants, perfuming agents, flavoring agents, and other known additives.

The pharmaceutical compositions of the present disclosure can be formulated by techniques known to those skilled in the art, such as those disclosed in Remington's Pharmaceutical Sciences 20th Edition.

The pharmaceutical compositions of the present disclosure can be administered in a standard manner. For example, suitable modes of administration include oral, intravenous, rectal, parenteral, topical, transdermal, ocular, nasal, buccal or pulmonary (inhalation) administration, wherein the parenteral infusion includes intramuscular, intravenous, intraarterial, intraperitoneal or subcutaneous administration. For these purposes, the compounds of the present disclosure may be formulated by methods known in the art into forms, such as tablets, capsules, syrups, powders, granules, aqueous or oily solutions or suspensions, (lipid) emulsions, dispersible powders, suppositories, ointments, creams, drops, aerosols, dry powder formulations and sterile injectable aqueous or oily solutions or suspensions.

The magnitude of a prophylactic or therapeutic dose of a compound of the disclosure will vary depending on a range of factors, including the individual being treated, the severity of disorder or condition, the rate of administration, the disposition of the compound, and the judgment of the prescribing physician. For the treatment of a particular disease, an effective amount is an amount sufficient to ameliorate or reduce the symptoms associated with the disease. Such amounts may be administered as a single dose, or may be administered according to an effective treatment regimen. In general, effective dose ranges from about 0.0001 to about 5000 mg/kg body weight/day, e.g., about 0.01 to about 1000 mg/kg/day (single or divided administration). For a human of 70 kg, this would add up to about 0.007mg/day to about 7000mg/day, e.g., about 0.7mg/day to about 1500mg/day. Depending on the mode of administration, the content or amount of the compound of the present disclosure in a pharmaceutical composition may be about 0.01 mg to about 1000 mg, suitably 0.1-500 mg, preferably 0.5-300 mg, more preferably 1-150 mg, particularly preferably 1-50 mg, e.g., 1.5 mg, 2 mg, 4 mg, 10 mg, 25 mg, etc.; accordingly, the pharmaceutical composition of the present disclosure will comprise 0.05 to 99% w/w (weight percent), e.g., 0.05 to 80% w/w, e.g., 0.10 to 70% w/w, e.g., 0.10 to 50% w/w of a compound of the invention, all weight percentages are based on the total composition. It is to be understood that it may be necessary in certain circumstances to use doses beyonding these limits.

In a specific embodiment, the present disclsoure provides a pharmaceutical composition comprising a compound of the present disclosure and one or more pharmaceutically acceptable excipients, and the composition is formulated for oral administration. The composition may be provided in unit dosage forms, e.g., in the form of a tablet, capsule, or oral liquid. Such unit dosage forms may contain 0.1 mg to 1 g, e.g., 5 mg to 250 mg, of a compound of the present disclosure as the active ingredient.

In a specific embodiment, the present disclosure provides a pharmaceutical composition comprising a compound of the present disclsoure and one or more pharmaceutically acceptable excipients, and the composition is formulated for topical administration. Topical administration can be in the form of, e.g., a cream, lotion, ointment or transdermal patch, wherein the concentration of the compound of the present disclosure may be from about 0.01 to 100 mg per gram of carrier.

In a specific embodiment, the present disclosure provides a pharmaceutical composition comprising a compound of the present disclosure and one or more pharmaceutically acceptable excipients, and the composition is formulated for administration by inhalation. Administration by inhalation can be by oral inhalation or intranasal administration. When administered by oral inhalation, a compound of the present disclosure can be effectively used in the present invention in daily dose, e.g., up to 500 µg, such as 0.1-50 µg, 0.1-40 µg, 0.1-30 µg, 0.1-20 µg or 0.1-10 µg of the present compound. The pharmaceutical composition of the present disclosure for oral inhalation may be formulated as dry powders, suspension (in liquid or gas) or solution (in liquid), and may be in any suitable form and using any suitable inhaler device known in the art, including, e.g., metered dose inhaler (MDI), dry powder inhaler (DPI), nebulizer, and soft mist inhaler. Multi-chamber devices can be used to deliver a compound of the present disclosure and one or more other active ingredients (when present).

In a specific embodiment, the pharmaceutical composition of the present disclsoure may additionally comprise additional therapeutically active ingredients suitable for use in combination with the compounds of the present disclosure.

Other therapeutically active ingredients suitable for administration in combination with the compounds of the present disclosure can be known anticancer drugs, especially other anticancer drugs associated with DNA damage and repair mechanisms, including PARP inhibitors, HDAC inhibitors etc. Other therapeutically active ingredients suitable for administration in combination with the compounds of the present disclosure can also be selected from anticancer drugs associated with cell division checkpoints, including ChK1/2 inhibitors, CDK4/6 inhibitors, ATM/ATR inhibitors. Other known anticancer drugs that can be used in combination include alkylating agents, topoisomerase I/II inhibitors, RNA/DNA antimetabolites, antimitotic agents, antibody drugs, kinase inhibitors, etc. For combined administration, the compounds of the present disclosure and at least one known anticancer drug can be administered as a single pharmaceutical composition, and can also be administered separately, simultaneously or sequentially as separate entities, eg, as a kit.

The compounds of the present disclosure may also be administered as bioconjugates. The bioconjugate is composed of a compound of the present disclosure and at least one known therapeutically active antibody such as Herceptin or MabThera (Rituximab), or growth factors such as EGF or FGF, or cytokines such as interleukin 2 or 4 or any molecular that can be combined with cell surface. The antibodies and other molecules can deliver the compound of the present disclosure to targets thereof to function, meanwhile the therapeutic activity of the antibodies or other molecules can also be enhanced.

The compounds of the present disclosure may also be used in combination with radiation therapy, which may be administered at the same or different times.

The above-mentioned pharmaceutical compositions provided in the present disclosure can be used for preventing or treating the above-defined ATR-associated diseases in a subject e.g. a mammal such as a human.

### Treatment Methods and Uses

Based on the beneficial effects of the compounds of the present disclosure as described above, the compounds of the present disclsoure can be used in a method for treating ATR associated diseases in an animal, especially a mammal such as a human.

Therefore, in a further aspect, the present disclsoure provides a method of modulating, especially inhibiting ATR kinase activity, comprising contacting a cell with a compound of the present disclsoure as described above to modulate, especially inhibit ATR activityin the cell.

Based on the same properties, the present disclosure accordingly provides a method of inhibiting abnormal cell growth in a mammal, comprising administering to the mammal a therapeutically effective amount of a compound of the present disclsoure, or a pharmaceutical composition comprising a compound of the present disclsoure.

In another aspect, the present disclosure provides a method of preventing or treating an ATR-associated disease(e.g., a disease treatable or preventable by ATR inhibition), comprising administering to an individual in need thereof an effective amount of a compound of the present disclosure or a pharmaceutical composition comprising the same as described above.

In another aspect, the present disclosure provides a use of a compound of the present disclosure or a pharmaceutical composition comprising the same as described above, for inhibiting ATR activity, or for treating and/or preventing an ATR-associated disease, e.g., a disease treatable or preventable by ATR inhibition.

In another aspect, the present disclosure also provides a use of a compound of the present disclosure or a pharmaceutical composition comprising the same as described above in the manufacture of a medicament, especially a medicament with ATR kinase inhibitory activity.

In another aspect, the present disclosure provides a use of a compound of the present disclosure or a pharmaceutical composition comprising the same as described above, in the manufacture of a medicament for the treatment or prevention of an ATR-associated disease, such as a disease treatable or preventable by ATR inhibition, wherein the compound or pharmaceutical composition is optionally combined with one or more chemotherapy or immunotherapy.

### Process for Preparing the Compounds of the Present Disclosure

The present disclosure also provides a process for the preparation of compounds of formula (I). General synthetic schemes for synthesizing the compounds of the present disclosure are exemplified below. For each reaction step, appropriate reaction conditions are known to those skilled in the art or can be routinely determined. The starting materials and reagents used in the preparation of these compounds are generally commercially available, or can be prepared by the methods below, the methods analogous to those given below, or the methods known in the art, unless otherwise specified. If necessary, the starting materials and intermediates in the synthetic schemes can be separated and purified by conventional techniques, including but not limited to filtration, distillation, crystallization, chromatography and the like. The materials can be characterized using conventional methods including physical constants and spectral data.

### Synthesis scheme 1:

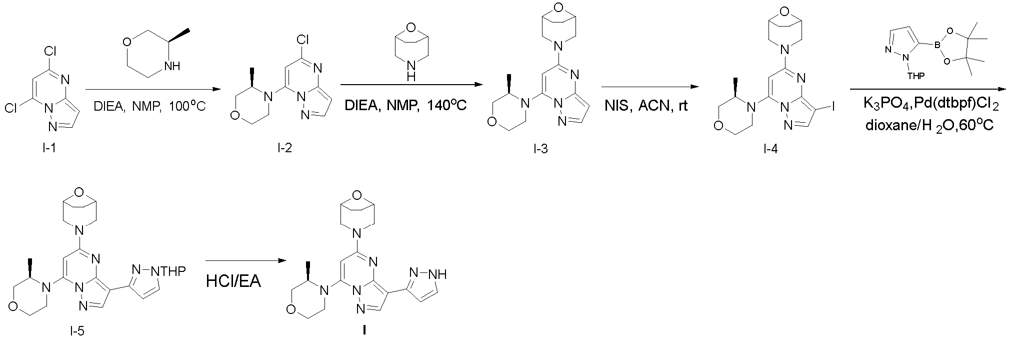

As illustrated in Scheme 1, compounds of the present disclosure can be synthesized by a process comprising the following steps:
Step 1: The compound of formula (I-1) is reacted under heating with an amine in the presence of a base such as DIEA, in a solvent such as NMP, to give the compound of formula (I-2);
Step 2: The compound of formula (I-2) is reacted under heating with an amine in the presence of a base such as DIEA, in a solvent such as NMP, to give the compound of formula (I-3);
Step 3: The compound of formula (I-3) is reacted with an iodination reagent such as NIS in a solvent such as CAN at room temperature, to give the compound of formula (I-4);
Step 4: The compound of formula (I-4) is subjected to Suzuki coupling under heating in the presence of a coupling agent such as Pd(dtbpf)Cl₂/H₃PO₄ in a solvent such as dioxane/water at room temperature, to give the compound of formula (I-5); and
Step 5: The compound of formula (I-5) is deprotected under the action of an acid, to give the compound of formula (I).

### Synthesis scheme 2:

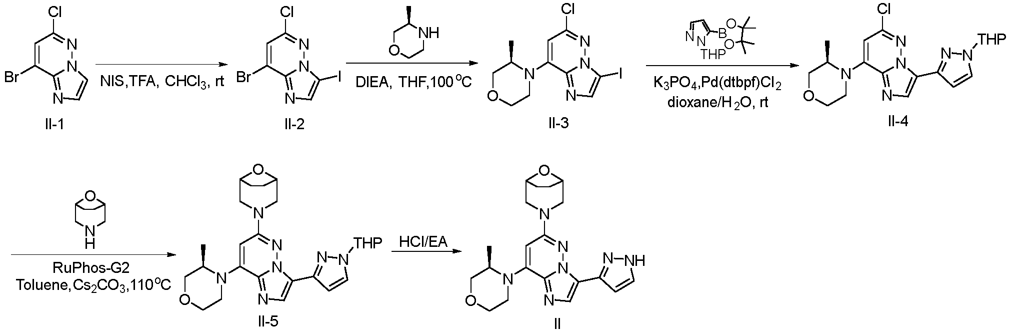

As illustrated in Scheme 2, compounds of the present disclosure can be synthesized by a process comprising the following steps:
Step 1: The compound of formula (II-1) is reacted with an iodination reagent such as NIS in the presence of an acid such as TFA in a solvent such as CHCl₃ at room temperature, to give the compound of formula (II-2);
Step 2: The compound of formula (II-2) is reacted under heating with an amine in the presence of a base such as DIEA, in a solvent such as THF, to give the compound of formula (II-3);
Step 3: The compound of formula (II-3) is subjected to Suzuki coupling under heating in the presence of a coupling agent such as Pd(dtbpf)Cl₂/H₃PO₄ in a solvent such as dioxane/water, to give the compound of formula (II-4);
Step 4: The compound of formula (II-4) is reacted under heating with an amine under the action of a catalyst such as RuPhos-G2 in the presence of a base such as cesium carbonate, in a solvent such as toluene, to give the compound of formula (II-4);
Step 5: The compound of formula (II-5) is deprotected under the action of an acid such as HCl, to give the compound of formula (II).

### Synthesis scheme 3:

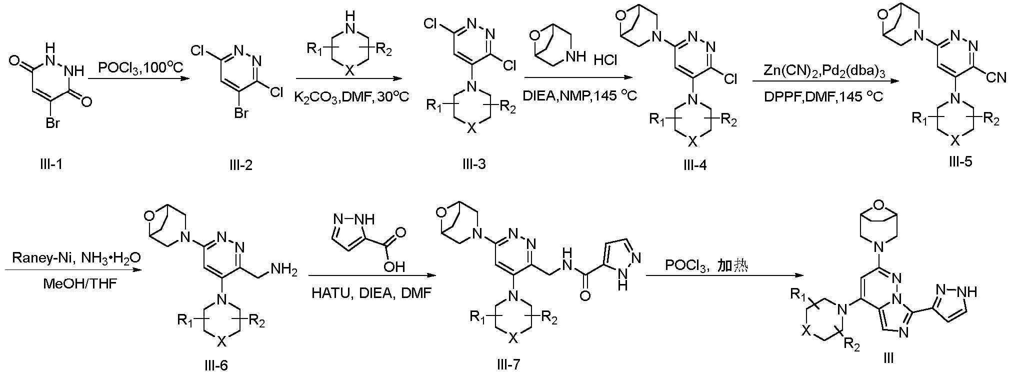

wherein R₁, R₂, X are as defined above for the general formula (I).

As illustrated in Scheme 3, compounds of the present disclosure can be synthesized by a process comprising the following steps:
Step 1: The compound of formula (III-1) is reacted with a chlorination reagent such as POCl₃ under heating, to give the compound of formula (III-2);
Step 2: The compound of formula (III-2) is reacted with an amine in the presence of a base such as K₂CO₃, in a solvent such as DMF at around room temperature, to give the compound of formula (III-3);
Step 3: The compound of formula (III-3) is reacted under heating with an amine in the presence of a base such as DIEA, in a solvent such as DMF, to give the compound of formula (III-4);
Step 4: The compound of formula (III-4) is reacted under heating with a cyanidating agent such as Zn(CN)₂ in the presence of a catalyst such as Pd₂(dba)₃/DPPF, in a solvent such as DMF, to give the compound of formula (III-5);
Step 5: The compound of formula (III-5) is subjected to reduction reaction in the presence of a catalyst such as Rainey nickel/ammonia in a solvent such as MeOH/THF, to give the compound of formula (III-6);
Step 6: The compound of formula (III-6) is subjected to condensation reaction in the presence of a condensation agent such as HATU/DIEA in a solvent such as DME, to give the compound of formula (III-7);
Step 7: The compound of formula (III-7) is cyclized in the presence of POCl₃ under heating such as at 100-150°C, to give a compound of formula (III).

### Synthesis scheme 4:

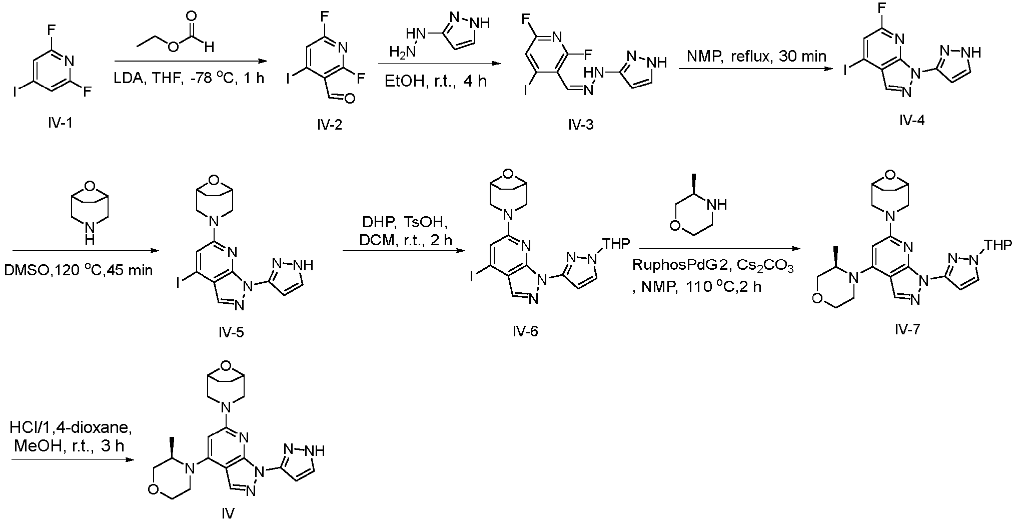

As illustrated in Scheme 4, compounds of the present disclosure can be synthesized by a process comprising the following steps:
Step 1: The compound of formula (IV-1) is reacted with a formate such as ethyl formate under the action of a base such as LDA in a solvent such as THF at low temperature, to give the compound of formula (IV-2);
Step 2: The compound of formula (IV-2) is reacted with 3-hydrazine-1H-pyrazole in a solvent such as ethanol at room temperature, to give the compound of formula (IV-3);
Step 3: The compound of formula (IV-3) is cyclized in a solvent such as NMP at an elevated temperature such as under reflux, to give the compound of formula (IV-4);
Step 4: The compound of formula (IV-4) is reacted under heating with an amine in a solvent such as DMSO, to give the compound of formula (IV-5);
Step 5: The compound of formula (IV-5) is protected with a protecting group such as DHP/TsOH in a solvent such as DCM at room temperature, to give the compound of formula (IV-6);
Step 6: The compound of formula (IV-6) is coupled under heating with an amine in the presence of a coupling agent such as Pd(dtbpf)Cl₂/CsCO₃ in a solvent such as NMP, to give the compound of formula (IV-7);
Step 7: The compound of formula (IV-6) is deprotected to give the compound of formula (IV).

The above synthetic schemes only exemplify the preparation of some compounds of the present disclosure. The compounds of the present disclosure, or stereoisomers, tautomers, stable isotopic derivatives, pharmaceutically acceptable salts or solvates thereof can be prepared by a variety of methods, including the methods given above, the methods given in the Examples or methods analogous to them, by those of ordinary skill in the art on the basis of the above-mentioned synthesis schemes in combination with conventional techniques in the art.

### EMBODIMENTS OF CARRYING OUT THE INVENTION

The technical solutions of the present disclosure are further illustrated below with reference to specific examples, but the protection scope of the present disclosure is not limited to them. All modifications or equivalent substitutions that do not depart from the concept of the present invention are included in the protection scope of the present disclosure.

The experimental methods without specified conditions in the following examples, are generally in accordance with the conventional conditions for this type of reaction, or in accordance with the conditions suggested by manufacturers. When the configuration of the chiral center is not indicated in the following examples, it means that it can exist as a single enantiomer or a mixture of enantiomers, and those skilled in the art can determine the stable and feasible isomeric forms of the compound. Percentages and parts are by weight unless otherwise specified. Unless otherwise specified, ratios of liquids are by volume, and temperatures used herein are in degrees Celsius.

Unless otherwise specified, the experimental materials and reagents used in the following examples can be obtained from commercial sources, prepared according to methods in the prior art, or prepared according to methods analogous to those disclosed in this application. Unless otherwise stated, the raw materials used in the present disclosure are all commercially available, which can be used directly without further purification, wherein the 5,7-dichloropyrazolo[1,5-A]pyrimidine used in the following examples was purchased from Shanghai Haohong Biomedical Technology Co., Ltd. (Leyan, CAS: 57489-77-7, Batch No.: Ld102321002), 8-bromo-6-chloroimidazo[1,2-B]pyridazine was purchased from Shaoyuan Technology (Shanghai) Co., Ltd. (CAS: 933190-51-3, Batch No.: J140-1157-27), 8-oxa-3-azabicyclo[3.2.1]octane hydrochloride was purchased from Shanghai Bide Medical Technology Co., Ltd. (CAS: 54745-74-3, Batch No.: BGX458).

Abbreviations used herein have the meanings commonly understood in the art unless clearly defined otherwise in the description. The meanings of the abbreviations used in the specification are listed below:

| | |
|---|---|
| Pd₂(dba)₃ | Tri(dibenzylideneacetone) dipalladium |
| Pd(dtbpf)Cl₂ | 1,1-bis(di-tert-butylphosphino)ferrocene dichloropalladium(ii) |
| DPPF | 1,1'-Ferrocenediyl-bis(diphenylphosphine) |
| RuPhosPdG2 | Chloro(2-dicyclohexylphosphino-2',6'-diisopropoxy-1,1'-biphenyl)(2'-amino-1,1'-biphenyl)palladium(II) |
| HATU | 2-(7-Azobenzotriazole)-*N,N,N',N'*,-tetramethylurea hexafluorophosphate |
| DIEA | N,N-Diisopropylethylamine |
| DCM | Dichloromethane |
| EA | Ethyl acetate |
| PE | Petroleum ether |
| NIS | N-Iodosuccinimide |
| DMF | N,N-Dimethylformamide |
| ACN | Acetonitrile |

| | |
|---|---|
| DHP | 3,4-Dihydro-2H-pyran |
| NMP | N-Methylpyrrolidone |
| LC-MS | Liquid Chromatography Mass Spectrometry |
| ESI | Electrospray ionization |
| *m*/*z* | Mass-to-charge ratio |
| HPLC | High Performance Liquid Chromatography |

### Synthesis Examples

In the preparation of the target compounds provided by the present disclosure, column chromatography was carried out with silica gel (300-400 mesh) produced by Rushan Sun Desiccant Co., Ltd.; thin-layer chromatography was carried out with GF254 (0.25mm); nuclear magnetic resonance spectroscopy (NMR) was carried out with Varian-400 nuclear magnetic resonance spectrometer; liquid chromatography/mass spectrometry (LC/MS) was carried out with Agilent TechnologiESI 6120 liquid chromatography/mass spectrometer.

In addition, all operations involving easily oxidizable or easily hydrolyzed raw materials are carried out under nitrogen protection.

Where the structure of the compound of the present disclosure is inconsistent with the name thereof, the structural formula generally controls, unless it can be determined from the context that the name of the compound is correct.

### Example 1: 3-(7-((R)-3-methylmorpholino)-3-(1H-pyrazol-3-yl)pyrazolo[1,5-a]pyrimidin-5-yl)-8-oxa-3-azabicyclo[3.2.1]octane

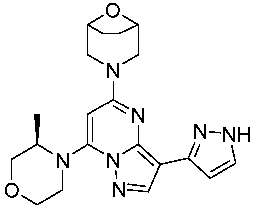

### Step 1: Synthesis of (R)-4-(5-chloropyrazolo[1,5-a]pyrimidin-7-yl)-3-methylmorpholine

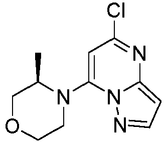

The solution of 5,7-dichloropyrazolo[1,5-a]pyrimidine (5.0g, 26.6 mmol), (*R*)-3-methylmorpholine (8.07 g, 79.8 mmol), DIEA (10.31 g, 79.8 mmol) in NMP (30.0 mL) was stirred at 100°C for 0.5 hour, then the reaction was stopped. After the reaction mixture was cooled to room temperature, DCM (150 mL) was added to dilute the solution, then washed with saturated saline (30.0 mL×5), dried over anhydrous sodium sulfate and filtered, the filtrate was concentrated under reduced pressure, and the obtained residue was separated and purified by column chromatography (PE:EA=3:1-1:2) to afford the target compound (6.50 g, yiled 96.7%, yellow solid). LC-MS (ESI) *m*/*z* 253.1 [M+H]⁺. ¹H NMR (400 MHz, CDCl₃) δ 8.01 (d, *J* = 2.3 Hz, 1H), 6.50 (d, *J* = 2.3 Hz, 1H), 6.04 (s, 1H), 5.22 - 5.08 (m, 1H), 4.13 - 3.95 (m, 2H), 3.89 - 3.80 (m, 1H), 3.80 - 3.75 (m, 1H), 3.74 - 3.68 (m, 1H), 3.67 - 3.62 (m, 1H), 1.30 (d, *J* = 6.8 Hz, 3H).

### Step 2: Synthesis of 3-(7-((R)-3-methylmorpholino)pyrazolo[1,5-a]pyrimidin-5-yl)-8-oxa-3-azabicyclo[3.2.1]octane

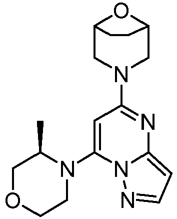

To a solution of (*R*)-4-(5-chloropyrazolo[1,5-a]pyrimidin-7-yl)-3-methylmorpholine (200 mg, 0.791 mmol) and 8-oxa-3-azabicyclo[3.2.1]octane hydrochloride (268 mg, 1.79 mmol) inNMP (15.0 mL) was added DIEA (306 mg, 2.37 mmol). The reaction mixture was reacted in a microwave reactor at 140°C for 2 hours under nitrogen protection, and the reaction was stopped. Then EA (35.0 mL) was added, washed with saturated saline (30.0 mL×3), dried over anhydrous sodium sulfate and filtered, the filtrate was concentrated under reduced pressure, and the obtained residue was separated and purified by column chromatography (PE: EA=3:1) to afford the target compound (120 mg, yield 46.0%, yellow oil). LC-MS (ESI) *m*/*z* 330.1[M+H]⁺.

### Step 3: Synthesis of 3-(3-iodo-7-((R)-3-methylmorpholino)pyrazolo[1,5-a]pyrimidin-5-yl)-8-oxa-3-azabicyclo[3.2.1]octane

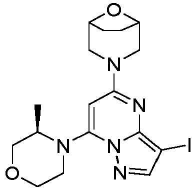

To a solution of -3-(7-((*R*)-3-methylmorpholino)pyrazolo[1,5-a]pyrimidin-5-yl)-8-oxa-3-azabicyclo[3.2.1]octane (120 mg, 0.364 mmol) in acetonitrile (5.00 mL) was added NIS (82 mg, 0.364 mmol), and the reaction mixture was stirred at room temperature for 0.5 hour, after the reaction was stopped, then water (10.0 mL) was added, extracted with EA(20.0 mL×3); the combined organic layers was washed with saturated saline (20.0 mL), dried over anhydrous sodium sulfate and filtered, the filtrate was concentrated under reduced pressure, and the obtained residue was separated and purified by column chromatography (PE: EA=3:1) to afford the target compound (100 mg, yield 60.3%, yellow solid). LC-MS (ESI) m/z 456.0 [M+H]⁺. ¹H NMR (400 MHz, CDCl₃) δ 7.85 (s, 1H), 5.38 (s, 1H), 5.10 (s, 1H), 4.51 (s, 2H), 4.08 - 3.91 (m, 3H), 3.90 - 3.80 (m, 2H), 3.74 (d, *J* = 11.6 Hz, 1H), 3.68 - 3.57 (m, 1H), 3.40 - 3.15 (m, 3H), 2.03 - 1.96 (m, 2H), 1.93 - 1.83 (m, 2H), 1.25 - 1.14 (m, 3H).

### Step 4: Synthesis of 3-(7-((R)-3-methylmorpholino)-3-(1-(tetrahydro-2H-pyran-2-yl)-1H-pyrazol-3-yl)pyrazolo[1,5-a]pyrimidin-5-yl)-8-oxa-3-azabicyclo[3.2.1]octane

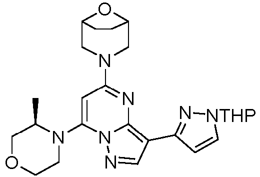

Under nitrogen protection, Pd(dtbpf)Cl₂ (14.0 mg, 0.0219 mmol) and potassium phosphate (139 mg, 0.658 mmol) were added to a solution of 3-(3-iodo-7-((*R*)-3-methylmorpholino)pyrazolo[1,5-a]pyrimidin-5-yl)-8-oxa-3-azabicyclo[3.2.1]octane (100 mg, 0.219 mmol) and 1-(tetrahydro-2*H-*pyran-2-yl)-3-(4,4,5,5-tetramethyl-1,3,2-dioxaborolan-2-yl)-1*H*-pyrazole (91.6 mg, 0.329 mmol) in 1,4-dioxane (5.00 mL) and water (1.00 mL), after the reaction mixture was stirred at 60°C for 16 hours, the reaction was stopped. Then water (30.0 mL) was added, extracted with EA (20.0 mL×3) and the combined organic layers were washed with saturated saline (25.0 mL), dried over anhydrous sodium sulfate and filtered, the filtrate was concentrated under reduced pressure, and the obtained residue was separated and purified by column chromatography (PE: EA=1:1) to afford the target compound (100 mg, yield 94.9%, yellow solid). LC- MS (ESI) m/z 480.1 [M+H]⁺.

### Step 5: 3 -(7-((R)-3-methylmorpholino)-3-(1H-pyrazol-3-yl)pyrazolo[1,5-a]pyrimidin-5-yl)-8-oxa-3-azabicyclo[3.2.1]octane

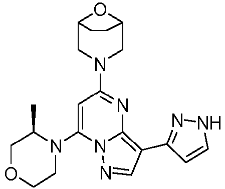

At room temperature, 3-(7-((*R*)-3-methylmorpholino)-3-(1-(tetrahydro-2*H*-pyran-2-yl)-1*H-*pyrazol-3-yl)pyrazolo[1,5-a]pyrimidin-5-yl)-8-oxa-3-azabicyclo[3.2.1]octane (100 mg, 0.208 mmol) and a solution of hydrochloric acid in ethyl acetate (5.00 mL, 3M) were added in an reaction flask, after the reaction mixture was stirred at room temperature for 2 hours, the reaction was stopped. The solution was concentrated under reduced pressure, and the obtained residue was separated and purified by reverse phase preparation chromatography (acetonitrile/water with 0.05% formic acid) to afford the target compound (75.8 mg, yield 91.9%, pale yellow solid). LC- MS (ESI) m/z 396.2 [M+H]⁺. ¹H NMR (400 MHz, DMSO-*d*₆) δ 12.62 (Brs, 1H), 8.23 (s, 1H), 7.64 - 7.47 (m, 1H), 6.77 - 6.63 (m, 1H), 5.77 (s, 1H), 5.15 - 5.07 (m, 1H), 4.49 - 4.44 (m, 2H), 4.19 - 4.10 (m, 1H), 4.09 - 4.01 (m, 1H), 3.96 - 3.91 (m, 1H), 3.85 - 3.80 (m, 1H), 3.70 - 3.63 (m, 2H), 3.57 - 3.50 (m, 1H), 3.40 - 3.34 (m, 1H), 3.14 - 3.07 (m, 2H), 1.88 - 1.81 (m, 2H), 1.79 - 1.72 (m, 2H), 1.10 (d, *J* = 6.8 Hz, 3H).

### Example 2: 3-(8-((R)-3-methylmorpholino)-3-(1H-pyrazol-3-yl)imidazo[1,2-b]pyridazin-6-yl)-8-oxa-3-azabicyclo[3.2.1]octane

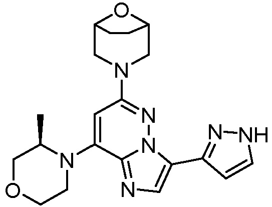

### Step 1: Synthesis of 8-bromo-6-chloro-3-iodoimidazo[1,2-b]pyridazine

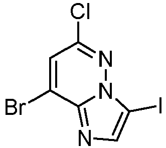

NIS (484 mg, 2.15 mmol) was added to a solution of 8-bromo-6-chloroimidazo[1,2-b]pyridazine (500 mg, 2.15 mmol) in trichloromethane (10.0 mL) and trifluoroacetic acid (1.00 mL), the reaction mixture was stirred at room temperature for 2 hours, then the reaction was stopped. Then saturated sodium bicarbonate solution (25.0 mL) was added, extracted with EA (30.0 mL×3) and the combined organic layers were washed with saturated sodium chloride aqueous solution (20.0 mL), dried over anhydrous sodium sulfate and filtered, the filtrate was concentrated under reduced pressure, and the obtained residue was separated and purified by column chromatography (PE: EA=5:1) to afford the target compound (500 mg, yield 64.8%, yellow solid). LC- MS (ESI) m/z 357.7, 359.7 [M+H]⁺. ¹H NMR (400 MHz, CDCl₃) δ 8.07 (s, 1H), 7.56 (s, 1H).

### Step 2: Synthesis of (R)-4-(6-chloro-3-iodoimidazo[1,2-b]pyridazin-8-yl)-3-methylmorpholine

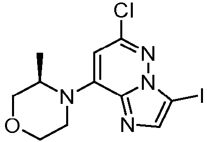

DIEA (541 mg, 4.19 mmol) was added to a solution of 8-bromo-6-chloro-3-iodoimidazo[1,2-b]pyridazine (500 mg, 1.40 mmol) and (R)-3-methylmorpholine (211 mg, 2.09 mmol) in THF (10 mL). The reaction mixture was stirred at 100°C for 2 hours under nitrogen protection, the reaction was stopped. Then EA (35.0 mL) was added, washed with saturated saline (35 mL×3), dried over anhydrous sodium sulfate and filtered, the filtrate was concentrated under reduced pressure, and the obtained residue was separated and purified by column chromatography (PE: EA=5:1) to afford the target compound (500 mg, yiled 94.6%, yellow oil). LC-MS (ESI) *m*/*z* 378.8 [M+H]⁺.

### Step 3: Synthesis of (3R)-4-(6-chloro-3-(1-(tetrahydro-2H-pyran-2-yl)-1H-pyrazol-3-yl)imidazo[1,2-b]pyridazin-8-yl)-3-methylmorpholine

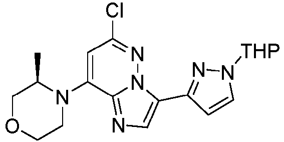

Under nitrogen protection, Pd(dtbpf)Cl₂ (67.3 mg, 0.105 mmol) and potassium phosphate (448 mg, 2.11 mmol) were added to a solution of (*R*)-4-(6-chloro-3-iodoimidazo[1,2-b]pyridazin-8-yl)-3-methylmorpholine (400 mg, 1.06 mmol) and 1-(tetrahydro-2*H*-pyran-2-yl)-3-(4,4,5,5-tetramethyl-1,3,2-dioxaborolan-2-yl)-1*H*-pyrazole (293 mg, 1.06 mmol) in 1,4-dioxane (10.0 mL) and water (2.00 mL), the reaction mixture was stirred at room temperature for 16 hours, the reaction was stopped. Then water (25.0 mL) was added, extracted with EA (30.0 mL×3) and the combined organic layers were washed with saturated saline (25.0 mL), dried over anhydrous sodium sulfate and filtered, the filtrate was concentrated under reduced pressure, and the obtained residue was separated and purified by column chromatography (PE: EA=1:1) to afford the target compound (320 mg, yield 75.1%, yellow solid). LC-MS (ESI) m/z 403.1 [M+H]⁺.

### Step 4: Synthesis of 3-(8-((R)-3-methylmorpholino)-3-(1-(tetrahydro-2H-pyran-2-yl)-1H-pyrazol-3-yl)imidazo[1,2-b]pyridazin-6-yl)-8-oxa-3-azabicyclo[3.2.1]octane

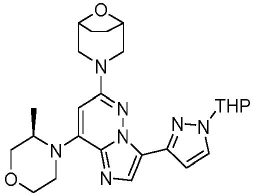

Under nitrogen protection, caesium carbonate (727 mg, 2.23 mmol) and 8-oxa-3-azabicyclic [3.2.1] octane hydrochloride (168 mg, 1.12 mmol) were added to a solution of (3*R*)-4-(6-chloro-3-(1-(tetrahydro-2*H*-pyran-2-yl)-1*H*-pyrazol-3-yl)imidazo[1,2-b]pyridazin-8-yl)-3-methylmorpholine (300 mg, 0.744 mmol) and RuPhosPdG2 (58.1 mg, 0.0744 mmol) in toluene (15.0 mL), the reaction mixture was stirred at 110°C for 16 hours, the reaction was stopped. Then water (25.0 mL) was added, extracted with EA (25.0 mL×3) and the combined organic layers were washed with saturated saline (25.0 mL), dried over anhydrous sodium sulfate and filtered, the filtrate was concentrated under reduced pressure, and the obtained residue was separated and purified by column chromatography (PE: EA=1:1) to afford the target compound (100 mg, yield 28.0%, yellow solid). LC-MS (ESI) m/z 480.2 [M+H]⁺.

### Step 5: Synthesis of 3-(8-((R)-3-methylmorpholino)-3-(1H-pyrazol-3-yl)imidazo[1,2-b]pyridazin-6-yl)-8-oxa-3-azabicyclo[3.2.1]octane

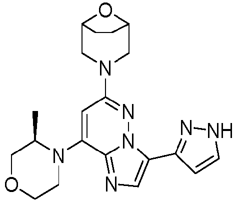

At room temperature, 3-(8-((*R*)-3-methylmorpholino)-3-(1-(tetrahydro-2*H*-pyran-2-yl)-1*H-*pyrazol-3-yl)imidazo[1,2-b]pyridazin-6-yl)-8-oxa-3-azabicyclo[3.2.1]octane (100 mg, 0.208 mmol) and hydrochloric acid solution in EA (10.0 mL, 3M) were added to a reaction flask, the reaction mixture was stirred at room temperature for 2 hours, the reaction was stoped. The reaction micture was concentrated under reduced pressure, the residue was purified by reverse phase preparative chromatography (acetonitrile/water with 0.05% formic acid) to afford the target compound (38.7 mg, yield 47.0%, white solid). LC- MS (ESI) m/z 396.3 [M+H]⁺. ¹H NMR (400 MHz, DMSO-*d*₆) δ 13.33 - 12.85 (m, 1H), 7.84 - 7.72 (m, 1H), 7.14 - 6.86 (m, 1H), 5.98 (s, 1H), 5.63 - 5.33 (m, 1H), 4.51 - 4.43 (m, 2H), 4.15 - 3.99 (m, 1H), 4.00 - 3.93 (m, 1H), 3.81 - 3.71 (m, 4H), 3.66 - 3.57 (m, 1H), 3.39 - 3.34 (m, 1H), 3.30 - 3.25 (m, 1H), 3.07 - 2.99 (m, 2H), 1.90 - 1.81 (m, 4H), 1.15 (d, *J* = 6.7 Hz, 3H).

### Example 3: 3-(4-((R)-3-methylmorpholino)-7-(1H-pyrazol-3-yl)imidazo[1,5-b]pyridazin-2-yl)-8-oxa-3-azabicyclo[3.2.1]octane

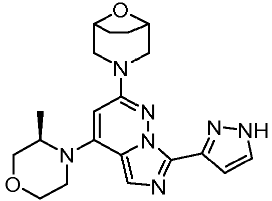

### Step 1: Synthesis of 4-bromo-3,6-dichloropyridazine

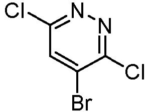

4-Bromo-1,2-dihydropyridazine-3,6-dione (5.50 g, 28.8 mmol) was dissolved in phosphorus oxychloride (35.0 mL), the mixture was heated to 100°C and reacted for 16 hours, the reaction was stopped. After the reaction mixture was cooled to room temperature, it was added to ice water slowly, adjusted to pH 7, then extracted with EA (100.0 mL×3), the organic layers was washed with saturated saline (50.0 mL×3), dried over anhydrous sodium sulfate and filtered, the filtrate was concentrated under reduced pressure, and the obtained residue was separated and purified by column chromatography (PE: EA=20:1-5:1) to afford the target compound (5.00 g, yield 76.2 %, pale yellow solid). LC-MS (ESI) *m*/*z* 227.0 [M+H]⁺.

### Step 2: Synthesis of (R)-4-(3,6-dichloropyridazin-4-yl)-3-methylmorpholine

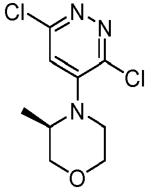

Potassium carbonate (6.59 g, 47.7 mmol) was added to a solution of 4-bromo-3,6-dichloropyridazine (2.73 g, 12.0 mmol) and (*R*) -3-methylmorpholine (2.40 g, 23.7 mmol) in DMF (37.0 mL), under nitrogen protection, the reaction mixture was stirred at 30°C for 16 hours, the reaction was stopped. Water (45.0 mL) was added, then extracted with EA (60.0 mL×3), the organic layers were washed with saturated saline (45.0 mL×3), dried over anhydrous sodium sulfate and filtered, the filtrate was concentrated under reduced pressure, and the obtained residue was separated and purified by column chromatography (PE: EA=5:1-2:1) to afford the target compound (2.36 g, yield 79.4%, white solid). LC-MS (ESI) m/z 248.1 [M+H]⁺.

### Step 3: Synthesis of 3-(6-chloro-5-((R)-3-methylmorpholino)pyridazin-3-yl)-8-oxa-3-azabicyclo[3.2.1]octane

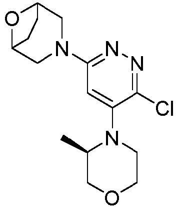

DIEA (2.26 g, 17.5 mmol) was added to a solution of (*R*)-4-(3,6-dichloropyridazin-4-yl)-3-methylmorpholine (1.44 g, 5.80 mmol) and 8-oxa-3-azabicyclic [3.2.1] octane hydrochloride (1.30 g, 8.68 mmol) in NMP (26.0 mL), after the reaction mixture was stirred at 145°C for 16 hours, the reaction was stopped. The reaction mixture was cooled to room temperature, water (25.0 mL) was added, then extracted with EA (30.0 mL×2), the organic layers were washed with saturated saline (25.0 mL×3), dried over anhydrous sodium sulfate and filtered, the filtrate was concentrated under reduced pressure, and the obtained residue was separated and purified by column chromatography (PE: EA=5:1-1:1) to afford the target compound (1.50 g, yield 79.6 %, brown solid). LC-MS (ESI) *m*/*z* 325.2 [M+H]⁺.

### Step 4: Synthesis of 6-(8-oxa-3-azabicyclo[3.2.1]octan-3-yl)-4-((R)-3-methylmorpholino)pyridazine-3-carbonitrile

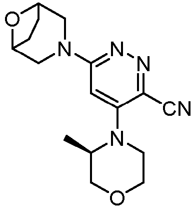

Zinc cyanide (1.08 g, 9.18 mmol), DPPF (510 mg, 0.918 mmol) and Pd2(dba)3 (421 mg, 0.459 mmol) were added to a mixture solution of 3-(6-chloro-5-((*R*)-3-methylmorpholino)pyridazin-3-yl)-8-oxa-3-azabicyclo[3.2.1]octane in water (8 drops) and DMF (40.0 mL). Under the protection of nitrogen, after the reaction mixture was stirred at 145°C overnight, the reaction was stopped. After the reaction mixture was cooled to room temperature, water (30.0 mL) was added, then extracted with EA (35.0 mL×3), the organic layers were washed with saturated saline (30.0 mL×3), dried over anhydrous sodium sulfate and filtered, the filtrate was concentrated under reduced pressure, and the obtained residue was separated and purified by column chromatography (PE: EA=5:1-1:2) to afford the target compound (1.10 g, yiled 76.0%, brown solid). LC-MS (ESI) *m*/*z* 316.3 [M+H]⁺.

### Step 5: Synthesis of (6-(8-oxa-3-azabicyclo[3.2.1]octan-3-yl)-4-((R)-3-methylmorpholino)pyridazin-3-yl)methanamine

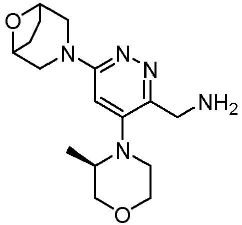

Ammonia (5.00 mL) and Rainey Nickel (-1.15 g) were added to a solution of 6-(8-oxa-3-azabicyclo[3.2.1]octan-3-yl)-4-((*R*)-3-methylmorpholino)pyridazine-3-carbonitrile (1.03 g, 3.27 mmol) in THF (30.0 mL). After three times of hydrogen replacement, the reaction mixture was reacted overnight at room temperature under a hydrogen atmosphere, and the reaction was stopped. The reaction mixture was filtered to remove solid residues, and the filtrate was concentrated under reduced pressure to obtain the target compound (1.00 g, yield 95.9%, brown solid). LC-MS (ESI) *m*/*z* 320.3 [M+H]⁺.

### Step 6: Synthesis of N-((6-(8-oxa-3-azabicyclo[3.2.1]octan-3-yl)-4-((R)-3-methylmorpholino)pyridazin-3-yl)methyl)-1H-pyrazole-5-carboxamide

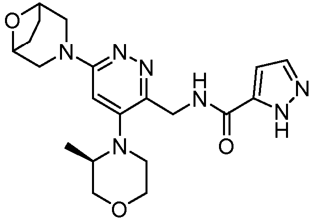

HATU (2.85 g, 7.48 mmol) and DIEA (1.19 g, 9.35 mmol) were added to a solution of (6-(8-oxa-3-azabicyclo[3.2.1]octan-3-yl)-4-((*R*)-3-methylmorpholino)pyridazin-3-yl)methanamine (1.20 g, 3.74 mmol) and 1*H*-pyrazole-5-carboxylic acid (505 mg, 4.49 mmol) in DMF (28.0 mL), the reaction mixture was reacted at room temperature for 4 hours, the reaction was stopped. The reaction micture was diluted with water (15.0 mL), then extracted with DCM: MeOH =10: 1 (20.0 mL×3), the combined organic layers were washed with saturated saline (15.0 mL×4), dried over anhydrous sodium sulfate and filtered, the filtrate was concentrated under reduced pressure, and the obtained residue was separated and purified by column chromatography (DCM: MeOH =50:1-10:1) to afford the target compound (660 mg, yield 42.7%, white solid). LC-MS (ESI) *m*/*z* 414.2 [M+H]⁺.

### Step 7: Synthesis of 3-(4-((R)-3-methylmorpholino)-7-(1H-pyrazol-3-yl)imidazo[1,5-b]pyridazin-2-yl)-8-oxa-3-azabicyclo[3.2.1]octane

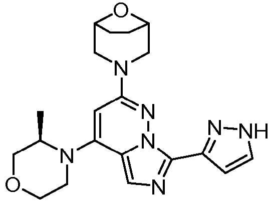

*N*-((6-(8-oxa-3-azabicyclo[3.2.1]octan-3-yl)-4-((*R*)-3-methylmorpholino)pyridazin-3-yl)methyl)-1*H*-pyrazole-5-carboxamide (660 mg, 1.60 mmol) was dissolved in phosphorous oxychloride solution (23.0 mL), after the reaction mixture was reacted at 145°C for 3 hours, the reaction was stopped. After the reaction mixture was cooled to room temperature, it was concentrated under reduced pressure to remove excess phosphorus oxychloride, the obtained residue was separated and purified by reverse phase preparative chromatography (acetonitrile/water with 0.05% formic acid) to afford the target compound (36.8 mg, yield 5.83%, white solid). LC-MS (ESI) *m*/*z* 396.3 [M+H]⁺. ¹H NMR (400 MHz, DMSO-*d₆*) δ 13.22 (brs, 1H), 7.76 - 7.54 (m, 2H), 7.11-7.04 (m, 1H), 5.79 (s, 1H), 4.50 - 4.42 (m, 2H), 4.38 - 4.30 (m, 1H), 3.99 - 3.92 (m, 1H), 3.85 - 3.75 (m, 3H), 3.74 - 3.68 (m, 1H), 3.65 - 3.57 (m, 1H), 3.50 - 3.38 (m, 2H), 3.11 - 3.00 (m, 2H), 1.90 - 1.78 (m, 4H), 1.15 (d, *J* = 6.6 Hz, 3H).

### Example 4: 3-(3-chloro-4-((R)-3-methylmorpholino)-7-(1H-pyrazol-3-yl)imidazo[1,5-b]pyridazin-2-yl)-8-oxa-3-azabicyclo[3.2.1]octane

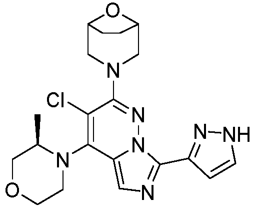

Phosphorus oxychloride (25.0 mL) was added to a solution of *N*-((6-(8-oxa-3-azabicyclic [3.2.1] octyl-3-yl)-4-((*R*)-3-methylmorpholine)pyrazine-3-yl)methyl)-1*H*-pyrazole 5-formamide (6.50 g, 15.72 mmol) in acetonitrile (100 mL), after the reaction mixture was reacted at 90°C for 1.5 hour, the reaction was stopped. After the reaction was cooled to room temperature, the reaction micture was slowly added dropwise to a saturated sodium bicarbonate solution at 0°C, then adjusted to pH 9 with saturated sodium bicarbonate solution at 0°C, then extracted with EA (200.0 mL×3), the combined organic layers were washed with saturated saline (30.0 mL×3), dried over anhydrous sodium sulfate and filtered, the filtrate was concentrated under reduced pressure, and the obtained residue was separated and purified by reverse phase preparation chromatography twice (acetonitrile/water with 0.05% Ammonia and acetonitrile/water with 0.05% formic acid) to afford the target compound (1.50 g, yield 22.2%, green solid). LC-MS (ESI) *m*/*z* 430.2 [M+H]⁺. ¹H NMR (400 MHz, MeOD-*d₄*) δ 7.74 (s, 1H), 7.64 (s, 1H), 7.17 (s, 1H), 4.50 - 4.40 (m, 2H), 4.10 - 4.01 (m, 1H), 4.00 - 3.91 (m, 2H), 3.84 - 3.71 (m, 3H), 3.66 - 3.60 (m, 1H), 3.42 - 3.35 (m, 2H), 3.34-3.32 (m, 1H), 2.97 (d, *J* = 11.8 Hz, 1H), 2.38 - 2.31 (m, 1H), 2.16 - 2.08 (m, 1H), 2.01 - 1.91 (m, 2H), 1.23 (d, *J* = 6.5 Hz, 3H).

### Example 5: 3-(4-((R)-3-methylmorpholino)-1-(1H-pyrazol-3-yl)-1H-pyrazolo[3,4-b]pyridin-6-yl)-8-oxa-3-azabicyclo[3.2.1]octane

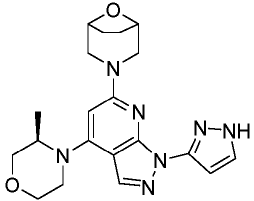

### Synthesis of intermediate 3-hydrazineyl-1H-pyrazole

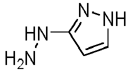

1*H*-pyrazol-3-amine (1.00 g, 12.0 mmol) was added to a solution of 6 mol/L aqueous hydrochloric acid (7 mL), the mixture reaction was cooled to -5°C, 1 mol/L aqueous solution of sodium nitrite (12 mL, 12.0 mmol) was added to the reaction mixture, the reaction mixture was stirred at room temperature for 2 hours. A solution of stannous chloride dihydrate (5.43 g, 24.1 mmol) in concentrated hydrochloric acid (24 mL) was added to the reaction mixture. After the reaction mixture was stirred at room temperature for 1 hour, the reaction mixture was concentrated under reduced pressure to afford crude target compound (9.00 g, crude, bright yellow solid).

### Step 1: Synthesis of 2,6-difluoro-4-iodonicotinaldehyde

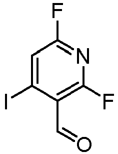

2,6-Difluoro-4-iodopyridine (5.00 g, 20.7 mmol) was added to a three-neck flask charged with anhydrous tetrahydrofuran (75 mL). The mixture was cooled to -78°C under nitrogen protection, lithium diisopropylamide (2 mol/L tetrahydrofuran solution) (12.5 mL, 24.9 mmol) was added, stirred for 1 hour, ethyl formate (2.31 g, 31.1 mmol) was added slowly. After further stirring at -78°C for 30 minutes, the TLC detected that the raw material was reacted completely, formic acid (1.91 g, 41.5 mmol) was added to the reaction solution, stirred at -78 °C for 10 minutes, EA (25 mL) was added, and then warmed to 0°C, water (30 mL) was added. Stirring wa stopped, additional EA (25 mL) was added for extraction, washed with saturated saline, dried over anhydrous sodium sulfate, concentrated under reduced pressure to remove the solvent, and the residue was separated and purified by silica column (PE: EA=5:1) to afford the target compound (3.54 g, yield 63.4%, solid).

### Step 2: Synthesis of 3-((2-(1H-pyrazol-3-yl)hydrazineylidene)methyl)-2,6-difluoro-4-iodopyridine

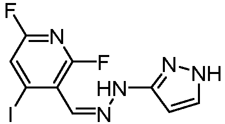

2,6-Difluoro-4-iodonicotinaldehyde (1.00 g, 3.72 mmol) and 3-hydrazineyl-1*H*-pyrazole (9.00 g, crude) were added to ethanol aqueous solution (20 mL, 95%). The mixture was stirred at room temperature for 3 hours under nitrogen protection. The solvent was removed under reduced pressure, EA (30 mL) was added to the residue and stirred well, a saturated sodium bicarbonate solution was added dropwise slowly into the well-stirred suspension and stirred vigorously to bring the mixture to basic, further stirred for 15 minutes, extracted with EA, washed with saturated saline, dried over anhydrous sodium sulfate, distilled under reduced pressure, and separated and purified by silica gel column (PE: EA=1:2) to afford the target compound (1.40 g, crude, yellow solid). LC-MS (ESI) *m*/*z:* 349.9 [M+H]⁺.

### Step 3: Synthesis of 6-fluoro-4-iodo-1-(1H-pyrazol-3-yl)-1H-pyrazolo[3,4-b]pyridine

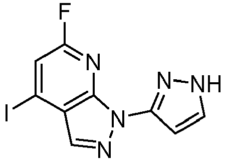

3-((2-(1*H*-pyrazol-3-yl)hydrazineylidene)methyl)-2,6-difluoro-4-iodopyridine (1.40 g, 4.01 mmol) was dissolved in NMP (19 mL). The reaction mixture was stirred and heated to reflux for 1 hour. After LC-MS monitored the reaction was completed, the reaction mixture was added dropwise to water (50 mL) to precipitate a brown solid, the mixture was stirred at room temperature for 10 minutes and then reduced to 0°C, further stirred for 10 minutes, the suspension was filtered with a suction funnel, the solids were collected, and the aqueous phase was extracted with EA (10 mL×2). The combined organic phases were washed with saturated saline, dried over anhydrous sodium sulfate, and filtered. The filtrate was concentrated under reduced pressure to obtain a crude product. The crude product was separated and purified by Pre-TLC (silica gel, PE: EA=1:1) to afford the target compound (838 mg, 2 step yield 68.5%, brown yellow solid). LC-MS (ESI) *m*/*:* 329.9 [M+H]⁺. ¹H NMR (400 MHz, DMSO-*d*₆) δ 13.09 (s, 1H), 8.27 (s, 1H), 7.93 (t, *J* = 2.0 Hz, 1H), 7.68 (d, *J* = 1.2 Hz, 1H), 6.66 (t, *J* = 2.2 Hz, 1H).

### Step 4: Synthesis of 3-(4-iodo-1-(1H-pyrazol-3-yl)-1H-pyrazolo[3,4-b]pyridin-6-yl)-8-oxa-3-azabicyclo[3.2.1]octane

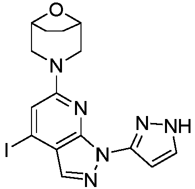

6-Fluoro-4-iodo-1-(1*H*-pyrazol-3-yl)-1*H*-pyrazolo[3,4-b]pyridine (538 mg, 1.63 mmol) was dissolved in DMSO (3 mL). The resulting mixture was stirred uniformly at room temperature under nitrogen protection, and 8-oxa-3-azabicyclic [3.2.1] octane hydrochloride (367 mg, 2.45 mmol) was added, the reaction solution was heated to 120°C and stirred for 45 minutes. After the reaction was completed as monitored by LC-MS, the reaction mixture was added dropwise to water (30 mL) to precipitate a brownish-yellow solid, and the mixed solution was stirred at room temperature for 10 minutes and then cooled to 0°C. Further stirring for 10 minutes, the suspension was filtered with a suction funnel, the solids were collected, and the aqueous phase was extracted with EA (10 mL×2). The residue was separated and purified by silica gel column (PE: EA=1:2) to afford the target compound (300 mg, yield 43.5%, yellow solid). LC-MS (ESI) *m*/*z:* 423.0 [M+H]⁺.

### Step 5: Synthesis of 3-(4-iodo-1-(1-(tetrahydro-2H-pyran-2-yl)-1H-pyrazol-3-yl)-1H-pyrazolo[3,4-b]pyridin-6-yl)-8-oxa-3-azabicyclo[3.2.1]octane

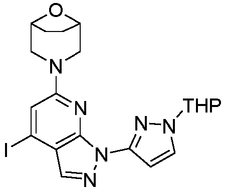

3-(4-iodo-1-(1*H*-pyrazol-3-yl)-1*H*-pyrazolo[3,4-b]pyridin-6-yl)-8-oxa-3-azabicyclo[3.2.1]octane (300 mg, 0.711 mmol), DHP(120 mg, 1.42 mmol), *P*-toluenesulfonic acid monohydrate (14 mg, 0.071mmol) were added sequentially to DCM (5 mL). The reaction mixture was stirred at room temperature for 16 hours. After the reaction was completed as monitored by LC-MS, the reaction mixture was added with water (10 mL), extracted with EA (10 mL×2). The organic layers were combined and washed with saturated saline, dried over anhydrous sodium sulfate, and filtered. The filtrate was concentrated under reduced pressure to obtain a crude product. The crude product was separated and purified by Pre-TLC (silica gel, PE: EA=1:2) to afford the target compound (330 mg, yield 91.7%, colorless oil). LC-MS (ESI) *m*/*z* 507.0 [M+H]⁺.

### Step 6: Synthesis of 3-(4-((R)-3-methylmorpholino)-1-(1-(tetrahydro-2H-pyran-2-yl)-1H-pyrazol-3-yl)-1H-pyrazolo[3,4-b]pyridin-6-yl)-8-oxa-3-azabicyclo[3.2.1]octane

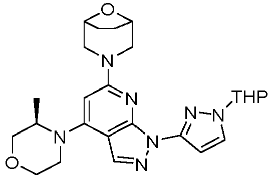

3-(4-Iodo-1-(1-(tetrahydro-2*H*-pyran-2-yl)-1*H*-pyrazol-3-yl)-1*H*-pyrazolo[3,4-b]pyridin-6-yl)-8-oxa-3-azabicyclo[3.2.1]octane (160 mg, 0.316 mmol), (*R*)-3-methylmorpholine (64 mg, 0.632 mmol), RuPhosPdG2 (25 mg, 0.032 mmol), cesium carbonate (309 mg, 0.948 mmol) were added to NMP (2 mL). The resulting mixture was stirred at 110°C for 2 hours under nitrogen protection, and after the reaction was completed as monitored by LC-MS, the reaction mixture was added with water (10 mL), extracted with EA(10 mL×2). The combined organic layers were washed with saturated saline, dried over anhydrous sodium sulfate, and filtered. The filtrate was concentrated under reduced pressure to obtain a crude product. The crude product was separated and purified by silica gel column (PE: EA=1:2) to afford the target compound (110 mg, yield 72.6%, colorless oil). LC-MS (ESI) *m*/*z:* 480.2 [M+H]⁺.

### Step 7: Synthesis of 3-(4-((R)-3-methylmorpholino)-1-(1H-pyrazol-3-yl)-1H-pyrazolo[3,4-b]pyridin-6-yl)-8-oxa-3-azabicyclo[3.2.1]octane

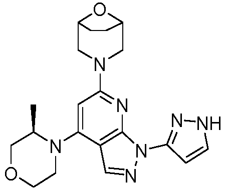

A 4 Mol/L solution of hydrochloric acid in 1,4-dioxane (2 mL) was added to a solution of 3-(4-((*R*)-3-methylmorpholino)-1-(1-(tetrahydro-2*H*-pyran-2-yl)-1*H*-pyrazol-3-yl)-1*H*-pyrazolo[3,4-b]pyridin-6-yl)-8-oxa-3-azabicyclo[3.2.1]octane (110 mg, 2.4 mmol) in methanol (2 mL). The resulting mixture was stirred at room temperature for 2 hours under nitrogen protection, and the solvent was removed under reduced pressure, the residue was separated and purified by Pre-HPLC to afford the target compound (12.2 mg, yield 13.4%, white solid). LC-MS (ESI) *m*/*z:* 396.2 [M+H]⁺. ¹H NMR (400 MHz, DMSO-*d*₆) δ 8.20 (s, 1H), 7.80 (d, *J* = 2.3 Hz, 1H), 6.80 (d, *J* = 2.2 Hz, 1H), 5.82 (s, 1H), 4.52 - 4.42 (m, 2H), 4.42 - 4.26 (m, 1H), 4.01 - 3.84 (m, 3H), 3.80 - 3.69 (m, 2H), 3.65 - 3.55 (m, 2H), 3.39 - 3.27 (m, 1H), 3.07 - 2.97 (m, 2H), 1.87 - 1.79 (m, 2H), 1.80 - 1.70 (m, 2H), 1.16 (d, *J* = 6.6 Hz, 3H).

### Example 6: 3-(4-(8-oxa-3-azabicydo[3.2.1]octan-3-yl)-7-(1H-pyrazol-3-yl)imidazo[1,5-b]pyridazin-2-yl)-8-oxa-3-azabicyclo[3.2.1]octane

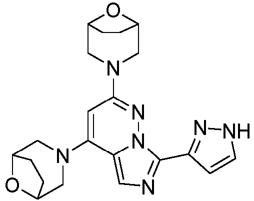

### Step 1: Synthesis of 3-(3,6-dichloropyridazin-4-yl)-8-oxa-3-azabicyclo[3.2.1]octane

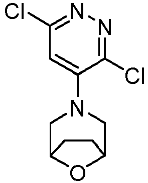

Potassium carbonate (1.21 g, 8.78 mmol) was added to a solution of 4-bromo-3,6-dichloropyridazine (1.00 g, 4.39 mmol) and 8-oxa-3-azabicyclic [3.2.1] octane hydrochloride (745 mg, 4.98 mmol) in DMF (15.0 mL). The reaction mixture was stirred at room temperature for 16 hours under nitrogen protection, and the reaction was stopped. Then the reaction mixture was added in EA (35.0 mL), washed with saturated saline (35.0 mL×3), dried over anhydrous sodium sulfate, filtered, concentrated under reduced pressure, and the resulting residue was separated and purified by silica gel column (PE: EA = 5:1) to obtain the target compound (700 mg, yiled 61.3%, yellow oil). LC-MS (ESI) *m*/*z* 260.1 [M+H]⁺.

### Step 2: Synthesis of 3-(6-(8-oxa-3-azabicyclo[3.2.1]octan-3-yl)-3-chloropyridazin-4-yl)-8-oxa-3-azabicyclo[3.2.1]octane

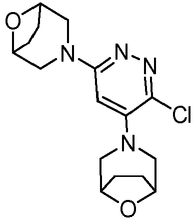

DIEA (1.04 g, 8.07 mmol) was added to a solution of 3-(3,6-dichloropyridazine-4-yl)-8-oxa-3-azabicyclic[3.2.1]octane (609 mg, 4.07 mmol) and 8-oxa-3-azabicyclic[3.2.1]octane hydrochloride (609 mg, 4.07 mmol) in NMP (15.0 mL). The reaction mixture was stirred at 145°C for 16 hours under nitrogen protection, and the reaction was stopped. Then the reaction mixture was added in EA (35.0 mL), washed with saturated saline (25.0 mL×3), dried over anhydrous sodium sulfate, filtered, concentrated under reduced pressure, and the resulting residue was separated and purified by column chromatography (PE: EA = 1:1) to obtain the target compound (550 mg, yield 60.7%, yellow solid). LC-MS (ESI) *m*/*z* 337.0 [M+H]⁺.

### Step 3: Synthesis of 4-(8-oxa-3-azabicyclo[3.2.1]octan-3-yl)-6-(8-oxa-3-azabicyclo[3.2.1]octan-3-yl)pyridazine-3-carbonitrile

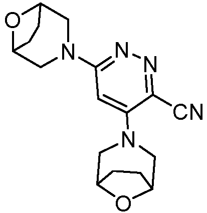

DPPF (181 mg, 0.326 mmol) and Pd₂(dba)₃ (149 mg, 0.163 mmol) were added to a solution of 3-(6-(8-oxa-3-azabicyclic[3.2.1]octane (550 mg, 1.63 mmol) and zinc cyanide (383 mg, 3.27 mmol) in DMF (10.0 mL). The reaction mixture was stirred at 140°C for 16 hours under nitrogen protection, and the reaction was stopped. Then the reaction mixture was added in EA (35.0 mL), washed with saturated saline (20.0 mL×3), dried over anhydrous sodium sulfate, filtered, concentrated under reduced pressure, and the resulting residue was separated and purified by column chromatography (PE: EA = 1:1) to obtain the target compound (350 mg, yield 65.5%, yellow solid). LC-MS (ESI) *m*/*z* 328.2 [M+H]⁺.

### Step 4: Synthesis of (4-(8-oxa-3-azabicyclo[3.2.1]octan-3-yl)-6-(8-oxa-3-azabicyclo[3.2.1]octan-3-yl)pyridazin-3-yl)methanamine

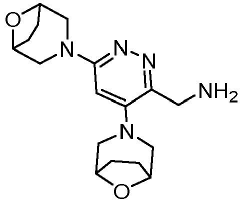

Rainey Nickel (-314 mg, 5.35 mmol) was added to a solution of 4-(8-oxa-3-azabicyclo[3.2.1]octan-3-yl)-6-(8-oxa-3-azabicyclo[3.2.1]octan-3-yl)pyridazine-3-carbonitrile (350 mg, 1.07 mmol) in tetrahydrofuran (10.0 mL). The reaction mixture was reacted at room temperature for 16 hours in a hydrogen environment, and the reaction was stopped. Then the reaction mixture was filtered, concentrated under reduced pressure, and the resulting residue was the target compound (300 mg, yield 84.7%, yellow oil). LC-MS (ESI) *m*/*z* 332.2 [M+H]⁺.

### Step 5: Synthesis of N-((4-(8-oxa-3-azabicyclo[3.2.1]octan-3-yl)-6-(8-oxa-3-azabicyclo[3.2.1]octan-3-yl)pyridazin-3-yl)methyl)-1H-pyrazole-5-carboxamide

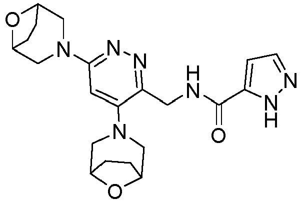

HATU (413 mg, 1.09 mmol) and DIEA (351 mg, 2.72 mmol) were added to a solution of (4-(8-oxa-3-azabicyclic[3.2.1]octane-3-yl)-6-(8-oxa-3-azabicyclic[3.2.1]octane-3-yl)pyridazine-3-yl) methylamine (300 mg, 0.905 mmol) and 1*H*-pyrazole-5-carboxylic acid (101 mg, 0.905 mmol) in tetrahydrofuran (10.0 mL). The reaction mixture was reacted at room temperature for 1 hour, and the reaction was stopped. The reaction solution was then filtered, concentrated under reduced pressure, and the resulting residue was separated and purified by column chromatography (PE: EA = 0:1) to obtain the target compound (200 mg, yield 51.9%, yellow oil). LC-MS (ESI) *m*/*z* 426.0 [M+H]⁺. ¹H NMR (400 MHz, DMSO-*d*₆) δ 13.27 (s, 1H), 8.30 (s, 1H), 7.83 (s, 1H), 6.65 (s, 1H), 6.46 (s, 1H), 4.60 (d, *J* = 5.1 Hz, 2H), 4.46 - 4.37 (m, 4H), 3.93 - 3.86 (m, 2H), 3.00 - 2.96 (m, 6H), 2.05 - 2.02 (m, 2H), 1.89 - 1.80 (m, 4H), 1.76 - 1.73 (m, 2H).

### Step 6: Synthesis of 3-(4-(8-oxa-3-azabicyclo[3.2.1]octan-3-yl)-7-(1H-pyrazol-3-yl)imidazo[1,5-b]pyridazin-2-yl)-8-oxa-3-azabicyclo[3.2.1]octane

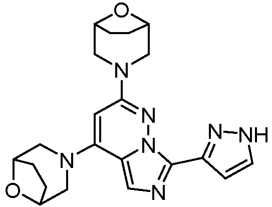

*N*-((4-(8-oxa-3-azabicyclo[3.2.1]octane-3-yl)-6-(8-oxa-3-azabicyclo[3.2.1]octane-3-yl) pyridazine-3-yl)methyl)-1*H*-pyrazole-5-formamide (200 mg, 0.470 mmol) and phosphorus oxychloride (10.0 mL) were added to a reaction flask at room temperature. The reaction mixture was reacted at 100°C for 1 hour and the reaction was stopped. The reaction solution was slowly added to a saturated aqueous sodium bicarbonate solution and adjusted to pH=7 with the saturated aqueous sodium bicarbonate solution, then the mixed solution was extracted with EA (20.0 mL×3), the organic phase was washed with saturated saline and dried over anhydrous sodium sulfate, filtered, concentrated under reduced pressure, and the resulting residue was purified by reverse phase preparative chromatography (acetonitrile/water with 0.05% formic acid) to give the target compound (21.3 mg, yield 11.1%, white solid). LC- MS (ESI) *m*/*z* 408.3 [M+H]⁺. ¹H NMR (400 MHz, DMSO-*d*₆) δ 13.39 - 12.85 (s, 1H), 7.72 - 7.55 (m, 2H), 7.07 (d, *J* = 1.6 Hz, 1H), 5.72 (s, 1H), 4.49-4.42 (m, 4H), 3.81 - 3.71 (m, 4H), 3.23 - 3.19 (m, 2H), 3.07 - 3.01 (m, 2H), 1.91 - 1.80 (m, 8H).

### Example 7: 3-(4-morpholino-7-(1H-pyrazol-3-yl)imidazo[1,5-b]pyridazin-2-yl)-8-oxa-3-azabicyclo[3.2.1]octane

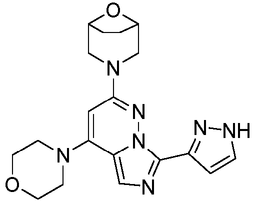

### Step 1: Synthesis of 4-(3,6-dichloropyridazin-4-yl)morpholine

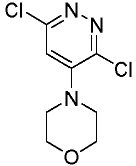

Potassium carbonate (1.69 g, 12.3 mmol) was added to a solution of 4-bromo-3,6-dichloropyridazine (700 mg, 3.07 mmol) and morpholine (535 mg, 6.14 mmol) in DMF (10.0 mL). The reaction mixture was stirred at 30°C for 12 hours under nitrogen protection, and the reaction was stopped. Then water (10.0 mL) was added, extracted with EA(15.0 mL×3), the organic phase was washed with saturated saline (15.0 mL×4), dried over anhydrous sodium sulfate, filtered, concentrated under reduced pressure, and the resulting residue was separated and purified by column chromatography (PE: EA = 5:1-1:1) to obtain the target compound (700 mg, yield 97.3%, white solid). LC-MS (ESI) *m*/*z* 234.0 [M+H]⁺.

### Step 2: Synthesis of 3-(6-chloro-5-morpholinopyridazin-3-yl)-8-oxa-3-azabicyclo[3.2.1]octane

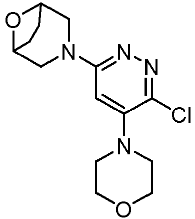

DIEA (1.16 g, 8.97 mmol) was added to a solution of 4-(3,6-dichloropyridazin-4-yl)morpholine (700 mg, 2.99 mmol) and 8-oxa-3-azabicyclic[3.2.1]octane hydrochloride (671 mg, 4.49 mmol) in NMP (10.0 mL). The reaction mixture was stirred at 145°C for 16 hours under nitrogen protection, and the reaction was stopped. Then the reaction mixture was cooled to room temperature, water was adde (15.0 mL), extracted with EA (20.0 mL×3), the combined organic layers were washed with water (15.0 mL×2) and saturated saline (15.0 mL×3), dried over anhydrous sodium sulfate, filtered, concentrated under reduced pressure, and the resulting residue was separated and purified by column chromatography (PE: EA = 3:1-1:2) to obtain the target compound (800 mg, yield 86.1%, white solid). LC-MS (ESI) *m*/*z* 311.2 [M+H]⁺.

### Step 3: Synthesis of 6-(8-oxa-3-azabicyclo[3.2.1]octan-3-yl)-4-morpholinopyridazine-3-carbonitrile

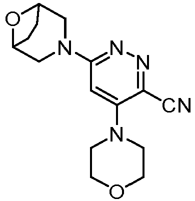

Zinc cyanide (453 mg, 3.86 mmol), DPPF (214 mg, 0.386 mmol) and Pd₂(dba)₃ (177 mg, 0.193 mmol) were added to a mixed solution of 3-(6-chloro-5-morpholinopyridazin-3-yl)-8-oxa-3-azabicyclo[3.2.1]octane (600 mg, 1.93 mmol) in water (5 drops) and DMF (10.0 mL). The reaction mixture was stirred at 145°C for 16 hours under nitrogen protection, and the reaction was stopped. Then the reaction mixture was cooled to room temperature, water was added (15.0 mL), extracted with EA (20.0 mL×3), the combined organic layers were washed with water (15.0 mL×2) and saturated saline (10.0 mL×2), dried over anhydrous sodium sulfate, filtered, concentrated under reduced pressure, and the resulting residue was separated and purified by column chromatography (PE: EA = 5:1-1:3) to obtain the target compound (360 mg, yield 61.9%, brown solid). LC-MS (ESI) *m*/*z* 302.3 [M+H]⁺.

### Step 4: Synthesis of (6-(8-oxa-3-azabicyclo[3.2.1]octan-3-yl)-4-morpholinopyridazin-3-yl)methanamine

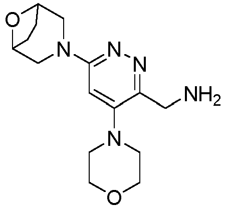

Ammonia (2.00 mL) and Rainey Nickel (~300 mg) were added to a solution of 6-(8-oxa-3-azabicyclo[3.2.1]octan-3-yl)-4-morpholinopyridazine-3-carbonitrile (350 mg, 1.07 mmol) in tetrahydrofuran (20.0 mL). After three times of hydrogen replacements, the reaction mixture was reacted at room temperature overnight in a hydrogen environment, and the reaction was stopped. Then the reaction mixture was filtered ro remove solid residue, concentrated under reduced pressure, to give the target compound (320 mg, yield 87.7%, brown solid). LC-MS (ESI) *m*/*z* 306.1 [M+H]⁺.

### Step 5: Synthesis of N-((6-(8-oxa-3-azabicyclo[3.2.1]octan-3-yl)-4-morpholinopyridazin-3-yl)methyl)-1H-pyrazole-5-carboxamide

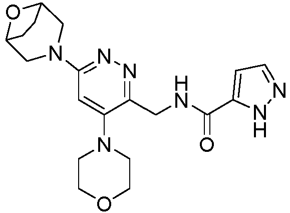

HATU (599 mg, 1.58 mmol) and DIEA (271 mg, 2.10 mmol) were added to a solution of (6-(8-oxa-3-azabicyclic[3.2.1]octane-3-yl)-4-morpholinopyridazine-3-yl) methylamine (320 mg, 1.05 mmol) and 1*H*-pyrazole-5-carboxylic acid (118 mg, 1.05 mmol) in tetrahydrofuran (13.0 mL), the reaction mixture was reacted at room temperature for 2 hours, and the reaction was stopped. The reaction mixture was concentrated under reduced pressure, and the resulting residue was separated and purified by column chromatography (DCM: methanol = 100:1-30:1) to give the target compound (170 mg, yield 40.6%, brown solid). LC-MS (ESI) *m*/*z* 400.3 [M+H]⁺.

### Step 6: Synthesis of 3-(3-chloro-4-morpholino-7-(1H-pyrazol-3-yl)imidazo[1,5-b]pyridazin-2-yl)-8-oxa-3-azabicyclo[3.2. 1]octane

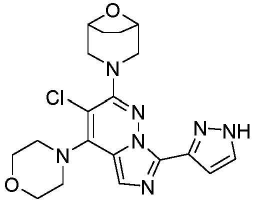

Phosphorus oxychloride (1.00 mL) was added to a solution of *N*-((6-(8-oxa-3-azabicyclic[3.2.1] octane-3-yl)-4-morpholinopyridine-3-yl)methyl)-1*H*-pyrazole-5-formamide (170 mg, 0.426 mmol) in acetonitrile (4.00 mL), the reaction mixture was reacted at 90°C for 1 hour, the reaction was stopped. After the reaction mixture was cooled to room temperature, the reaction solution was slowly added dropwise to the saturated sodium bicarbonate solution at 0 °C, then the pH was adjusted to 9 with the saturated sodium bicarbonate solution at 0°C, and extracted with EA (15.0 mL×3). The combined organic phases were dried over anhydrous sodium sulfate, filtered, and the filtrate was concentrated under reduced pressure. The resulting residue was separated and purified by a silica gel plate (DCM: methanol = 20:1) to obtain the target compound (80.0 mg, yield 45.2%, yellow solid). LC-MS (ESI) *m*/*z* 416.1 [M+H]⁺.

### Step 7: Synthesis of 3-(4-morpholino-7-(1H-pyrazol-3-yl)imidazo[1,5-b]pyridazin-2-yl)-8-oxa-3-azabicyclo[3.2.1]octane

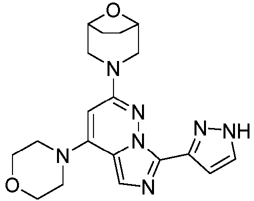

Palladium carbon (50.0 mg) was added to a mixed solution of 3-(3-chloro-4-morpholino-7-(1*H-*pyrazolyl-3-yl) imidazolo[1,5-b]pyridazine-2-yl)-8-oxa-3-azabicyclic[3.2.1]octane (80.0 mg, 0.192 mmol) in methanol (3.00 mL) and tetrahydrofuran (3.00 mL), after three times of hydrogen replacemet, the reaction mixture was reacted overnight at 50°C under a hydrogen atmosphere, and the reaction was stopped. The reaction mixture was filtered to remove solid residues, and the filtrate was concentrated under reduced pressure. The obtained residues were separated and purified by reversed-phase preparative chromatography (acetonitrile/water containing 0.05% formic acid) to obtain the target compound (4.60 mg, yield 6.27%, white solid). LC-MS (ESI) *m*/*z* 382.1 [M+H]⁺. ¹H NMR (400 MHz, MeOD-*d₄*) δ 7.83 - 7.64 (m, 1H), 7.60 - 7.43 (m, 1H), 7.25 - 7.02 (m, 1H), 5.84 (s, 1H), 4.55-4.45 (m, 2H), 3.94 - 3.79 (m, 6H), 3.51 - 3.43 (m, 4H), 3.23 - 3.14 (m, 2H), 2.03 - 1.93 (m, 4H).

### Example 8: 8-(2-(8-oxa-3-azabicyclo[3.2.1]octan-3-yl)-7-(1H-pyrazol-3-yl)imidazo[1,5-b]pyridazin-4-yl)-3-oxa-8-azabicyclo[3.2.1]octane

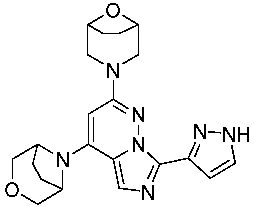

### Step 1: Synthesis of 8-(3,6-dichloropyridazin-4-yl)-3-oxa-8-azabicyclo[3.2.1]octane

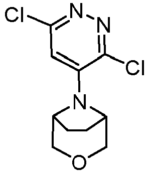

Potassium carbonate (1.82 g, 13.2 mmol) was added to a solution of 4-bromo-3,6-dichloropyridazine (1.00 g, 4.39 mmol) and 3-oxa-8-azabicyclic[3.2.1]octane hydrochloride (985 mg, 6.58 mmol) in DMF (15.0 mL). The reaction mixture was stirred at room temperature under nitrogen protection for 16 hours and the reaction was stopped. The reaction solution was then diluted with EA (45.0 mL), washed with saturated saline (35.0 mL×3), dried over anhydrous sodium sulfate, filtered, concentrated under reduced pressure, and the resulting residue was separated and purified by column chromatography (PE: EA = 5:1) to obtain the target compound (1.00 g, yield 87.6%, yellow oil). LC-MS (ESI) *m*/*z* 259.9 [M+H]⁺, 261.9 [M+2+H]⁺. ¹H NMR (400 MHz, CDCl₃) δ 6.72 (s, 1H), 4.44 - 4.33 (m, 2H), 3.91 - 3.82 (m, 2H), 3.72 - 3.62 (m, 2H), 2.21 - 2.12 (m, 2H), 2.06 - 1.95 (m, 2H).

### Step 2: Synthesis of 8-(6-(8-oxa-3-azabicyclo[3.2.1]octan-3-yl)-3-chloropyrazin-4-yl)-3-oxa-8-azabicyclo[3.2.1]octane

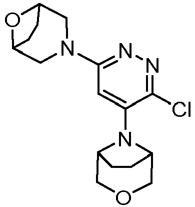

DIEA (1.49 g, 11.5 mmol) was added to a solution 8-(3,6-dichloropyridazine-4-yl)-3-oxa-8-azabicyclic[3.2.1]octane (1.00 g, 3.84 mmol) and 8-oxa-3-azabicyclic[3.2.1]octane hydrochloride (1.15 g, 7.69 mmol) in NMP (15.0 mL). The reaction mixture was stirred at 145°C under nitrogen protection for 16 hours and the reaction was stopped. The reaction solution was then diluted with EA (45.0 mL), washed with saturated saline (35.0 mL×3), dried over anhydrous sodium sulfate, filtered, concentrated under reduced pressure, and the resulting residue was separated and purified by column chromatography (PE: EA = 1:1) to obtain the target compound (770 mg, yield 59.5%, yellow solid). LC-MS (ESI) *m*/*z* 337.0 [M+H]⁺.

### Step 3: Synthesis of 6-(8-oxa-3-azabicyclo[3.2.1]octan-3-yl)-4-(3-oxa-8-azabicyclo[3.2.1]octan-8-yl)pyridin-3-carbonitrile

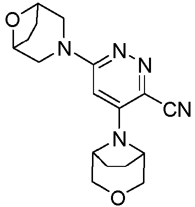

DPPF (247 mg, 0.445 mmol) and Pd₂(dba)₃ (204 mg, 0.222 mmol) were added to a solution of 8-(6-(8-oxa-3-azabicyclo[3.2.1]octan-3-yl)-3-chloropyrazin-4-yl)-3-oxa-8-azabicyclo[3.2.1]octa-ne (750 mg, 2.23 mmol)) and zinc cyanide (523 mg, 4.45 mmol) in DMF (15.0 mL). The reaction mixture was stirred at 140°C for 16 hours under nitrogen protection, and the reaction was stopped. The reaction solution was then diluted with EA (35.0 mL), washed with saturated saline (30.0 mL× 3), dried over anhydrous sodium sulfate, filtered, concentrated under reduced pressure, and the resulting residue was separated and purified by column chromatography (PE: EA = 1:1) to give the target compound (600 mg, 82.3% yield, yellow solid). LC-MS (ESI) *m*/*z* 328.0 [M+H]⁺.

### Step 4: Synthesis of (6-(8-oxa-3-azabicyclo[3.2.1]octan-3-yl)-4-(3-oxa-8-azabicyclo[3.2.1]octan-8-yl)pyridazin-3-yl)methanamine

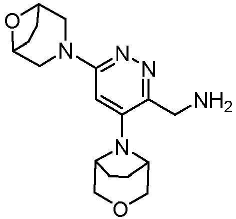

To a solution of 6-(8-oxa-3-azabicyclo[3.2.1]octan-3-yl)-4-(3-oxa-8-azabicyclo[3.2.1]octan-8-yl)pyridin-3-carbonitrile (600 mg, 1.83 mmol) in tetrahydrofuran (10.0 mL) was added Rainey nickel (~ 323 mg, 5.50 mmol). The reaction mixture was reacted at room temperature for 16 hours under a hydrogen atmosphere, and the reaction was stopped. The reaction solution was then filtered, concentrated under reduced pressure, and the resulting residue was the target compound (480 mg, 79.0% yield, yellow oil). LC-MS (ESI) *m*/*z* 332.1 [M+H]⁺.

### Step 5: Synthesis of N-((6-(8-oxa-3-azabicyclo[3.2.1]octan-3-yl)-4-(3-oxa-8-azabicyclo[3.2.1]octan-8-yl)pyridazin-3-yl)methyl)-1H-pyrazole-5-carboxamide

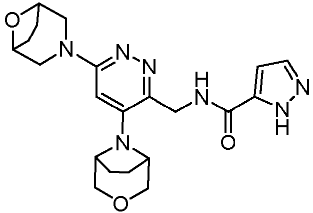

To a solution of (6-(8-oxa-3-azabicyclo[3.2.1]octan-3-yl)-4-(3-oxa-8-azabicyclo[3.2.1]octan-8-yl)pyridazin-3-yl)methanamine (480 mg, 1.45 mmol) and 1*H*-pyrazole-5-carboxylic acid (146 mg, 1.30 mmol) in tetrahydrofuran (10.0 mL) was added HATU (661 mg, 1.74 mmol) and DIEA (562 mg, 4.34 mmol). The reaction mixture was reacted at room temperature for 1 hour, and the reaction was stopped. The reaction solution was then filtered, concentrated under reduced pressure, and the resulting residue was separated and purified by column chromatography (PE: EA = 0:1) to obtain the target compound (400 mg, yield 64.9%, yellow oil). LC-MS (ESI) *m*/*z* 426.0 [M+H]⁺.

### Step 6: Synthesis of 8-(2-(8-oxa-3-azabicyclo[3.2.1]octan-3-yl)-7-(1H-pyrazol-3-yl)imidazo[1,5-b]pyridazin-4-yl)-3-oxa-8-azabicyclo[3.2.1]octane

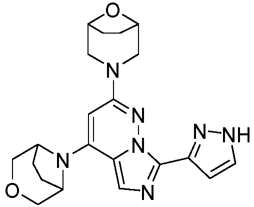

At room temperature, *N-*((6-(8-oxa-3-azabicyclic[3.2.1]octane-3-yl)-4-(3-oxa-8-azabicyclic [3.2.1]octane-8-yl)pyridazine-3-yl)methyl)-1*H*-pyrazole-5-formamide (100 mg, 0.235 mmol) and phosphorus oxychloride (5.00 mL) were added to a reaction flask. The reaction mixture was reacted at 100°C for 1 hour and the reaction was stopped. The reaction solution was added to a saturated aqueous sodium bicarbonate solution to adjust pH=7 under an ice bath, then the mixed solution was extracted with EA (20.0 mL×3), the organic phase was washed with saturated saline and dried over anhydrous sodium sulfate, filtered, concentrated under reduced pressure, concentrated under reduced pressure, the resulting residue was purified by reverse phase preparative chromatography (acetonitrile/water with 0.05% formic acid) to give the target compound (10.5 mg, 11.0% yield, white solid). LC-MS (ESI) *m*/*z* 408.3 [M+H]⁺. ¹H NMR (400 MHz, MeOD-*d₄*) δ 7.70 (s, 1H), 7.58 (s, 1H), 7.13 (s, 1H), 5.78 (s, 1H), 4.56 - 4.48 (m, 4H), 3.90 - 3.80 (m, 4H), 3.67 - 3.61 (m, 2H), 3.23 - 3.14 (m, 2H), 2.18 - 2.09 (m, 4H), 2.01 - 1.94 (m, 4H).

### Example 9: 3-(4-((R)-2-methylpiperazin-1-yl)-7-(1Hpyrazol-3-yl)imidazo[1,5-b]pyridazin-2-yl)-8-oxa-3-azabicyclo[3.2.1]octane formate

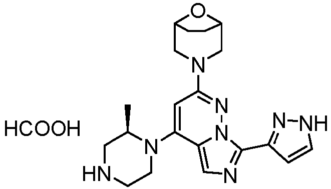

### Step 1: Synthesis of tert-butyl (R)-4-(3,6-dichloropyridazin-4-yl)-3-methylpiperazine-1-carboxylate

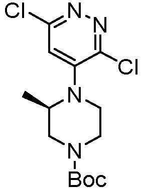

To a solution of 4-bromo-3,6-dichloropyridazine (2.00 g, 8.78 mmol) and (R)-3-methylpiperazine-1-carboxylate (2.64 g, 13.2 mmol) in DMF (25.0 mL) was added potassium carbonate (2.43 g, 17.6 mmol). The reaction mixture was stirred at room temperature under nitrogen protection for 16 hours and the reaction was stopped. The reaction solution was then diluted with EA (55.0 mL), washed with saturated saline (35.0 mL×3), dried over anhydrous sodium sulfate, filtered, concentrated under reduced pressure, and the resulting residue was separated and purified by column chromatography (PE: EA = 5:1) to obtain the target compound (1.60 g, yield 52.5%, yellow solid). LC-MS (ESI) *m*/*z* 346.9 [M+H]⁺. ¹H NMR (400 MHz, CDCl₃) δ 6.83 (s, 1H), 4.25 - 4.08 (m, 2H), 3.89 - 3.79 (m, 1H), 3.46 - 3.28 (m, 2H), 3.23 - 3.02 (m, 2H), 1.48 (s, 9H), 1.12 (d, *J=* 6.6 Hz, 3H).

### Step 2: Synthesis of tert-butyl (3R)-4-(6-(8-oxa-3-azabicyclo[3.2.1]octan-3-yl)-3-chloropyridazin-4-yl)-3-methylpiperazine-1-carboxylate

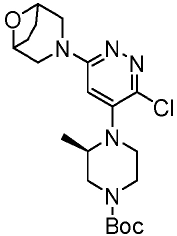

DIEA (1.79 g, 13.8 mmol) was added to a solution of tert-butyl (A)-4-(3,6-dichloropyridazin-4-yl)-3-methylpiperazine-1-carboxylate (1.60 g, 4.61 mmol) and 8-oxa-3-azabicyclic[3.2.1]octane hydrochloride (1.38 g, 9.22 mmol) in NMP (15.0 mL). The reaction mixture was stirred at 145°C for 16 hours under nitrogen protection, and the reaction was stopped. The reaction solution was then diluted with EA (55.0 mL), washed with saturated saline (35.0 mL×3), dried over anhydrous sodium sulfate, filtered, concentrated under reduced pressure, and the resulting residue was separated and purified by column chromatography (PE: EA = 1:1) to obtain the target compound (1.20 g, yield 61.4%, yellow solid). LC-MS (ESI) *m*/*z* 424.2 [M+H]⁺. ¹H NMR (400 MHz, CDCl₃) δ 6.11 (s, 1H), 4.54 - 4.49 (m, 2H), 4.14 - 3.93 (m, 2H), 3.86 - 3.81 (m, 1H), 3.77 - 3.65 (m, 2H), 3.44 - 3.31 (m, 2H), 3.26-3.10 (m, 3H), 2.90-2.84 (m, 1H), 2.03 - 1.95 (m, 2H), 1.90-1.82 (m, 2H), 1.48 (s, 9H), 1.02 (d, *J*= 6.5 Hz, 3H).

### Step 3: Synthesis of tert-butyl (3R)-4-(6-(8-oxa-3-azabicyclo[3.2.1]octan-3-yl)-3-cyanopyridazin-4-yl)-3-methylpiperazine-1-carboxylate

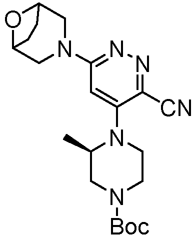

DPPF (314 mg, 0.556 mmol) and Pd₂(dba)₃ (259 mg, 0.283 mmol) were added to a solution of tert-butyl (3R)-4-(6-(8-oxa-3-azabicyclo[3.2.1]octan-3-yl)-3-chloropyridazin-4-yl)-3-methylpiperazine-1-carboxylate (1.20 g, 2.83 mmol) and zinc cyanide (665 mg, 5.66 mmol) in DMF (20.0 mL). The reaction mixture was stirred at 140°C for 16 hours under nitrogen protection and the reaction was stpped. The reaction solution was then diluted with EA (45.0 mL), washed with saturated saline (30.0 mL×3), dried over anhydrous sodium sulfate, filtered, concentrated under reduced pressure, and the resulting residue was separated and purified by column chromatography (PE: EA = 1:1) to give the target compound (1.00 g, 85.2% yield, yellow solid). LC-MS (ESI) *m*/*z* 415.0 [M+H]⁺.

### Step 4: Synthesis of tert-butyl (3R)-4-(3-(aminomethyl)-6-(8-oxa-3-azabicyclo[3.2.1]octan-3-yl)pyridazin-4-yl)-3-methylpiperazine-1-carboxylate

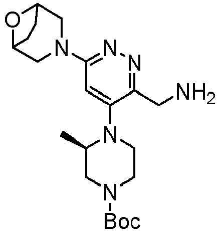

To a solution of tert-butyl (3R)-4-(6-(8-oxa-3-azabicyclo[3.2.1]octan-3-yl)-3-cyanopyridazin-4-yl)-3-methylpiperazine-1-carboxylate (500 mg, 1.21 mmol) in tetrahydrofuran (10.0 mL) was added Rainey nickel (-212 mg, 3.62 mmol). The reaction mixture was reacted at room temperature for 16 hours under a hydrogen atmosphere, and the reaction was stopped. The reaction solution was then filtered and concentrated under reduced pressure, and the resulting residue was the target compound (400 mg, 79.2% yield, yellow oil). LC-MS (ESI) *m*/*z* 419.1 [M+H]⁺.

### Step 5: Synthesis of tert-butyl (3R)-4-(3-((1H-pyrazole-5-carboxamido)methyl)-6-(8-oxa-3-azabicyclo[3.2.1]octan-3-yl)pyridin-4-yl)-3-methylpiperazine-1-carboxylate

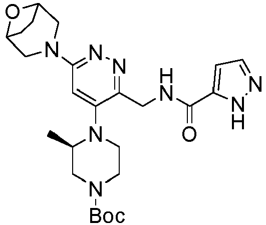

HATU (436 mg, 1.15 mmol) and DIEA (371 mg, 2.87 mmol) were added to a solution of *(3R)* -4-(3-(aminomethyl)-6-(8-oxa-3-azabicyclic[3.2.1]octane-3-yl)pyridazine-4-yl)-3-methylpiperazine-1-carboxylate (400 mg, 0.956 mmol) and 1*H*-pyrazole-5-carboxylic acid (96.4 mg, 0.860 mmol) in tetrahydrofuran (10.0 mL). The reaction mixture was reacted at room temperature for 1 hour, and the reaction was stopped. The reaction solution was then filtered, concentrated under reduced pressure, and the resulting residue was separated and purified by column chromatography (PE: EA = 0:1) to obtain the target compound (300 mg, yield 61.2%, yellow oil). LC-MS (ESI) m/z 513.1 [M+H]⁺.

### Step 6: Synthesis of 3-(4-((R)-2-methylpiperazin-1-yl)-7-(1H-pyrazol-3-yl)imidazo[1,5-b]pyridazin-2-yl)-8-oxa-3-azabicyclo[3.2.1]octane formate

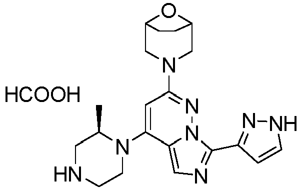

At room temperature, tert-butyl (3R)-4-(3-((1H-pyrazole-5-carboxamido)methyl)-6-(8-oxa-3-azabicyclo[3.2.1]octan-3-yl)pyridin-4-yl)-3-methylpiperazine-1-carboxylate (100 mg, 0.195 mmol) and phosphorus oxychloride (5.00 mL) were added to a reaction flask. The reaction mixture was reacted at 100°C for 1 hour and the reaction was stopped. The reaction solution was added to a saturated aqueous sodium bicarbonate solution to adjust pH=7 under an ice bath, then the mixed solution was extracted with EA (20.0 mL×3), the organic phase was washed with saturated saline and dried over anhydrous sodium sulfate, filtered, concentrated under reduced pressure, the resulting residue was purified by reverse phase preparative chromatography (acetonitrile/water with 0.05% formic acid) to give the target compound (8.50 mg, 11.0% yield, white solid). LC-MS (ESI) *m*/*z* 395.3 [M+H]⁺. ¹H NMR (400 MHz, MeOD-*d₄*) δ 7.72 (s, 1H), 7.59 (s, 1H), 7.14 (s, 1H), 5.93 (s, 1H), 4.54 - 4.49 (m, 2H), 3.93 - 3.86 (m, 4H), 3.84 - 3.81 (m, 1H), 3.73 - 3.59 (m, 2H), 3.53 - 3.44 (m, 2H), 3.23 - 3.17 (m, 2H), 2.01 - 1.93 (m, 4H), 1.32 (d, *J=* 6.9 Hz, 3H).

### Example 10: 3-(4-(piperazin-1-yl)-7-(1H-pyrazol-3-yl)imidazo[1,5-b]pyridazin-2-yl)-8-oxa-3-azabicyclo[3.2.1]octane formate

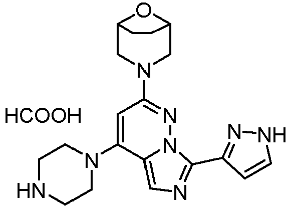

### Step 1: Synthesis of tert-butyl 4-(3,6-dichloropyridazin-4-yl)piperazine-1-carboxylate

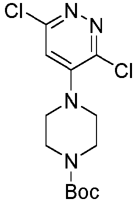

To solution of 4-bromo-3,6-dichloropyridazine (1.00 g, 4.39 mmol) and tert-butylpiperazine-1-carboxylate (1.23 g, 6.58 mmol) in DMF (12.0 mL) was added potassium carbonate (1.82 g, 13.2 mmol), the reaction mixture was stirred at room temperature for 16 hours, and the reaction was stopped. Water was then added (35.0 mL), extracted with EA (30.0 mL×3), the combined organic phases were washed with saturated saline (30.0 mL), dried over anhydrous sodium sulfate, filtered, and concentrated under reduced pressure. The resulting residue was separated and purified by column chromatography (PE: EA = 5:1) to obtain the target compound (1.50 g, 100% yield, yellow solid). LC-MS (ESI) *m*/*z* 334.5 [M+2+H]⁺.

### Step 2: Synthesis of tert-butyl 4-(6-(8-oxa-3-azabicyclo[3.2.1]octan-3-yl)-3-chloropyridazin-4-yl)piperazine-1-carboxylate

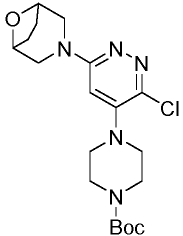

DIEA (1.72 g, 13.3 mmol) was added to a solution of tert-butyl 4-(3,6-dichloropyridazin-4-yl)piperazine-1-carboxylate (1.48 g, 4.44 mmol) and 8-oxa-3-azabicyclic[3.2.1]octane hydrochloride (997 mg, 6.66 mmol) of in NMP (20.0 mL), the reaction mixture was stirred at 145°C for 16 hours, the reaction was stopped. Then water (35.0 mL) was added, extracted with EA (30.0 mL×3), the combined organic phases were washed with saturated saline (30.0 mL), dried over anhydrous sodium sulfate, filtered, and concentrated under reduced pressure. The resulting residue was separated and purified by a chromatography plate (PE: EA = 5:1) to obtain the target compound (1.20 g, yiled 65.9%, yellow solid). LC-MS (ESI) *m*/*z* 410 [M+H]⁺.

### Step 3: Synthesis of tert-butyl 4-(6-(8-oxa-3-azabicyclo[3.2.1]octan-3-yl)-3-cyanopyridazin-4-yl)piperazine-1-carboxylate

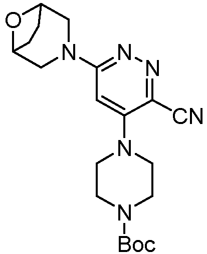

DPPF (297 mg, 0.537 mmol) and Pd₂(dba)₃ (246 mg, 0.268 mmol) were added to a solution of tert-butyl 4-(6-(8-oxa-3-azabicyclo[3.2.1]octan-3-yl)-3-chloropyridazin-4-yl)piperazine-1-carboxylate (1.10 g, 2.68 mmol) and zinc cyanide (630 mg, 5.37 mmol) in DMF (20.0 mL). The reaction mixture was stirred at 140°C for 16 hours under nitrogen protection and the reaction was to stopped. The reaction solution was then diluted with EA (30.0 mL), washed with saturated saline (25.0 mL×3), dried over anhydrous sodium sulfate, filtered, concentrated under reduced pressure, and the resulting residue was separated and purified by column chromatography (PE: EA = 1:1) to give the target compound (600 mg, yield 55.8%, yellow solid). LC-MS (ESI) *m*/*z* 401.1 [M+H]⁺. ¹H NMR (400 MHz, CDCl₃) δ 5.89 (s, 1H), 4.54 - 4.50 (m, 2H), 4.01 - 3.93 (m, 2H), 3.64 - 3.61 (m, 4H), 3.35 - 3.26 (m, 6H), 2.04 - 1.99 (m, 2H), 1.85 - 1.79 (m, 2H), 1.48 (s, 9H).

### Step 4: Synthesis of tert-butyl 4-(3-(aminomethyl)-6-(8-oxa-3-azabicyclo[3.2.1]octan-3-yl)pyridazin-4-yl)piperazine-1-carboxylate

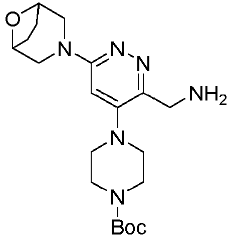

To a solution of tert-butyl 4-(6-(8-oxa-3-azabicyclo[3.2.1]octan-3-yl)-3-cyanopyridazin-4-yl)piperazine-1-carboxylate (600 mg, 1.50 mmol) in tetrahydrofuran (12.0 mL) was added Rainey nickel (439 mg, 7.49 mmol) and ammonia (5 drops). The reaction mixture was reacted at room temperature for 16 hours under a hydrogen atmosphere, and the reaction was stopped. The reaction was then filtered, concentrated under reduced pressure, and the resulting residue was the target compound (530 mg, 87.5% yield, yellow oil). LC-MS (ESI) *m*/*z* 405.1 [M+H]⁺.

### Step 5: Synthesis of tert-butyl 4-(3-((1H-pyrazole-5-carboxamido)methyl)-6-(8-oxa-3-azabicyclo[3.2.1]octan-3-yl)pyridazin-4-yl)piperazine-1-carboxylate

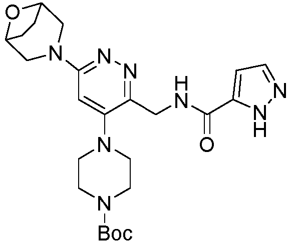

Under nitrogen protection, HATU (541 mg, 1.42 mmol) and DIEA (306 mg, 2.37 mmol) were added to a solution of tert-butyl 4-(3-(aminomethyl)-6-(8-oxa-3-azabicyclo[3.2.1]octan-3-yl)pyridazin-4-yl)piperazine-1-carboxylate (480 mg, 1.19 mmol) and 1*H*-pyrazole-5-carboxylic acid (119 mg, 1.07 mmol) in tetrahydrofuran (10.0 mL), the reaction mixture was stirred at room temperature for 1 hour, the reaction was stopped. Then water (25.0 mL) was added, extracted with EA (20.0 mL×3), the combined organic phases were washed with saturated saline (20.0 mL), dried over anhydrous sodium sulfate, filtered, and concentrated under reduced pressure. The resulting residue was isolated and purified by chromatography plate (DCM: methanol = 10:1) to give the target compound (500 mg, 84.5% yield, yellow solid). LC-MS (ESI) *m*/*z* 499.2 [M+H]⁺.

### Step 6: Synthesis of 3-(4-(piperazin-1-yl)-7-(1H-pyrazol-3-yl)imidazo[1,5-b]pyridazin-2-yl)-8-oxa-3-azabicyclo[3.2.1]octane formate

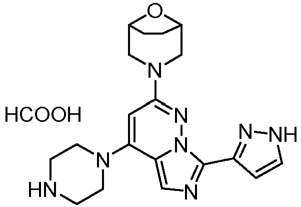

*Tert*-butyl 4-(3-((1*H*-pyrazole-5-carboxamido)methyl)-6-(8-oxa-3-azabicyclo[3.2.1]octan-3-yl)pyridazin-4-yl)piperazine-1-carboxylate (150 mg, 0.301 mmol) and phosphorus oxychloride (15.0 mL) were added to a reaction flask at room temperature. The reaction mixture was reacted at 100°C for 1 hour and the reaction was stopped. The reaction solution was added to a saturated aqueous sodium bicarbonate solution to pH=7 under an ice bath, and then the mixed solution was extracted with EA (25.0 mL×3), the organic phase was washed with saturated saline and dried over anhydrous sodium sulfate and filtered, and the organic phase was concentrated under reduced pressure. The resulting residue was purified by reverse phase preparative chromatography (acetonitrile/water with 0.05% formic acid) to give the target compound (5.66 mg, 4.94% yield, white solid). LC-MS (ESI) *m*/*z* 381.0 [M+H]⁺. ¹H NMR (400 MHz, MeOD-*d₄*) δ 7.72 (s, 1H), 7.55 (s, 1H), 7.14 (s, 1H), 5.94 (s, 1H), 4.53 - 4.48 (m, 2H), 3.88 - 3.82 (m, 2H), 3.70 - 3.63 (m, 4H), 3.41 - 3.36 (m, 4H), 3.23 - 3.17 (m, 2H), 2.01 - 1.92 (m, 4H).

### Example 11: 3-(4-((R)-2,4-dimethylpiperazin-1-yl)-7-(1H-pyrazol-3-yl)imidazo[1,5-b]pyridazin-2-yl)-8-oxa-3-azabicyclo[3.2.1]octane formate

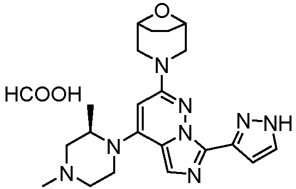

### Step 1: Synthesis of 6-(8-oxa-3-azabicyclo[3.2.1]octan-3-yl)-4-((R)-2-methylpiperazin-1-yl)pyridazine-3-carbonitrile

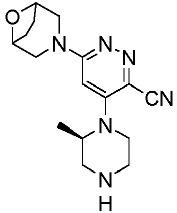

At room temperature, 6-(8-oxa-3-azabicyclo[3.2.1]octan-3-yl)-4-((R)-2-methylpiperazin-1-yl)pyridazine-3-carbonitrile (500 mg, 1.21 mmol) and hydrochloric acid in EA (10.0 mL, 3M) were added to a reaction flask, the reaction mixture was stirred at room temperature for 2 hours, the reaction was stopped. Concentration under reduced pressure provided the resulting residue as the target compound (400 mg, 94.5% yield, white solid). LC-MS (ESI) *m*/*z* 315.1 [M+H]⁺.

### Step 2: Synthesis of 6-(8-oxa-3-azabicyclo[3.2.1]octan-3-yl)-4-((R)-2,4-dimethylpiperazin-1-yl)pyridazine-3-carbonitrile

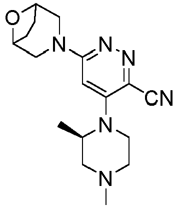

Sodium borohydride (115 mg, 3.82 mmol) was added to a solution of 6-(8-oxa-3-azabicyclic [3.2.1]octane-3-yl)-4-((*R*)-2-methylpiperazine-1-yl)pyridazine-3-carboxonitrile (400 mg, 1.27 mmol) and paraformaldehyde (144 mg, 3.82 mmol) in methanol (10.0 mL), and the reaction mixture was stirred at room temperature for 1 hour, and the reaction was stopped. Water was then added (20.0 mL), extracted with EA (20.0 mL×3), the combined organic phases were washed with saturated saline (20.0 mL), dried over anhydrous sodium sulfate, filtered, and concentrated under reduced pressure. The resulting residue was separated and purified by column chromatography (PE: EA = 1:1) to obtain the target compound (400 mg, 95.7% yield, white solid). LC-MS (ESI) *m*/*z* 329.2 [M+H]⁺.

### Step 3: Synthesis of (6-(8-oxa-3-azabicyclo[3.2.1]octan-3-yl)-4-((R)-2,4-dimethylpiperazin-1-yl)pyridazin-3-yl)methanamine

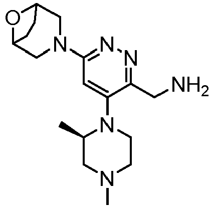

To a solution of 6-(8-oxa-3-azabicyclo[3.2.1]octan-3-yl)-4-((R)-2,4-dimethylpiperazin-1-yl)pyridazine-3-carbonitrile (400 mg, 1.22 mmol) in tetrahydrofuran (10.0 mL) was added Rainey nickel (214 mg, 3.65 mmol). The reaction mixture was reacted at room temperature for 16 hours under a hydrogen atmosphere and the reaction was stopped. The reaction solution was then filtered, concentrated under reduced pressure, and the resulting residue was the target compound (300 mg, 74.1% yield, yellow oil). LC-MS (ESI) *m*/*z* 333.1 [M+H]⁺.

### Step 4: Synthesis of N-((6-(8-oxa-3-azabicyclo[3.2.1]octan-3-yl)-4-((R)-2,4-dimethylpiperazin-1-yl)pyridazin-3-yl)methyl)-1H-pyrazole-5-carboxamide

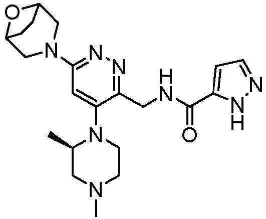

HATU (412 mg, 1.08 mmol) and DIEA (350 mg, 2.71 mmol) were added to a solution of (6-(8-oxa-3-azabicyclo[3.2.1]octan-3-yl)-4-((*R*)-2,4-dimethylpiperazin-1-yl)pyridazin-3-yl)methanamine (300 mg, 0.902 mmol) and 1*H*-pyrazole-5-carboxylic acid (91.0 mg, 0.812 mmol) in tetrahydrofuran (10.0 mL). The reaction mixture was reacted at room temperature for 1 hour, and the reaction was stopped. The reaction solution was then filtered, concentrated under reduced pressure, and the resulting residue was separated and purified by column chromatography (EA = 100%) to obtain the target compound (200 mg, yield 51.9%, yellow oil). LC-MS (ESI) *m*/*z* 427.2 [M+H]⁺.

### Step 5: Synthesis of 3-(3-chloro-4-((R)-2,4-dimethylpiperazin-1-yl)-7-(1H-pyrazol-3-yl)imidazo[1,5-b]pyridazin-2-yl)-8-oxa-3-azabicyclo[3.2.1]octane

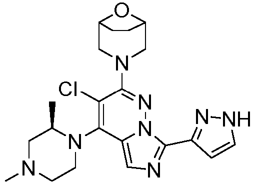

*N-*((6-(8-oxa-3-azabicyclo[3.2.1]octan-3-yl)-4-((*R*)-2,4-dimethylpiperazin-1-yl)pyridazin-3-yl)methyl)-1*H*-pyrazole-5-carboxamide (100 mg, 0.234 mmol) and phosphorus oxychloride (5.00 mL) were added to a reaction flask at room temperature. The reaction mixture was reacted at 100°C for 1 hour and the reaction was stopped. Under an ice bath, the reaction solution was added to a saturated aqueous sodium bicarbonate solution and adjust to pH=7, then the mixed solution was extracted with EA (20.0 mL×3), the organic phase was washed with saturated saline and dried over anhydrous sodium sulfate, filtered, concentrated under reduced pressure, and the resulting residue was the target compound (100 mg, 96.3% yield, white solid). LC-MS (ESI) *m*/*z* 443.3 [M+H]⁺.

### Step 6: Synthesis of 3-(4-((R)-2,4-dimethylpiperazin-1-yl)-7-(1H-pyrazol-3-yl)imidazo[1,5-b]pyridazin-2-yl)-8-oxa-3-azabicyclo[3.2. 1]octane formate

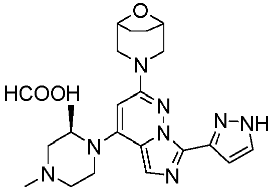

Palladium carbon (53.5 mg, 5%) was added to a solution of 3-(3-chloro-4-((R)-2,4-dimethylpiperazin-1-yl)-7-(1*H*-pyrazol-3-yl)imidazo[1,5-b]pyridazin-2-yl)-8-oxa-3-azabicyclo-[3.2.1]octane (100 mg, 0.226 mmol) in methanol (10.0 mL). The reaction mixture was reacted at 60°C for 16 hours under a hydrogen atmosphere and the reaction was stopped. The reaction solution was then filtered, concentrated under reduced pressure, and the resulting residue was purified by reverse phase preparative chromatography (acetonitrile/water with 0.05% formic acid) to give the target compound (12.5 mg, 13.6% yield, white solids). LC-MS (ESI) *m*/*z* 409.3 [M+H]⁺. ¹H NMR (400 MHz, MeOD-*d₄*) δ 7.72 (s, 1H), 7.63 - 7.44 (m, 1H), 7.26 - 6.99 (m, 1H), 5.84 (s, 1H), 4.52 - 4.49 (m, 2H), 3.95 - 3.75 (m, 3H), 3.63 - 3.49 (m, 2H), 3.22 - 3.11 (m, 3H), 3.06 - 2.98 (m, 1H), 2.81 - 2.73 (m, 1H), 2.62 - 2.54 (m, 1H), 2.52 (s, 3H), 2.00 - 1.91 (m, 4H), 1.28 (d, *J=* 6.4 Hz, 3H).

### Example 12: 3-(6-(-8-oxa-3-azabicyclo[3.2.1]octan-3-yl)-1-(1H-pyrazol-3-yl)-1H-pyrazolo[3,4-b]pyridin-4-yl)-8-oxa-3-azabicyclo[3.2.1]octane

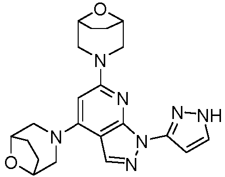

### Synthesis of intermediate 6-fluoro-4-iodo-1-(1H-pyrazole-3-yl)-1H-pyrazolo[3,4-b] pyridine may refer to Example 5

### Step 1: Synthesis of 3-(4-iodo-1-(1H-pyrazol-3-yl)-1H-pyrazolo[3,4-b]pyridin-6-yl)-8-oxa-3-azabicyclo[3.2.1]octane

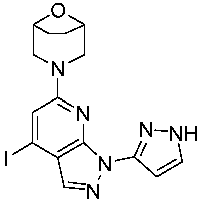

6-Fluoro-4-iodo-1-(1*H*-pyrazole-3-yl)-1*H*-pyrazolo[3,4-b] pyridine (718 mg, 2.18 mmol) was dissolved in dimethyl sulfoxide (7 mL). The resulting mixture was stirred uniformly at room temperature under nitrogen protection, 8-oxa-3-azabicyclic[3.2.1]octane hydrochloride (326 mg, 2.18 mmol) was added, the reaction solution was heated to 120°C and stirred for 45 minutes, after the reaction was completed as monitored by LC-MS, the reaction mixture was added dropwise to water (30 mL) to precipitate a brownish-yellow solid, the mixed solution was stirred at room temperature for 10 minutes and then reduced to 0°C, further stirred for 10 minutes, the suspension was filtered with a suction funnel, the solids were collected, the aqueous phase was extracted with EA (10 mL×2), and the obtained crude product was separated and purified by silica gel column (PE: EA=1:2) to give the target compound (215 mg, yield 23.3%, yellow solid). LC-MS (ESI) *m*/*z:* 423.0 [M+H]⁺.

### Step 2: Synthesis of 3-(4-iodo-1-(1-(tetrahydro-2H-pyran-2-yl)-1H-pyrazol-3-yl)-1H-pyrazolo[3,4-b]pyridin-6-yl)-8-oxa-3-azabicyclo[3.2.1]octane

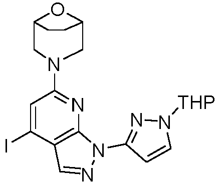

3-(4-Iodo-1-(1*H*-pyrazol-3-yl)-1*H*-pyrazolo[3,4-b]pyridin-6-yl)-8-oxa-3-azabicyclo[3.2.1]-octane (215 mg, 0.509 mmol), DHP (86 mg, 1.02 mmol), p-toluenesulfonic acid monohydrate (10 mg, 0.051 mmol) were sequentially added to DCM (2 mL). The reaction mixture was stirred at room temperature overnight. After the reaction was completed as monitored by LC-MS, water (5 mL) was added to the reaction mixture, extracted with EA (10 mL×2). The combined organic phases were washed with saturated saline, dried over anhydrous sodium sulfate, and filtered. The filtrate was concentrated under reduced pressure to obtain a crude product. The crude product was separated and purified by a preparative plate (silica gel, EA: PE = 1:2) to obtain the target compound (210 mg, yield 81.4%, yellow solid). LC-MS (ESI) *m*/*z:* 507.1 [M+H]⁺.

### Step 3: Synthesis of 3-(6-(-8-oxa-3-azabicyclo[3.2.1]octan-3-yl)-1-(1-(tetrahydro-2H-pyran-2-yl)-1H-pyrazol-3-yl)-1H-pyrazolo[3,4-b]pyridin-4-yl)-8-oxa-3-azabicyclo[3 .2.1]octane

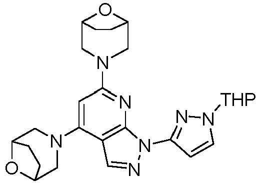

3-(4-Iodo-1-(1-(tetrahydro-2*H*-pyran-2-yl)-1*H*-pyrazol-3-yl)-1*H*-pyrazolo[3,4-b]pyridin-6-yl)-8-oxa-3-azabicyclo[3.2.1]octane (210 mg, 0.415 mmol), 8-oxa-3-azabicyclic[3.2.1]octane hydrochloride (124 mg, 0.829 mmol), RuPhosPdG2 (32 mg, 0.042mmol), cesium carbonate (405 mg, 1.24 mmol) were added to 1,4-dioxane (2 mL). The resulting mixture was stirred at 110°C for 2 hours under nitrogen protection. After the reaction was completed as monitored by LC-MS, water (10 mL) was added to the reaction mixture and extracted with EA (10 mL×2). The combined organic phases were washed with saturated saline, dried over anhydrous sodium sulfate, and filtered. The filtrate was concentrated under reduced pressure to obtain a crude product, which was separated and purified by silica gel column (PE: EA = 1:1) to obtain the target compound (55 mg, yield 27.0%, brown oil). LC-MS (ESI) *m*/*z:* 492.2 [M+H]⁺.

### Step 4: Synthesis of 3-(6-(-8-oxa-3-azabicyclo[3.2.1]octan-3-yl)-1-(1H-pyrazol-3-yl)-1H-pyrazolo[3,4-b]pyridin-4-yl)-8-oxa-3-azabicyclo[3 .2.1]octane

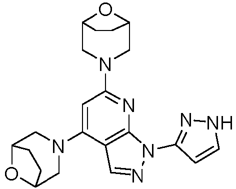

To a solution of 3-(6-(-8-oxa-3-azabicyclo[3.2.1]octan-3-yl)-1-(1-(tetrahydro-2H-pyran-2-yl)-1*H*-pyrazol-3-yl)-1*H*-pyrazolo[3,4-b]pyridin-4-yl)-8-oxa-3-azabicyclo[3.2.1]octane (55 mg, 0.112 mmol) in methanol (2 mL) was addde 4 mol/L 1,4-dioxane hydrochloric acid solution (2 mL). The resulting mixture was stirred at room temperature for 2 hours under nitrogen protection, the solvent was removed under reduced pressure, and the residue was separated and purified by a pre-HPLC column to obtain the target compound (3.3 mg, yield 7.24%, white solid). LC-MS (ESI) *m*/*z*: 408.2 [M+H]⁺. ¹H NMR (400 MHz, MeOD) δ 8.31-8.23 (m, 1H), 7.90-7.77 (m, 1H), 6.80 (s, 1H), 4.63 - 4.49 (m, 4H), 3.92 (d, *J* = 12.2 Hz, 2H), 3.81 (d, *J =* 11.9 Hz, 2H), 3.48 (d, J=11.8 Hz, 2H), 3.35 (d, *J=* 12.0 Hz, 2H), 2.09 - 1.99 (m, 4H), 1.99 - 1.89 (m, 4H).

### Example 13: 3-(7-(8-oxa-3-azabicyclo[3.2.1]octan-3-yl)-1-methyl-3-(1H-pyrazol-3-yl)-1H-pyrazolo[4,3-b]pyridin-5-yl)-8-oxa-3-azabicyclo[3.2.1]octane

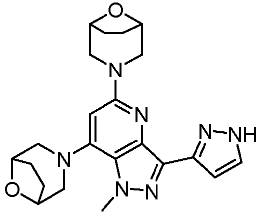

### Synthesis of intermediate 8-oxa-3-azabicyclo[3.2.1]octan-3-yl)ethan-1-one

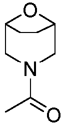

8-Oxa-3-azabicyclo[3.2.1]octane hydrochloride (3.20 g, 32.6 mmol) was added to DCM (60 mL), the mixture was cooled to 0°C, potassium carbonate (28.2 g, 204 mmol) was added to the reaction mixture, the reaction solution was stirred at 0 °C for 30 minutes, and acetyl chloride (8 mL, 114 mmol) was added to the reaction mixture. After stirring the reaction solution at room temperature for 16 hours, the TLC monitored the completion of the reaction (EA: PE = 1:1, phosphomolybdate colorization), suction filtered off the solids, concentrated under reduced pressure to give the target compound (3.50 g, crude, yellow oil).

### Step 1: Synthesis of methyl 4-amino-1-methyl-1H-pyrazole-5-carboxylate

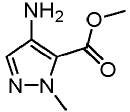

Methyl 1-methyl-4-nitro-1*H*-pyrazole-5-carboxylate (5.00 g, 27.0 mmol) was added to methanol (200 mL), 10% palladium carbon (100 mg) was added to the reaction mixture, the reaction solution was replaced with hydrogen for 3 times, the reaction was stirred overnight at room temperature under a hydrogen atmosphere, LC-MS monitored the completion of the reaction, the reaction was filtered through diatomaceous earth pad, and the filtrate was concentrated under reduced pressure to obtain a crude target compound (4.50 g, crude, bluish-violet solid). LC-MS (ESI) m/z: 156.2[M+H]⁺.

### Step 2: Synthesis of methyl 4-((-1-(8-oxa-3-azabicyclo[3.2.1]octan-3-yl)ethylidene)amino)-1-methyl-1H-pyrazole-5-carboxylate

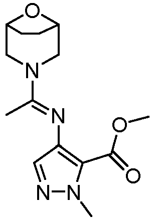

Methyl 4-amino-1-methyl-1*H*-pyrazole-5-carboxylate (3.48 g, 22.4 mmol) was added to a solution of 1,2-dichloroethane (35 mL), the mixture was cooled to 0°C, to the reaction mixture was added 8-oxa-3-azabicyclo[3.2.1]octan-3-yl)ethan-1-one (3.48 g, 22.4 mmol), the reaction solution was stirred at 0°C for 10 minutes, and phosphorus oxychloride (6.25 mL, 67.3 mmol) was added to the reaction mixture. After the reaction solution was stirred at 80°C for 0.5 hours, LC-MS monitored the completion of the reaction, concentrated under reduced pressure and separated by silica gel column (PE: EA = 1: 1) to give the target compound (3.56 g, yield 54.3%, yellow oil). LC-MS (ESI) m/z: 293. 1[M+H]⁺.

### Step 3: Synthesis of 5-(-8-oxa-3-azabicyclo[3.2.1]octan-3-yl)-1-methyl-1H-pyrazolo[4,3-b]pyridin-7-ol

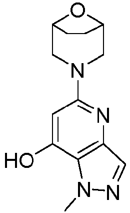

Methyl 4-((-1-(-8-oxa-3-azabicyclo[3 .2.1]octan-3-yl)ethylidene)amino)-1-methyl-1*H-*pyrazole-5-carboxylate (3.56 g, 12.2 mmol) was dissolved in DMF (70 mL), and 1M bistrimethylsilylamide lithium tetrahydrofuran solution (36.5 mL) was slowly added at 0°C. The resulting mixture was stirred at 0°C for 1 hour under nitrogen protection, after the reaction was monitored by LC-MS, an appropriate amount of aqueous ammonium chloride was added to quench the reaction, the mixture was distilled under reduced pressure, separated and purified by silica gel column (DCM: MeOH = 20:1-10:1), to obtain the target compound (2.16 g, yield 68.1%, yellow oil). LC-MS (ESI) *m*/*z:* 261.2 [M+H]⁺.

### Step 4: Synthesis of 3-(7-chloro-1-methyl-1H-pyrazolo[4,3-b]pyridin-5-yl)-8-oxa-3-azabicyclo[3.2.1]octane

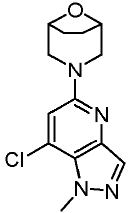

5-(-8-Oxa-3-azabicyclo[3.2.1]octan-3-yl)-1-methyl-1*H*-pyrazolo[4,3-b]pyridin-7-ol (2.16 g, 8.30 mmol) was dissolved in acetonitrile (22 mL). After stirring at room temperature for half an hour, phosphorus oxychloride (2.3 mL, 24.9 mmol) was added to the reaction mixture and stirred at 80°C overnight. After the reaction was monitored by LC-MS, the reaction mixture was spin-dried and purified by silica gel column (silica gel, EA: PE = 1:2) to obtain the target compound (2.28 g, yield 98.6%, yellow solid). LC-MS (ESI) m/z: 279.1 [M+H]⁺. ¹H NMR (400 MHz, DMSO-*d₆*) δ 7.89 (s, 1H), 7.10 (s, 1H), 4.43-4.39 (m, 2H), 4.17 (s, 3H), 3.82 (d, *J* = 12.2 Hz, 2H), 2.95 (d, *J=* 12.2 Hz, 2H), 1.85 - 1.76 (m, 2H), 1.79 - 1.69 (m, 2H).

### Step 5: Synthesis of 3-(7-(8-oxa-3-azabicyclo[3.2.1]octan-3-yl)-1-methyl-1H-pyrazolo[4,3-b]pyridin-5-yl)-8-oxa-3-azabicyclo[3.2.1]octane

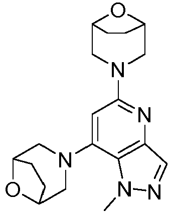

3-(7-Chloro-1-methyl-1*H*-pyrazolo[4,3-b]pyridin-5-yl)-8-oxa-3-azabicyclo[3.2.1]octane (1.20 g, 4.50 mmol) was dissolved in N-methylpyrrolidone (5 mL), then 8-oxa-3-azabicyclic[3.2.1]octane hydrochloride (537 mg, 3.59 mmol), RuphosPdG2 (139 mg, 0.179 mmol), and caesium carbonate (1.75 g, 5.38 mmol) were added to the mixture. The resulting mixture was stirred at 110°C for 2 hours under nitrogen protection. After the reaction was completed as monitored by LC-MS, the reaction mixture was added to water (50 mL), the aqueous phase was extracted with EA (20 mL×2), and washed with saturated saline (10 mL). The organic phase was dried by rotary evaporation and then was separated and purified by silica gel column (DCM: MeOH = 20:1) to give the target compound (245 mg, yield 38.4%, brown solid). LC-MS (ESI) *m*/*z:* 356.2 [M+H]⁺. ¹H NMR (400 MHz, DMSO-*d₆*) δ 7.78 (s, 1H), 6.43 (s, 1H), 4.46 - 4.30 (m, 4H), 4.15 (s, 3H), 3.79 (d, *J* = 12.1 Hz, 2H), 3.10 (d, *J* = 11.4 Hz, 2H), 2.99 (d, *J* = 11.4 Hz, 2H), 2.90 (d, *J* = 11.7 Hz, 2H), 2.13 - 2.03 (m, 2H), 1.94 - 1.86 (m, 2H), 1.83 - 1.71 (m, 4H).

### Step 6: Synthesis of 3-(7-(8-oxa-3-azabicyclo[3.2.1]octan-3-yl)-3-bromo-1-methyl-1H-pyrazolo[4,3-b]pyridin-5-yl)-8-oxa-3-azabicyclo[3.2.1]octane

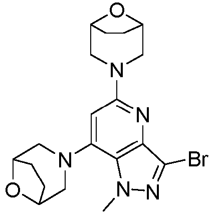

3-(7-(8-Oxa-3-azabicyclo[3.2.1]octan-3-yl)-1-methyl-1*H*-pyrazolo[4,3-b]pyridin-5-yl)-8-oxa-3-azabicyclo[3.2.1]octane (95 mg, 0.267 mmol) was dissolved in DCM (5 mL). A-bromosuccinimide (48 mg, 0.267 mmol) was added to the reaction mixture under nitrogen protection, and the reaction solution was stirred at room temperature for 10 minutes. After the reaction was completed as monitored by LC-MS, water (5 mL) was added to the reaction mixture and extracted with DCM (5 mL×3). The combined organic phases were washed with saturated saline, dried over anhydrous sodium sulfate, and filtered. The filtrate was concentrated under reduced pressure to obtain crude product. The crude product was separated and purified by preparative silica gel plate to obtain the target compound 3-(7-(8-oxa-3-azabicyclo[3.2.1]octan-3-yl)-3-bromo-1-methyl-1H-pyrazolo[4,3-b]pyridin-5-yl)-8-oxa-3-azabicyclo[3.2.1]octane (67 mg, yield 57.7%, yellow solid) LC-MS (ESI) *m*/*z:* 434.1 [M+H]⁺. ¹H NMR (400 MHz, DMSO-*d₆*) δ 6.49 (s, 1H), 4.46 - 4.36 (m, 4H), 4.12 (s, 3H), 3.87 (d, *J=* 12.3 Hz, 2H), 3.10 (d, *J=* 11.5 Hz, 2H), 3.04 - 2.89 (m,4H), 2.11 - 2.02 (m, 2H), 1.97 - 1.85 (m, 2H), 1.83 - 1.71 (m, 4H).

### Step 7: Synthesis of 3-(7-(8-oxa-3-azabicyclo[3.2.1]octan-3-yl)-1-methyl-3-(1-(tetrahydro-2H-pyran-2-yl)-1H-pyrazol-3-yl)-1H-pyrazolo[4,3-b]pyridin-5-yl)-8-oxa-3-azabicyclo[3.2.1]octane

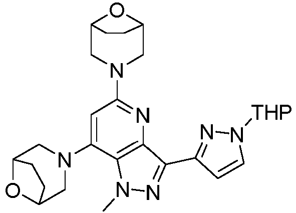

3-(7-(8-Oxa-3-azabicyclo[3.2.1]octan-3-yl)-3-bromo-1-methyl-1*H*-pyrazolo[4,3-b]pyridin-5-yl)-8-oxa-3-azabicyclo[3.2.1]octane (67 mg, 0.154 mmol) was dissolved in 1,4-dioxane (1 mL) and water (0.2 mL), and 1-(tetrahydro-2*H*-pyran-2-yl)-3-(4,4,5,5-tetramethyl-1,3,2-dioxyborane-2-yl)-1*H*-pyrazole (215 mg, 0.772 mmol), Pd(dtbpf)Cl₂ (10 mg, 0.015 mmol) and potassium phosphate (98 mg, 0.463 mmol) were added. The resulting mixture was stirred at 110°C for 2 hours under nitrogen protection. After the reaction was completed as monitored by LC-MS, water (5 mL) was added to the reaction mixture and extracted with EA (10 mL×2). The combined organic phases were washed with saturated saline, dried over anhydrous sodium sulfate, and filtered. The filtrate was concentrated under reduced pressure to obtain a crude product, which was separated and purified by silica gel column (PE: EA = 1:2) to obtain the target compound (53 mg, yield 67.9%, brown oil). LC-MS (ESI) *m*/*z:* 506.2 [M+H]⁺.

### Step 8: Synthesis of 3-(7-(8-oxa-3-azabicyclo[3.2.1]octan-3-yl)-1-methyl-3-(1H-pyrazol-3-yl)-1H-pyrazolo[4,3-b]pyridin-5-yl)-8-oxa-3-azabicyclo[3.2.1]octane

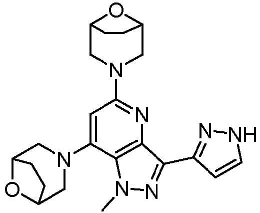

4 Mol/L of 1,4-dioxane hydrochloric acid solution (2 mL) was added to a solution of 3-(7-(8-oxa-3-azabicyclo[3.2.1]octan-3-yl)-1-methyl-3-(1-(tetrahydro-2*H*-pyran-2-yl)-1*H*-pyrazol-3-yl)-1*H*-pyrazolo[4,3-b]pyridin-5-yl)-8-oxa-3-azabicyclo[3.2.1]octane (53 mg, 0.105 mmol) in methanol (2 mL). The resulting mixture was stirred at room temperature for 2 hours under nitrogen protection, and after the reaction was completed as monitored by LC-MS, the solvent was removed under reduced pressure, and the residue was separated and purified by a prep-HPLC preparative chromatographic column to obtain the target compound (2.9 mg, yield 6.57%, white solid). LC-MS (ESI) *m*/*z:* 422.2 [M+H]⁺. ¹H NMR (400 MHz, MeOD) δ 7.91-7.75 (m, 1H), 6.90-6.77 (m, 1H), 6.41 (s, 1H), 4.67 - 4.57 (m, 2H), 4.57 - 4.49 (m, 2H), 4.27 (s, 3H), 3.81 (d, *J=* 11.8 Hz, 2H), 3.58 (d, J=12.3 Hz, 2H), 3.48 (d, *J* = 11.8 Hz, 2H), 3.37 (d, *J* = 12.2 Hz, 2H), 2.22 - 2.04 (m, 4H), 2.04 - 1.91 (m, 4H).

### Example 14: 3-(4-(4,4-difluoropiperidin-1-yl)-7-(1H-pyrazol-3-yl)imidazo[1,5-b]pyridazin-2-yl)-8-oxa-3-azabicyclo[3.2.1]octane

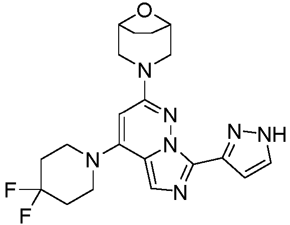

### Step 1: Synthesis of 3,6-dichloro-4-(4,4-difluoropiperidin-1-yl)pyridazine

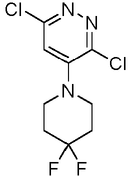

Potassium carbonate (1.82 g, 13.2 mmol) was added to a solution of 4-bromo-3,6-dichloropyridazine (1.00 g, 4.39 mmol) and 4,4-difluoropiperidine (797 mg, 6.58 mmol) in DMF (15.0 mL), the reaction mixture was stirred at room temperature for 16 hours under nitrogen protection, and the reaction was stopped. Water (20.0 mL) was added, then extracted with EA (25.0 mL×3), the combined organic phase was washed with water (20.0 mL×2) and saturated saline (20.0 mL×3), dried over anhydrous sodium sulfate, filtered, and the filtrate was concentrated under reduced pressure. The resulting residue was separated and purified by column chromatography (PE: EA = 10:1-3:1) to obtain the target compound (1.10 g, yield 93.5%, white solid). LC-MS (ESI) m/z 268.0 [M+H]⁺.

### Step 2: Synthesis of 3-(6-chloro-5-(4,4-difluoropiperidin-1-yl)pyridazin-3-yl)-8-oxa-3-azabicyclo[3 .2.1]octane

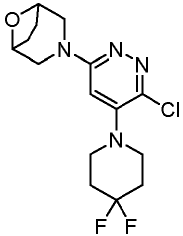

DIEA (1.59 g, 12.3 mmol) was added to a solution of 3,6-dichloro-4-(4,4-difluoropiperidin-1-yl)pyridazine (1.10 g, 4.10 mmol) and 8-oxa-3-azabicyclo[3.2.1] octane hydrochloride (921 mg, 6.16 mmol) in NMP (23.0 mL), the reaction mixture was stirred at 145°C for 16 hours, the reaction was stopped. After the reaction mixture was cooled to room temperature, water (25.0 mL) was added, extracted with EA (30.0 mL×3), the organic phases were combined, and then washed with water (30.0 mL×2) and saturated saline (30.0 mL×2), dried over anhydrous sodium sulfate, and filtered, the filtrate was concentrated under reduced pressure, and the resulting residue was separated and purified by column chromatography (PE: EA = 10:1-1:1) to obtain the target compound (1.20 g, yield 84.8%, white solid). LC-MS (ESI) *m*/*z* 345.0 [M+H]⁺.

### Step 3: Synthesis of 6-(8-oxa-3-azabicyclo[3.2.1]octan-3-yl)-4-(4,4-difluoropiperidin-1-yl)pyridazine-3-carbonitrile

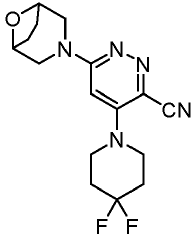

Zinc cyanide (476 mg, 4.05 mmol), DPPF (225 mg, 0.406 mmol) and Pd₂(dba)₃ (186 mg, 0.203 mmol) were sequentially added to a mixed solution of 3-(6-chloro-5-(4,4-difluoropiperidin-1-yl)pyridazin-3-yl)-8-oxa-3-azabicyclo[3.2.1]octane (700 mg, 2.03 mmol) in DMF (15.0 mL) and water (3 drops). The reaction mixture was stirred at 145°C for 16 hours under nitrogen protection, and the reaction was stopped. After the reaction mixture was cooled to room temperature, water (20.0 mL) was added, then extracted with EA (25.0 mL×3), the combined organic phase was washed with water (20.0 mL×2) and saturated saline (20.0 mL×2), dried over anhydrous sodium sulfate, filtered, and the filtrate was concentrated under reduced pressure. The resulting residue was separated and purified by column chromatography (PE: EA=3:1-1:3) to obtain the target compound (450 mg, yield 66.1%, brown solid). LC-MS (ESI) *m*/*z* 336.3 [M+H]⁺.

### Step 4: Synthesis of (6-(8-oxa-3-azabicyclo[3.2.1]octan-3-yl)-4-(4,4-difluoropiperidin-1-yl)pyridazin-3-yl)methanamine

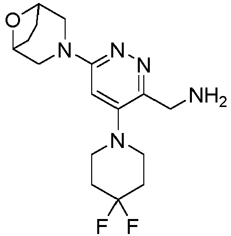

Ammonia (2.00 mL) and raney nickel (400 mg) were successively added to a solution of 6-(8-oxa-3-azabicyclo[3.2.1]octan-3-yl)-4-(4,4-difluoropiperidin-1-yl)pyridazine-3-carbonitrile (450 mg, 1.34 mmol) in tetrahydrofuran (20.0 mL), after three times of hydrogen replacement, the reaction mixture was reacted overnight at room temperature under a hydrogen atmosphere, and the reaction was stopped. The reaction mixture was filtered to remove solid residues, the filtrate was concentrated under reduced pressure, and dried in vacuo to obtain the target compound (450 mg, 98.8% yield, brown solid). LC-MS (ESI) *m*/*z* 340.2 [M+H]⁺.

### Step 5: Synthesis of N-((6-(8-oxa-3-azabicyclo[3.2.1]octan-3-yl)-4-(4,4-difluoropiperidin-1-yl)pyridazin-3-yl)methyl)-1H-pyrazole-5-carboxamide

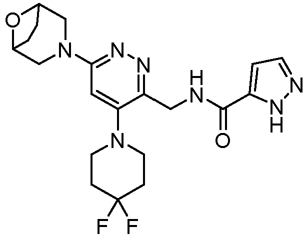

HATU (605 mg, 1.60 mmol) and DIEA (343 mg, 2.66 mmol) were added to a solution of (6-(8-oxa-3-azabicyclo[3.2.1]octan-3-yl)-4-(4,4-difluoropiperidin-1-yl)pyridazin-3-yl)methanamine (450 mg, 1.33 mmol) and 1*H*-pyrazole-5-carboxylic acid (134 mg, 1.20 mmol) in tetrahydrofuran (20.0 mL), after the reaction mixture was reacted at room temperature for 1 hour, sodium hydroxide solution (1.60 mL, 1M) was added to the reaction solution, and the mixture was further stirred and reacted for 1 hour, and the reaction was stopped. The reaction mixture was diluted with water (25.0 mL), then extracted with (DCM: methanol)=10:1 (30.0 mL×3), the organic phase was washed with saturated saline (30.0 mL), dried over anhydrous sodium sulfate, filtered, and the filtrate was concentrated under reduced pressure. The resulting residue was separated and purified by column chromatography (DCM: methanol = 100:1-30:1) to give the target compound (330 mg, yield 57.4%, brown solid). LC-MS (ESI) *m*/*z* 434.3 [M+H]⁺.

### Step 6: Synthesis of 3-(4-(4,4-difluoropiperidin-1-yl)-7-(1H-pyrazol-3-yl)imidazo[1,5-b]pyridazin-2-yl)-8-oxa-3-azabicyclo[3.2.1]octane

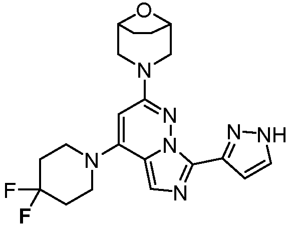

*N-*((6-(8-oxa-3-azabicyclo[3.2.1]octan-3-yl)-4-(4,4-difluoropiperidin-1-yl)pyridazin-3-yl)methyl)-1*H*-pyrazole-5-carboxamide (330 mg, 0.761 mmol) was dissolved in phosphorus oxychloride (12.0 mL), the reaction mixture was reacted at 120°C for 1 hour, the reaction was stopped. After the reaction mixture was cooled to room temperature, the reaction solution was slowly added dropwise to a saturated sodium bicarbonate solution at 0°C, then the pH was adjusted to 9 with the saturated sodium bicarbonate solution at 0°C, and extracted with EA (40.0 mL×3). The combined organic layer was dried over anhydrous sodium sulfate, filtered, and the filtrate was concentrated under reduced pressure. The resulting residues were separated and purified by reverse phase preparative chromatography (acetonitrile/water with 0.05% formic acid) to obtain the target compound (127.46 mg, yield 40.3%, gray solid). LC-MS (ESI) *m*/*z* 416.1 [M+H]⁺. ¹H NMR (400 MHz, DMSO-*d₆*) δ 13.16 (Brs, 1H), 7.71 - 7.64 (m, 1H), 7.62(s, 1H), 7.12-7.03 (m, 1H), 5.93 (s, 1H), 4.47 (s, 2H), 3.82 (d, *J* = 12.2 Hz, 2H), 3.65 - 3.59 (m, 4H), 3.06 (d, *J* = 11.1 Hz, 2H), 2.22 - 2.11 (m, 4H), 1.87 - 1.81 (m, 4H).

### Example 15: 3-(4-((S)-3-methylmorpholino)-7-(1H-pyrazol-3-yl)imidazon[1,5-b]pyridazin-2-yl)-8-oxa-3-azabicyclo[3.2.1]octane

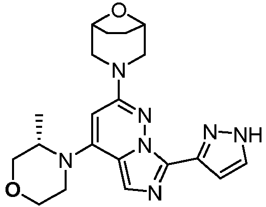

### Step 1: Synthesis of (S)-4-(3,6-dichloropyridazin-4-yl)-3-methylmorpholine

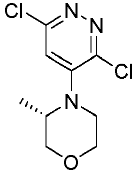

Potassium carbonate (4.52 g, 32.7 mmol) was added to a solution of 3,4,6-trichloropyridazine (2.00 g, 10.9 mmol) and (*S*)-3-methylmorpholine (1.65 g, 16.3 mmol) in NMP (20.0 mL). Under nitrogen protection, the reaction mixture was stirred at room temperature for 16 hours, and then the reaction was stopped. Water (25.0 mL) was added, then extracted with EA (30.0 mL×3), the combined organic phases were washed with water (20.0 mL×2) and saturated saline (20.0 mL×3), dried over anhydrous sodium sulfate and filtered, and the filtrate was concentrated under reduced pressure. The residue was separated and purified by column chromatography (PE: EA=10:1-3:1) to obtain the target compound (1.70 g, yield 62.8%, light yellow solid). LC-MS (ESI) *m*/*z* 247.9 [M+H]⁺.

### Step 2: Synthesis of 3-(6-chloro-5-((5)-3-methylmorpholino)pyridazin-3-yl)-8-oxa-3-azabicyclo[3 .2.1]octane

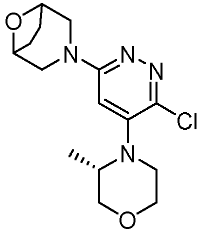

DIEA (1.41 g, 10.9 mmol) was added to a solution of (*S*)-4-(3,6-dichloropyridazin-4-yl)-3-methylmorpholine (900 mg, 3.63 mmol) and 8-oxa-3-azabicyclo[3.2.1]octane hydrochloride (814 mg, 5.44 mmol) in NMP (15.0 mL), the reaction mixture was stirred at 145°C for 16 hours, and then the reaction was stopped. After the reaction mixture was cooled to room temperature, water (20.0 mL) was added, extracted with EA (25.0 mL×3), the organic phases were combined, washed with water (20.0 mL×2) and saturated saline (20.0 mL×2), dried over anhydrous sodium sulfate and filtered, and the filtrate was concentrated under reduced pressure. The residue was separated and purified by column chromatography (PE: EA=5:1-1:1) to obtain the target compound (770 mg, yield 65.4%, white solid). LC-MS (ESI) *m*/*z* 325.3 [M+H]⁺.

### Step 3: Synthesis of 6-(8-oxa-3-azabicyclo[3.2.1]octan-3-yl)-4-((S)-3-methylmorpholino)pyridazine-3-carbonitrile

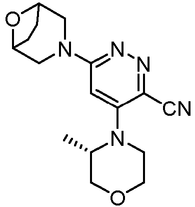

Zinc cyanide (557 mg, 4.74 mmol), DPPF (263 mg, 0.474 mmol) and Pd₂(dba)₃ (217 mg, 0.237 mmol) were added successively to a mixed solution of 3-(6-chloro-5-((*S*)-3-methylmorpholino)pyridazin-3-yl)-8-oxa-3-azabicyclo[3.2.1]octane (770 mg, 2.37 mmol) in water (3 drops) and DMF (15.0 mL). Under nitrogen protection, the reaction mixture was stirred at 145°C for 16 hours, and the reaction was then stopped. After the reaction solution was cooled to room temperature, water (20.0 mL) was added, and then extracted with EA (25.0 mL×3), organic phases were combined and washed with waer (20.0 mL×2) and saturated saline (20.0 mL×2), dried over anhydrous sodium sulfate, and filtered, and the filtrate was concentrated under reduced pressure. The residue was separated and purified by column chromatography (PE: EA=3:1-1:4) to obtain the target compound (670 mg, yield 89.6%, brown solid). LC-MS (ESI) *m*/*z* 316.1 [M+H]⁺.

### Step 4: Synthesis of (6-(8-oxa-3-azabicyclo[3.2.1]octan-3-yl)-4-((S)-3-methylmorpholino)pyridazin-3-yl)methanamine

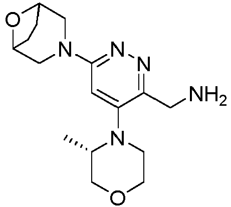

Ammonia (1.50 mL) and raney nickel (500 mg) were added successively to a solution of 6-(8-oxa-3-azabicyclo[3.2.1]octan-3-yl)-4-((*S*)-3-methylmorpholino)pyiidazine-3-carbonitrile (670 mg, 2.12 mmol) in tetrahydrofuran (15.0 mL). After hydrogen replacement for three times, the reaction mixture was reacted overnight at room temperature under a hydrogen atmosphere, and the reaction was stopped. The reaction mixture was filtered to remove solid residue, and the filtrate was concentrated under reduced pressure and vacuum dried to obtain the target compound (630 mg, yield 92.8%, brown solid). LC-MS (ESI) *m*/*z* 320.3 [M+H]⁺.

### Step 5: Synthesis of N-((6-(8-oxa-3-azabicyclo[3.2.1]octan-3-yl)-4-((S)-3-methylmorpholino)pyridazin-3-yl)methyl)-1H-pyrazole-5-carboxamide

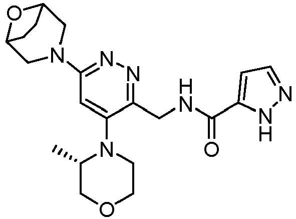

HATU (471 mg, 1.24 mmol) and DIEA (267 mg, 2.06 mmol) were added to a solution of (6-(8-oxa-3-azabicyclo[3.2.1]octan-3-yl)-4-((*S*)-3-methylmorpholino)pyridazin-3-yl)methanamine (330 mg, 1.03 mmol) and 1*H*-pyrazole-5-carboxylic acid (116 mg, 1.03 mmol) in tetrahydrofuran (15.0 mL). After the reaction mixture was reacted at room temperature for 1 hour, sodium hydroxide solution (1mol/L) was added, the reaction mixture was further stirred for 1 hour, and the reaction was stopped. The reaction mixture was diluted with water (20.0 mL), and then extracted with (DCM: methanol)=10:1 (25.0 mL×2), the organic phase was washed with saturated saline (20.0 mL), dried over anhydrous sodium sulfate and filtered, and the filtrate was concentrated under reduced pressure. The residue was separated and purified by column chromatography (DCM: methanol=100:1-20:1) to obtain the target compound (275 mg, yield 64.4%, brown solid). LC-MS (ESI) *m*/*z* 414.3 [M+H]⁺.

### Step 6: Synthesis of 3-(4-((S)-3-methylmorpholino)-7-(1H-pyrazol-3-yl)imidazo[1,5-b]pyridazin-2-yl)-8-oxa-3-azabicyclo[3 .2.1]octane

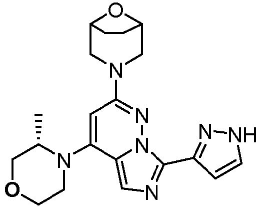

*N*-((6-(8-oxa-3-azabicyclo[3.2.1]octan-3-yl)-4-((*S*)-3-methylmorpholino)pyridazin-3-yl)methyl)-1*H*-pyrazole-5-carboxamide (275 mg, 0.665 mmol) was dissolved in phosphorus oxychloride (12.0 mL). The reaction was stopped after the reaction mixture was reacted at 120°C for 1 hour. After the reaction mixture was cooled to room temperature, the reaction mixture was added dropwise slowly into a saturated sodium bicarbonate solution at 0°C, adjusted the pH to 9 with the saturated sodium bicarbonate solution at 0°C, and then extracted with EA (30.0 mL×3), the combined organic layer was dried over anhydrous sodium sulfate and filtered, and the filtrate was concentrated under reduced pressure. The residue was separated and purified by reverse phase preparative chromatography (acetonitrile/water with 0.05% formic acid) to obtain the target compound (112.12 mg, yield 42.6%, white solid). LC-MS (ESI) *m*/*z* 396.2 [M+H]⁺. ¹H NMR (400 MHz, DMSO-*d₆*) δ 13.20 (Brs, 1H), 7.72 - 7.61 (m, 2H), 7.08 (s, 1H), 5.78 (s, 1H), 4.49 - 4.43 (m, 2H), 4.38 - 4.31 (m, 1H), 3.99 - 3.92 (m, 1H), 3.84 - 3.75 (m, 3H), 3.74 - 3.68 (m, 1H), 3.65 - 3.57 (m, 1H), 3.49 - 3.39 (m, 2H), 3.10 - 3.02 (m, 2H), 1.88 - 1.80 (m, 4H), 1.15 (d, *J* = 6.6 Hz, 3H).

### Example 16: 3-(4-(cis-2,6-dimethylmorpholino)-7-(1H-pyrazol-3-yl)imidazo[1,5-b]pyridazin-2-yl)-8-oxa-3-azabicyclo[3.2.1]octane

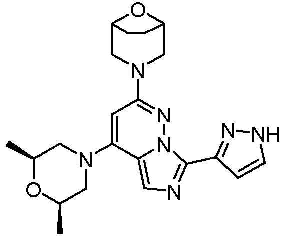

### Step 1: Synthesis of 4-(3,6-dichloropyridazin-4-yl)-cis-2,6-dimethylmorpholine

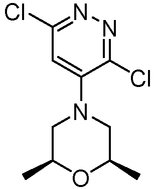

Potassium carbonate (6.78 g, 49.1 mmol) was added to a solution of 3,4,6-trichloropyridazine (3.00 g, 16.4 mmol) and cis-2,6-dimethylmorpholine (3.01 g, 26.1 mmol) in NMP (30.0 mL). Under nitrogen protection, the reaction mixture was stirred at room temperature for 16 hours, and then the reaction was stopped. Water (35.0 mL) was added, then extracted with EA (40.0 mL×3) and the organic phases were combined, washed with water (30.0 mL×2) and saturated saline (30.0 mL×2), dried over anhydrous sodium sulfate and filtered, and the filtrate was concentrated under reduced pressure. The residue was separated and purified by column chromatography (PE: EA=10:1-3:1) to obtain the target compound (3.70 g, yield 86.3%, white solid). LC-MS (ESI) m/z 262.2 [M+H]⁺.

### Step 2: Synthesis of 3-(6-chloro-5-(cis-2,6-dimethylmorpholino)pyridazin-3-yl)-8-oxa-3-azabicyclo[3.2.1]octane

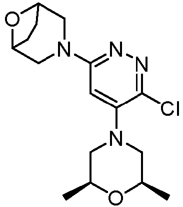

DIEA (1.48 g, 11.4 mmol) was added to a solution of 4-(3,6-dichloropyridazin-4-yl)-cis-2,6-dimethylmorpholine (1.00 g, 3.81 mmol) and 8-oxa-3-azabicyclo[3.2.1]octane hydrochloride (913 mg, 6.10 mmol) in NMP (15.0 mL), the reaction mixture was stirred at 145°C for 16 hours, and then the reaction was stopped. After the reaction mixture was cooled to room temperature, water (25.0 mL) was added and extracted with EA (30.0 mL×3), the organic phases were combined, and then washed with water (25.0 mL×2) and saturated saline (25.0 mL×2), dried over anhydrous sodium sulfate and filtered, and the filtrate was concentrated under reduced pressure. The residue was separated and purified by column chromatography (PE: EA=5:1-1:1) to obtain the target compound (1.00 g, yield 77.3%, brown solid). LC-MS (ESI) *m*/*z* 339.3 [M+H]⁺.

### Step 3: Synthesis of 6-(8-oxa-3-azabicyclo[3.2.1]octan-3-yl)-4-(cis-2,6-dimethylmorpholino)pyridazine-3-carbonitrile

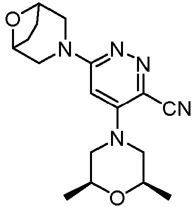

Zinc cyanide (695 mg, 5.92 mmol), DPPF (328 mg, 0.592 mmol) and Pd₂(dba)₃ (271 mg, 0.295 mmol) were added successively to a mixed solution of 3-(6-chloro-5-(cis-2,6-dimethylmorpholino)pyridazin-3-yl)-8-oxa-3-azabicyclo[3.2.1]octane (1.00 g, 2.95 mmol) in water (5 drops) and DMF (20.0 mL). Under nitrogen protection, the reaction mixture was stirred at 145°C for 16 hours, and then the reaction was stopped. After the reaction solution was cooled to room temperature, water (20.0 mL) was added, and then extracted with EA (25.0 mL×3) and the organic phase was combined, washed with saturated saline (20.0 mL×4), dried over anhydrous sodium sulfate and filtered, and the filtrate was concentrated under reduced pressure. The residue obtained was separated and purified by column chromatography (PE: EA=5:1-1:3) to obtain the target compound (860 mg, yield 88.5%, brown solid). LC-MS (ESI) *m*/*z* 330.3 [M+H]⁺.

### Step 4: Synthesis of (6-(8-oxa-3-azabicyclo[3.2.1]octan-3-yl)-4-(cis-2,6-dimethylmorpholino)pyridazin-3-yl)methanamine

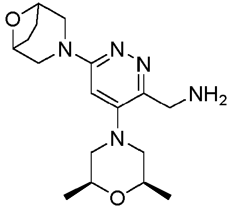

Ammonia (2.00 mL) and raney nickel (-600 mg) were added successively to a solution of 6-(8-oxa-3-azabicyclo[3.2.1]octan-3-yl)-4-(cis-2,6-dimethylmorpholino)pyridazine-3-carbonitrile (860 mg, 2.61 mmol) in tetrahydrofuran (20.0 mL). After hydrogen replacement for three times, the reaction mixture was reacted overnight at room temperature under a hydrogen atmosphere, and the reaction was stopped. The reaction mixture was filtered to remove solid residues, and the filtrate was concentrated under reduced pressure and dried in vacuum to obtain the target compound (710 mg, yield 81.6%, brown solid). LC-MS (ESI) *m*/*z* 334.3 [M+H]⁺.

### Step 5: Synthesis of N-((6-(8-oxa-3-azabicyclo[3.2.1]octan-3-yl)-4-(cis-2,6-dimethylmorpholino)pyridazin-3-yl)methyl)-1H-pyrazole-5-carboxamide

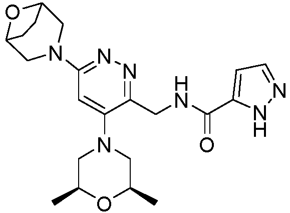

HATU (479 mg, 1.26 mmol) and DIEA (271 mg, 2.10 mmol) were added to a solution of (6-(8-oxa-3-azabicyclo[3.2.1]octan-3-yl)-4-(cis-2,6-dimethylmorpholino)pyridazin-3-yl)methanamine (350 mg, 1.05 mmol) and 1*H*-pyrazole-5-carboxylic acid (118 mg, 1.05 mmol) in tetrahydrofuran (15.0 mL). After the reaction mixture was reacted at room temperature for 1 hour, sodium hydroxide (1mol/L) solution was added in the reaction solution, the mixture was further stirred for 1 hour, and the reaction was stopped. The reaction mixture was diluted with water (20.0 mL), and then extracted with (DCM: methanol=10:1) (25.0 mL×3), the organic phase was washed with saturated saline (20.0 mL×2), dried over anhydrous sodium sulfate and filtered, and the filtrate was concentrated under reduced pressure. The residue was separated and purified by column chromatography (methanol: DCM=5%) to obtain the target compound (280 mg, yield 62.4%, brown solid). LC-MS (ESI) m/z 428.4 [M+H]⁺.

### Step 6: Synthesis of 3-(4-(cis-2,6-dimethylmorpholino)-7-(1H-pyrazol-3-yl)imidazo[1,5-b]pyridazin-2-yl)-8-oxa-3-azabicyclo[3.2.1]octane

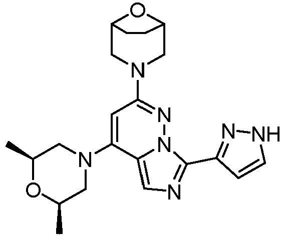

*N-*((6-(8-oxa-3-azabicyclo[3.2.1]octan-3-yl)-4-(cis-2,6-dimethylmorpholino)pyridazin-3-yl)methyl)-1*H*-pyrazole-5-carboxamide (150 mg, 0.351 mmol) was dissolved in a solution of phosphorus oxychloride (6.00 mL). The reaction mixture was reacted at 120°C for 1 hour, then the reaction was stopped. After the reaction mixture was cooled to room temperature, the reaction solution was slowly dropped into a saturated sodium bicarbonate solution at 0°C, then adjusted the pH to 9 with the saturated sodium bicarbonate solution at 0°C, and extracted with EA (30.0 mL×3), the combined organic layer was dried over anhydrous sodium sulfate and filtered, and the filtrate was concentrated under reduced pressure. The residue was separated and purified by reversed-phase preparative chromatography (acetonitrile/water with 0.05% formic acid) to obtain the target compound (54.42 mg, yield 37.9%, white solid). LC-MS (ESI) *m*/*z* 410.4 [M+H]⁺.¹H NMR (400 MHz, DMSO-*d₆*) δ 13.12 (Brs, 1H), 7.68-7.62 (m, 2H), 7.07 (d, *J=* 1.8 Hz, 1H), 5.85 (s, 1H), 4.47 (s, 2H), 3.90 - 3.74 (m, 6H), 3.09 - 3.02 (m, 2H), 2.66 - 2.57 (m, 2H), 1.88 - 1.81 (m, 4H), 1.19 (d, *J* = 6.2 Hz, 6H).

### Example 17: 3-(4-(piperidin-1-yl)-7-(1H-pyrazol-3-yl)imidazo[1,5-b]pyridazin-2-yl)-8-oxa-3-azabicyclo[3.2.1]octane

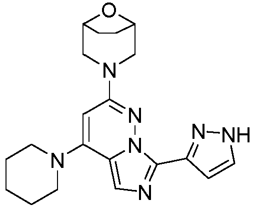

### Step 1: Synthesis of 3,6-dichloro-4-(piperidin-1-yl)pyridazine

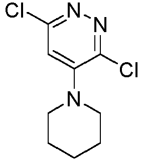

Potassium carbonate (3.01 g, 21.8 mmol) was added to a solution of 3,4,6-trichloropyridazine (2.00 g, 10.9 mmol) and piperidine (1.39 g, 16.4 mmol) in NMP (25.0 mL). The reaction mixture was stirred at room temperature for 16 hours under nitrogen protection, then the reaction was stopped. Then the reaction solution was diluted with EA (45.0 mL), and washed with saturated saline (45.0 mL×3), dried over anhydrous sodium sulfate and filtered, and concentrateed under reduced pressure. The residue was separated and purified by column chromatography (PE: EA=5:1) to obtain the target compound (2.30 g, yield 90.9%, yellow solid). LC-MS (ESI) *m*/*z* 232.1 [M+H]⁺.

### Step 2: Synthesis of 3-(6-chloro-5-(piperidin-1-yl)pyridazin-3-yl)-8-oxa-3-azabicyclo[3.2.1]octane

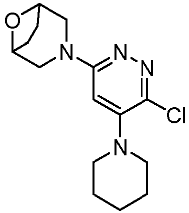

DIEA (6.75 mL, 38.7 mmol) was added to a solution of 3,6-dichloro-4-(piperidin-1-yl)pyridazine (3.00 g, 12.9 mmol) and 8-oxa-3-azabicyclo[3.2.1]octane hydrochloride (3.87 g, 25.8 mmol) in the NMP (60.0 mL), the reaction mixture was stirred at 145°C for 16 hours, and the reaction was stopped. Then water (50.0 mL) was added and extracted with EA (40.0 mL×3), the organic phases were combined and washed with saturated saline (30.0 mL), dried over anhydrous sodium sulfate and filtered, and concentrated under reduced pressure. The residue was separated and purified by chromatographic column (PE: EA=5:1) to obtain the target compound (1.20 g, yield 30.1%, yellow solid). LC-MS (ESI) *m*/*z* 309.1 [M+H]⁺.¹H NMR (400 MHz, CDCl₃) δ 6.12 (s, 1H), 4.52 - 4.46 (m, 2H), 3.82 - 3.75 (m, 2H), 3.20 - 3.18 (m, 1H), 3.18 - 3.15 (m, 1H), 3.13 - 3.07 (m, 4H), 2.01 - 1.94 (m, 2H), 1.88 - 1.82 (m, 2H), 1.76 - 1.71 (m, 4H), 1.66 - 1.58 (m, 2H).

### Step 3: Synthesis of 6-(8-oxa-3-azabicyclo[3.2.1]octan-3-yl)-4-(piperidin-1-yl)pyridazine-3-carbonitrile

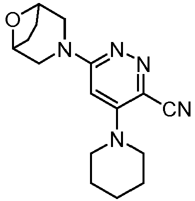

DPPF (431 mg, 0.777 mmol) and Pd₂(dba)₃ (356 mg, 0.388 mmol) were added to a solution of 3-(6-chloro-5-(piperidin-1-yl)pyridazin-3-yl)-8-oxa-3-azabicyclo[3.2.1]octane (1.20 g, 3.89 mmol) and zinc cyanide (912 mg, 7.77 mmol) in DMF (25.0 mL). The reaction mixture was stirred at 140°C for 16 hours under nitrogen protection, and the reaction was stopped. Then the reaction solution was diluted with EA (40.0 mL), and washed with saturated saline (35.0 mL×3), dried over anhydrous sodium sulfate and filtered, and concentrated under reduced pressure. The residue was separated and purified by column chromatography (PE: EA=1: 1) to obtain the target compound (1.20g, yield 100%, yellow solid). LC-MS (ESI) *m*/*z* 300.2 [M+H]⁺.

### Step 4: Synthesis of (6-(8-oxa-3-azabicyclo[3.2.1]octan-3-yl)-4-(piperidin-1-yl)pyridazin-3-yl)methanamine

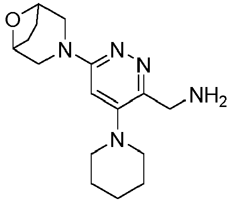

Raney nickel (-1.18 g, 20.0 mmol) and ammonia (5 drops) were added to a solution of 6-(8-oxa-3-azabicyclo[3.2.1]octan-3-yl)-4-(piperidin-1-yl)pyridazine-3-carbonitrile (1.2 g, 4.01 mmol) in tetrahydrofuran (20.0 mL). The reaction mixture was reacted at room temperature under a hydrogen atmosphere for 16 hours and the reaction was stopped. Then the reaction solution was filtered and concentrated under reduced pressure. The resulting residue was the target compound (1.10 g, yield 90.4%, yellow oil). LC-MS (ESI) *m*/*z* 304.1 [M+H]⁺.

### Step 5: Synthesis of N-((6-(8-oxa-3-azabicyclo[3.2.1]octan-3-yl)-4-(piperidin-1-yl)pyridin-3-yl)methyl)-1H-pyrazole-5-carboxamide

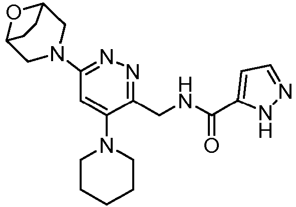

HATU (752 mg, 1.98 mmol) and DIEA (639 mg, 4.94 mmol) were added to a solution of (6-(8-oxa-3-azabicyclo[3.2.1]octan-3-yl)-4-(piperidin-1-yl)pyridazin-3-yl)methanamine (500 mg, 1.65 mmol) and 1*H*-pyrazol-5-carboxylic acid (166 mg, 1.48 mmol) in tetrahydrofuran (12.0 mL), the reaction mixture was stirred at room temperature for 45 minutes, and the reaction was stopped. Sodium hydroxide solution was added dropwise into the reaction solution and the mixture was stirred for 2 minutes. The mixture solution was concentrated under reduced pressure. The residue was separated and purified by column chromatography (DCM: methanol=10:1) to obtain the target compound (300 mg, yield 45.8%, yellow solid). LC- MS (ESI) *m*/*z* 398.2 [M+H]⁺.

### Step 6: Synthesis of 3-(4-(piperidin-1-yl)-7-(1H-pyrazol-3-yl)imidazo[1,5-b]pyridazin-2-yl)-8-oxa-3-azabicyclo[3.2.1]octane

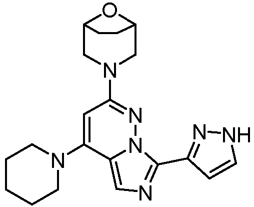

*N*-((6-(8-oxa-3-azabicyclo[3.2.1]octan-3-yl)-4-(piperidin-1-yl)pyridin-3-yl)methyl)-1*H-*pyrazole-5-carboxamide (150 mg, 0.327 mmol) and phosphorus oxychloride (9.00 mL) were added to a reaction flask at room temperature. The reaction mixture was reacted at 100°C for 1 hour and the reaction was stopped. The reaction solution was added dropwise to a saturated sodium bicarbonate aqueous solution, adjusted to pH=7, then the mixed solution was extracted with EA (20.0 mL×3), the organic phase was washed with saturated saline, dried over anhydrous sodium sulfate and filtered, and concentrated under reduced pressure. The residue was purified by reverse phase preparative chromatography (acetonitrile/water with 0.05% formic acid) to obtain the target compound (51.7 mg, yield 36.1%, white solid). LC- MS (ESI) *m*/*z* 380.1[M+H]⁺. ¹H NMR (400 MHz, MeOD-*d₄*) δ 7.69 (s, 1H), 7.47 (s, 1H), 7.10 (s, 1H), 5.77 (s, 1H), 4.51 - 4.48 (m, 2H), 3.83 - 3.79 (m, 2H), 3.52 - 3.48 (m, 4H), 3.21 - 3.16 (m, 2H), 1.99 - 1.95 (m, 4H), 1.79 - 1.74 (m, 6H).

### Example 18: 3-(4-(2-methylmorpholino)-7-(1H-pyrazol-3-yl)imidazo[1,5-b]pyridazin-2-yl)-8-oxa-3-azabicyclo[3.2.1]octane

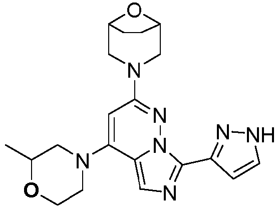

### Step 1: Synthesis of 4-(3,6-dichloropyridazin-4-yl)-2-methylmorpholine

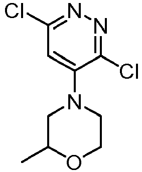

Potassium carbonate (3.01 g, 21.8 mmol) was adde to a solution of 3,4,6-trichloropyridazine (2.00 g, 10.9 mmol) and 2-methylmorpholine (1.32 g, 13.1 mmol) in NMP (25.0 mL). The reaction mixture was stirred at room temperature for 16 hours under nitrogen protection and then the reaction was to stopped. Then the reaction solution was diluted with EA (45.0 mL), washed with saturated saline (45.0 mL×3), dried over anhydrous sodium sulfate and filtered, and concentrated under reduced pressure. The residue was separated and purified by column chromatography (PE: EA=5:1) to obtain the target compound (2.30 g, yield 85.0%, yellow solid). LC-MS (ESI) *m*/*z* 248.1 [M+H]⁺.

### Step 2: Synthesis of 3-(6-chloro-5-(2-methylmorpholino)pyridazin-3-yl)-8-oxa-3-azabicyclo[3 .2.1]octane

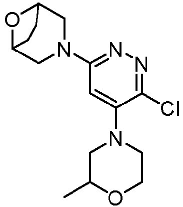

DIEA (2.40 g, 18.5 mmol) was added to a solution of 4-(3,6-dichloropyridazin-4-yl)-2-methylmorpholine (2.30 g, 9.27 mmol) and 8-oxa-3-azabicyclo[3.2.1]octane hydrochloride (2.08 g, 13.9 mmol) in NMP (25.0 mL). The reaction mixture was stirred at 145°C for 16 hours under nitrogen protection, and the reaction was stopped. Then the reaction solution was diluted with EA (45.0 mL), and washed with saturated saline (45.0 mL×3), dried over anhydrous sodium sulfate and filtered, and concentrated under reduced pressure. The residue was separated and purified by column chromatography (PE: EA=1:1) to obtain the target compound (2.00 g, yield 76.4%, yellow solid). LC-MS (ESI) *m*/*z* 225.1 [M+H]⁺. ¹H NMR (400 MHz, CDCl₃) δ 6.13 (s, 1H), 4.55 - 4.48 (m, 2H), 4.01 - 3.94 (m, 1H), 3.89 - 3.80 (m, 4H), 3.50 - 3.43 (m, 2H), 3.25 - 3.19 (m, 2H), 2.89 - 2.80 (m, 1H), 2.57 - 2.49 (m, 1H), 2.04 - 1.96 (m, 2H), 1.91 - 1.82 (m, 2H), 1.24 (d, *J* = 6.3 Hz, 3H).

### Step 3: Synthesis of 6-(8-oxa-3-azabicyclo[3.2.1]octan-3-yl)-4-(2-methylmorpholino)pyridazine-3-carbonitrile

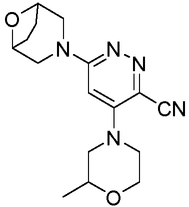

DPPF (614 mg, 1.11 mmol) and Pd₂(dba)₃ (507 mg, 0.554 mmol) were added to a solution of 3-(6-chloro-5-(2-methylmorpholino)pyridazin-3-yl)-8-oxa-3-azabicyclo[3.2.1]octane (1.80 g, 5.54 mmol) and zinc cyanide (1.30 g, 11.1 mmol) in DMF (35.0 mL). The reaction mixture was stirred at 140°C for 16 hours under nitrogen protection, and the reaction was stopped. Then the reaction solution was diluted with EA (55.0 mL), and washed with saturated saline (40.0 mL×3), dried over anhydrous sodium sulfate and filtered, and concentrated under reduced pressure. The residue was separated and purified by column chromatography (PE: EA=1:1) to obtain the target compound (1.50 g, yield 85.8%, yellow solid). LC-MS (ESI) *m*/*z* 316.0 [M+H]⁺.

### Step 4: Synthesis of (6-(8-oxa-3-azabicyclo[3.2.1]octan-3-yl)-4-(2-methylmorpholino)pyridazin-3-yl)methanamine

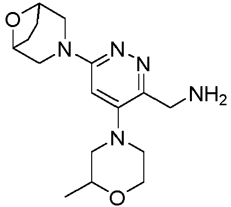

Raney nickel (-1.40 g, 23.8 mmol) was added to a solution of 6-(8-oxa-3-azabicyclo[3.2.1]octan-3-yl)-4-(2-methylmorpholino)pyridazine-3-carbonitrile (1.50 g, 4.76 mmol) in tetrahydrofuran (30.0 mL). The reaction mixture was reacted at room temperature for 16 hours under a hydrogen atmosphere and then the reaction was stopped. Then the reaction solution was filtered, concentrated under reduced pressure, and the residue was separated and purified by column chromatography (DCM: methanol=10:1) to obtain the target compound (1.50g, yield 98.7%, yellow solid). LC-MS (ESI) *m*/*z* 320.0 [M+H]⁺.

### Step 5: Synthesis of N-((6-(8-oxa-3-azabicyclo[3.2.1]octan-3-yl)-4-(2-methylmorpholino)pyridazin-3-yl)methyl)-1H-pyrazole-5-carboxamide

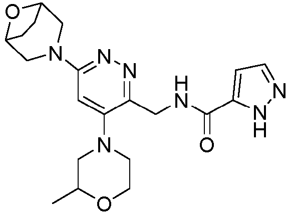

HATU (714 mg, 1.88 mmol) and DIEA (607 mg, 4.70 mmol) were added to a solution of (6-(8-oxa-3-azabicyclo[3.2.1]octan-3-yl)-4-(2-methylmorpholino)pyridazin-3-yl)methanamine (500 mg, 1.57 mmol) and 1*H*-pyrazole-5-carboxylic acid (158 mg, 1.41 mmol) in tetrahydrofuran (10.0 mL). The reaction mixture was reacted at room temperature for 1 hour and the reaction was stopped. Then the reaction solution was filtered and concentrated under reduced pressure. The residue was separated and purified by column chromatography (PE: EA=0:1) to obtain the target compound (500 mg, yield 77.2%, yellow solid). LC-MS (ESI) *m*/*z* 414.0 [M+H]⁺.

### Step 6: Synthesis of 3-(4-(2-methylmorpholino)-7-(1H-pyrazol-3-yl)imidazo[1,5-b]pyridazin-2-yl)-8-oxa-3-azabicyclo[3.2.1]octane

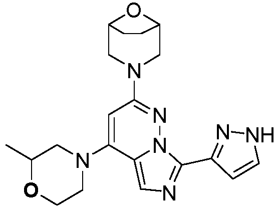

At room temperature, *N-*((6-(8-oxa-3-azabicyclo[3.2.1]octan-3-yl)-4-(2-methylmorpholino)pyridazin-3-yl)methyl)-1*H*-pyrazole-5-carboxamide (200 mg, 0.484 mmol) and phosphorus oxychloride (6.00 mL) were added to a reaction flask. The reaction mixture was reacted at 100°C for 1 hour and the reaction was stopped. Then the reaction solution was added dropwise to a saturated sodium bicarbonate aqueous solution and adjusted to pH 7, then the mixture solution was extracted with EA (30.0 mL×3), the organic phase was washed with saturated saline, dried over anhydrous sodium sulfate and filtered, and concentrated under reduced pressure. The residue was purified by reverse phase preparative chromatography (acetonitrile/water with 0.05% formic acid) to obtain the target compound (82.0 mg, yield 42.9%, white solid). LC- MS (ESI) *m*/*z* 396.1 [M+H]⁺. ¹H NMR (400 MHz, MeOD-*d₄*) δ 7.86 - 7.63 (m, 1H), 7.62 - 7.41 (m, 1H), 7.13 (s, 1H), 5.84 (s, 1H), 4.54 - 4.47 (m, 2H), 4.04 - 3.98 (m, 1H), 3.87 - 3.78 (m, 6H), 3.22 - 3.16 (m, 2H), 3.13 - 3.04 (m, 1H), 2.79 - 2.70 (m, 1H), 2.01 - 1.93 (m, 4H), 1.25 (d, *J* = 6.1 Hz, 3H).

### Example 19: 3-(1-methyl-7-((R)-3-methylmorpholino)-3-(1H-pyrazol-3-yl)-1H-pyrazolo[4,3-b]pyridin-5-yl)-8-oxa-3-azabicyclo[3.2.1]octane

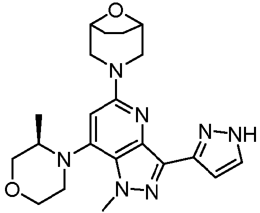

Synthesis of intermediate 3-(7-chloro-1-methyl-1*H*-pyrazolo[4,3-b]pyridin-5-yl)-8-oxa-3-azabicyclo[3.2.1]octane may refer to Example 13.

### Step 1: Synthesis of 3-(3-bromo-7-chloro-1-methyl-1H-pyrazolo[4,3-b]pyridin-5-yl)-8-oxa-3-azabicyclo[3 .2.1]octane

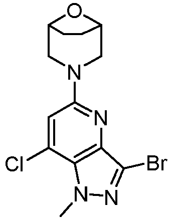

3-(7-Chloro-1-methyl-1*H*-pyrazolo[4,3-b]pyridin-5-yl)-8-oxa-3-azabicyclo[3.2.1]octane (200 mg, 0.718 mmol) was dissolved in DCM (5 mL). A-bromosuccinimide (128 mg, 0.718 mmol) was added to the reaction mixture under nitrogen protection, and the reaction solution was stirred at room temperature for 10 minutes. After LC-MS monitored the reaction completed, water (5 mL) was added to the reaction mixture and extracted with DCM (5 mL×3). The combined organic phase was washed with saturated saline, dried over anhydrous sodium sulfate, and filtered. The filtrate was concentrated under reduced pressure to obtain crude product. The crude product was separated and purified by silica gel column (EA: PE=1:4) to abtain the target compound (241 mg, yield 93.9%, yellow solid). LC-MS (ESI) *m*/*z:* 357.1 [M+H]⁺. ¹H NMR (400 MHz, DMSO-*d₆*) δ 7.20 (s, 1H), 4.45 - 4.36 (m, 2H), 4.20 (s, 3H), 3.87 (d, *J=* 12.4 Hz, 2H), 2.99 (d, *J=* 12.4 Hz, 2H), 1.86 - 1.76 (m, 2H), 1.75 - 1.69 (m, 2H).

### Step 2: Synthesis of 3-(7-chloro-1-methyl-3-(1-(tetrahydro-2H-pyran-2-yl)-1H-pyrazol-3-yl)-1H-pyrazolo[4,3-b]pyridin-5-yl)-8-oxa-3-azabicyclo[3.2.1]octane

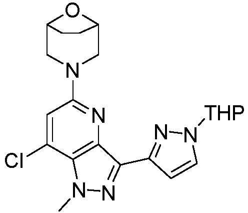

3-(3-Bromo-7-chloro-1-methyl-1*H*-pyrazolo[4,3-b]pyridin-5-yl)-8-oxa-3-azabicyclo[3.2.1]octane (201 mg, 0.562 mmol) was dissolved in a mixture of 1,4-dioxane (2 mL) and water (0.2 mL), and 1-(tetrahydro-2*H*-pyran-2-yl)-3-(4,4,5,5-tetramethyl-1,3,2-dioxaboran-2-yl)-1*H-*pyrazole (313 mg, 1.12 mmol), Pd(dtbpf)Cl₂ (36.3 mg, 0.056 mmol) and potassium phosphate (358 mg, 1.69 mmol) were added to the mixture. The obtained mixture was stirred at 40°C under nitrogen protection for 2h. After LC-MS monitoried the reaction was completed, water (5 mL) was added to the reaction mixture and extracted with EA (10 mL×2). The combined organic phase was washed with saturated saline, dried over anhydrous sodium sulfate, and filtered. The filtrate was concentrated under reduced pressure to obtain the crude product, which was separated and purified by silica gel column (PE: EA=1:2) to obtain the target compound (102 mg, yield 42.3%, brown oil). LC-MS (ESI) *m*/*z:* 429.2 [M+H]⁺.

### Step 3: Synthesis of 3-(1-methyl-7-((R)-3-methylmorpholino)-3-(1-(tetrahydro-2H-pyran-2-yl)-1H-pyrazol-3-yl)-1H-pyrazolo[4,3-b]pyridin-5-yl)-8-oxa-3-azabicyclo[3.2.1]octane

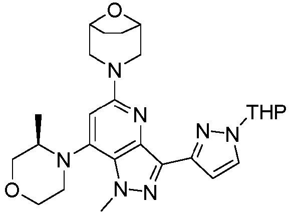

3 -(7-Chloro-1-methyl-3-(1-(tetrahydro-2*H*-pyran-2-yl)-1*H*-pyrazol-3-yl)-1*H*-pyrazolo[4,3-b]pyridin-5-yl)-8-oxa-3-azabicyclo[3.2.1]octane (80 mg, 0.187 mmol) was dissolved in *N-*methylpyrrolidone (1 mL), and (3R)-3-methylmorpholine (37.7 mg, 0.373 mmol), RuphosPdG2 (14.5 mg, 0.019 mmol), cesium carbonate (182 mg, 0.560 mmol) and cuprous iodide (35.5 mg, 0.187 mmol) were added. The obtained mixture was stirred at 110°C for 2 hours under nitrogen protection. After the LC-MS monitored the reaction was completed, water (10 mL) was added to the reaction mixture. The aqueous phase was extracted with EA (20 mL×2) and washed with saturated saline (10 mL). Th organic phase was dried by rotary evaporation, and then was separated and purified by silica gel column (PE: EA=1:1) to obtain the target compound (39 mg, yield 42.4%, brown solid). LC-MS (ESI) *m*/*z:* 494.3 [M+H]⁺.

### Step 4: Synthesis of 3-(1-methyl-7-((R)-3-methylmorpholino)-3-(1H-pyrazol-3-yl)-1H-pyrazolo[4,3-b]pyridin-5-yl)-8-oxa-3-azabicyclo[3.2.1]octane

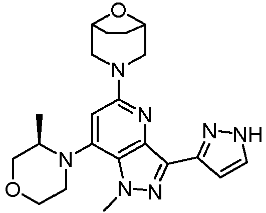

4 mol/L HCl solution in 1,4-dioxane (2 mL) was added to a solution of 3-(1-methyl-7-((R)-3-methylmorpholino)-3-(1-(tetrahydro-2*H*-pyran-2-yl)-1*H*-pyrazol-3-yl)-1*H*-pyrazolo[4,3-b]pyridin-5-yl)-8-oxa-3-azabicyclo[3.2.1]octane (39.0 mg, 0.079 mmol) in methanol (2 mL), the obtained mixture was stirred at room temperature for 2 hours under nitrogen protection. After the LC-MS monitored the reaction was completed, the solvent was removed under reduced pressure. The residue was separated and purified by prep-HPLC to obtain the target compound (4.2 mg, yield 13.0%, white solid). LC-MS (ESI) *m*/*z:* 410.2 [M+H]⁺. ¹H NMR (400 MHz, CD₃OD) δ7.82-7.73 (m, 1H), 6.89-6.85 (m, 1H), 6.50 (s, 1H), 4.26 (s, 3H), 4.07 - 3.94 (m, 2H), 3.91 - 3.78 (m, 4H), 3.74 - 3.64 (m, 1H), 3.65 - 3.55 (m, 1H), 3.51 - 3.37 (m, 3H), 3.17 - 3.02 (m, 2H), 2.09 - 2.00 (m, 2H), 2.02 - 1.93 (m, 2H), 1.17 (d, *J* = 6.4 Hz, 3H).

### Example 20: 3-(4-(3,3-dimethylmorpholino)-7-(1H-pyrazol-3-yl)imidazo[1,5-b]pyridazin-2-yl)-8-oxa-3-azabicyclo[3.2.1]octane

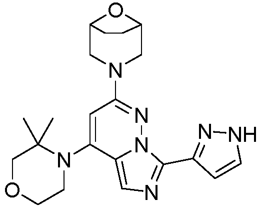

### Step 1: Synthesis of 4-(3,6-dichloropyridazin-4-yl)-3,3-dimethylmorpholine

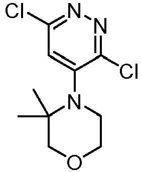

3,3-Dimethylmorpholine (4.00 g, 34.7 mmol) and DIEA (13.5 g, 104 mmol) were added to a solution of 3,4,6-trichloropyridine (7.64 g, 41.7 mmol) in N-methylpyrrolidone (30.0 mL), the reaction mixture was stirred at 100°C for 16 hours under nitrogen protection, and the reaction was stopped. Then the reaction solution was diluted with EA (70.0 mL), and washed with saturated saline (50.0 mL×3), dried over anhydrous sodium sulfate and filtered, and concentrated under reduced pressure. The residue was separated and purified by column chromatography (PE: EA=3:1) to obtain the target compound (2.00 g, yield 22.0%, yellow solid). LC-MS (ESI) *m*/*z* 262.1 [M+H]⁺. ¹H NMR (400 MHz, CDCl₃) δ 7.13 (s, 1H), 3.90 - 3.86 (m, 2H), 3.47 (s, 2H), 3.32 - 3.29 (m, 2H), 1.32 (s, 6H).

### Step 2: Synthesis of 3-(6-chloro-5-(3,3-dimethylmorpholino)pyridazin-3-yl)-8-oxa-3-azabicyclo[3.2.1]octane

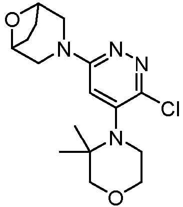

DIEA (3.70 g, 28.6 mmol) was added to a solution of 4-(3,6-dichloropyridazin-4-yl)-3,3-dimethylmorpholine (2.50 g, 9.54 mmol) and 8-oxa-3-azabicyclo[3.2.1]octane hydrochloride (2.14 g, 14.3 mmol) in *N-*methylpyrrolidone (30.0 mL). The reaction mixture was stirred at 145°C for 16 hours under nitrogen protection, and the reaction was stopped. Then the reaction solution was diluted with water (50.0 mL) and extracted with ethyl acetate (40.0 mL×3), the organic phases were combined and washed with saturated saline (30.0 mL), dried over anhydrous sodium sulfate and filtered, and concentrated under reduced pressure. The residue was separated and purified by column chromatography(petroleum ether: ethyl acetate=1:1) to obtain the target compound (2.50 g, yield 77.4%, yellow solid)._LC-MS (ESI) *m*/*z* 339.1 [M+H]⁺. ¹H NMR (400 MHz, CDCl₃) δ 6.49 (s, 1H), 4.54 - 4.51 (m, 2H), 3.87 - 3.84 (m, 2H), 3.79 (d, *J* = 12.1 Hz, 2H), 3.47 (s, 2H), 3.26 - 3.24 (m, 1H), 3.23 - 3.19 (m, 3H), 2.04 - 1.99 (m, 2H), 1.90 - 1.85 (m, 2H), 1.27 (s, 6H).

### Step 3: Synthesis of 6-(8-oxa-3-azabicyclo[3.2.1]octan-3-yl)-4-(3,3-dimethylmorpholino)pyridazine-3-carbonitrile

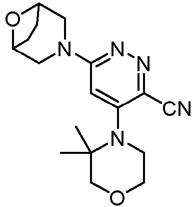

Bis(Diphenylphosphino)cyclopentadienyl iron (458 mg, 0.826 mmol) and tri(dibenzylidene acetone)dipalladium (378 mg, 0.413 mmol) were added to a solution of 3-(6-chloro-5-(3,3-dimethylmorpholino)pyridazin-3-yl)-8-oxa-3-azabicyclo[3.2.1]octane (1.40 g, 4.13 mmol) and zinc cyanide (970 mg, 8.26 mmol) in the *N,N*-dimethylformamide (30.0 mL). The reaction mixture was stirred at 135°C for 16 hours under nitrogen protection, and the reaction was stopped. Then the reaction solution was diluted with ethyl acetate (50.0 mL), and washed with saturated saline (30.0 mL×3), dried over anhydrous sodium sulfate and filtered, and concentrated under reduced pressure. The residue was separated and purified by column chromatography (PE: EA=1:1) to obtain the target compound (800 mg, yield 58.8%, yellow solid). LC-MS (ESI) *m*/*z* 330.2 [M+H]⁺.

### Step 4: Synthesis of (6-(8-oxa-3-azabicyclo[3.2.1]octan-3-yl)-4-(3,3-dimethylmorpholino)pyridazin-3-yl)methanamine

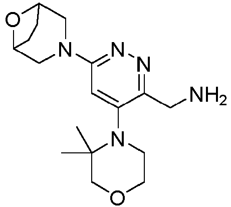

Raney nickel (428 mg, 7.29 mmol) and ammonia (1.00 mL) were added to a solution of 6-(8-oxa-3-azabicyclo[3.2.1]octan-3-yl)-4-(3,3-dimethylmorpholino)pyridazine-3-carbonitrile (800 mg, 2.43 mmol) in tetrahydrofuran (15.00 mL). The reaction mixture was reacted at room temperature for 16 hours under hydrogen atmosphere, and the reaction was stopped. Then the reaction solution was filtered and concentrated under reduced pressure, and the resulting residue was the target compound (500 mg, yield 61.7%, yellow oil). LC-MS (ESI) *m*/*z* 334.1 [M+H]⁺.

### Step 5: Synthesis of N-((6-(8-oxa-3-azabicyclo[3.2.1]octan-3-yl)-4-(3,3-dimethylmorpholino)pyridazin-3-yl)methyl)-1H-pyrazole-5-carboxamide

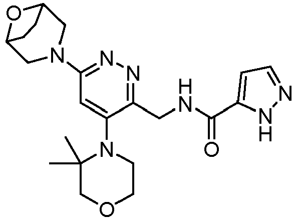

(684 mg, 1.80 mmol) and DIEA (581 mg, 4.50 mmol) were added to a solution of (6-(8-oxa-3-azabicyclo[3.2.1]octan-3-yl)-4-(3,3-dimethylmorpholino)pyridazin-3-yl)methanamine (500 mg, 1.50 mmol) and 1*H*-pyrazol-5-carboxylic acid (151 mg, 1.35 mmol) in tetrahydrofuran (15.0 mL), the reaction mixture was stirred at room temperature for 1 hour and then the reaction was stopped. Then water (30.0 mL) was added and extracted with ethyl acetate (25.0 mL×3), the organic phases were combined and washed with saturated saline (20.0 mL), dried over anhydrous sodium sulfate and filtered, concentrated under reduced pressure. The residue was separated and purified by column chromatography (DCM: methanol=10:1) to obtain the target compound (300 mg, yield 46.8%, yellow solid). LC- MS (ESI) *m*/*z* 428.4 [M+H]⁺. ¹H NMR (400 MHz, CDCl₃) δ 9.16 (s, 1H), 7.60 - 7.56 (m, 1H), 6.81 - 6.77 (m, 1H), 6.65 (s, 1H), 5.29 (s, 1H), 4.91 - 4.78 (m, 2H), 4.55 - 4.50 (m, 2H), 3.97 - 3.89 (m, 2H), 3.84 - 3.76 (m, 2H), 3.55 (s, 2H), 3.26 - 3.21 (m, 2H), 3.19 - 3.09 (m, 2H), 2.04 - 1.99 (m, 2H), 1.91 - 1.85 (m, 2H), 1.16 (s, 6H).

### Step 6: Synthesis of 3-(4-(3,3-dimethylmorpholino)-7-(1H-pyrazol-3-yl)imidazo[1,5-b]pyridazin-2-yl)-8-oxa-3-azabicyclo[3.2.1]octane

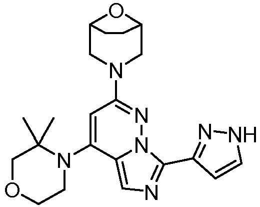

Phosphorus oxychloride (6.00 mL) was added to a solution of N-((6-(8-oxa-3-azabicyclo[3.2.1]octan-3-yl)-4-(3,3-dimethylmorpholino)pyridazin-3-yl)methyl)-U/-pyrazole-5-carboxamide (250 mg, 0.585 mmol) in acetonitrile (5.00 mL) at room temperature. The reaction mixture was reacted at 100°C for 1 hour and the reaction was stopped. The reaction solution was concentrated under reduced pressure, and the residue was purified by reverse phase preparative chromatography (acetonitrile/water with 0.05% formic acid) to obtain the target compound (58.9 mg, yield 24.6%, white solid). LC- MS (ESI) *m*/*z* 410.1[M+H]⁺. ¹H NMR (400 MHz, CDCl₃) δ 7.75 - 7.69 (m, 1H), 7.44 (s, 1H), 7.09 - 7.05 (m, 1H), 5.99 (s, 1H), 4.60 - 4.54 (m, 2H), 3.94 - 3.89 (m, 2H), 3.67 - 3.62 (m, 2H), 3.52 (s, 2H), 3.50 - 3.46 (m, 2H), 3.36 - 3.31 (m, 2H), 2.09 - 2.02 (m, 2H), 1.96 - 1.89 (m, 2H), 1.35 (s, 6H).

### Example 23 : 4-(5-((1R,5S)-8-oxa-3-azabicyclo[3.2.1]octan-3-yl)-3-(1H-pyrazol-5-yl)pyrazolo[1,5-a]pyrimidin-7-yl)tetrahydro-2H-pyran-4-ol

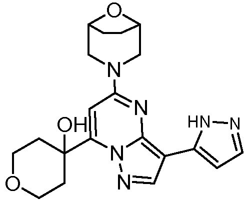

### Step 1: Synthesis of 3-(pyrazolo[1,5-a]pyrimidin-5-yl)-8-oxa-3-azabicyclo[3.2.1]octane

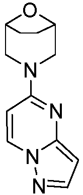

DIEA (12.6 g, 97.7 mmol) was added to a solution of 5-chloropyrazolo[1,5-a]pyrimidine (5.00 g, 32.6 mmol) and 8-oxa-3-azabicyclo[3.2.1]octane hydrochloride (7.31 g, 48.8 mmol) in *N-*methylpyrrolidone (60.0 mL). The reaction mixture was stirred at 100°C for 1 hour under nitrogen protection, and the reaction was stopped. Then the reaction solution was diluted with ethyl acetate (85.0 mL), and washed with saturated saline (55 mL×3), dried over anhydrous sodium sulfate and filtered, and concentrated under reduced pressure. The residue was separated and purified by column chromatography (PE: EA=5:1) to obtain the target compound (3.60 g, yield 48.0%, yellow solid). LC-MS (ESI) *m*/*z* 231.1 [M+H]⁺. ¹H NMR (400 MHz, CDCl₃) δ 8.33 (d, *J* = 7.8 Hz, 1H), 7.88 (d, *J* = 2.0 Hz, 1H), 6.26 (d, *J* = 7.8 Hz, 1H), 6.13 (d, *J* = 1.9 Hz, 1H), 4.57 - 4.47 (m, 2H), 3.92 (d, *J* = 12.5 Hz, 2H), 3.27 (dd, *J=* 12.6, 2.4 Hz, 2H), 2.06 - 1.94 (m, 2H), 1.87 - 1.74 (m, 2H).

### Step 2: Synthesis of 4-(5-((1R,SS)-8-oxa-3-azabicyclo[3.2.1]octan-3-yl)pyrazolo[1,5-a]pyrimidin-7-yl)tetrahydro-2H-pyran-4-ol

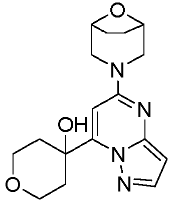

Under nitrogen atmosphere, a solution of (1R,5S)-3-(pyrazolo[1,5-a]pyrimidin-5-yl)-8-oxa-3-azabicyclo[3.2.1]octane (1.50 g, 6.51 mmol) in tetrahydrofuran (20.0 mL) was cooled to -78°C. Then n-butyl lithium (8.14 mL, 13.0 mmol, 1.6 M) was added dropwise, the reaction mixture was stirred at -78°C for 1 hour, then tetrahydro-4H-pyran-4-one (1.30 g, 13.0 mmol) was added dropwise slowly, the reaction solution was stirred at room temperature overnight, and the reaction was stopped. Ammonium chloride aqueous solution (45.0 mL) was added to dilute the reaction solution, and then extracted with EA (45.0 mL×3), the organic phases were combined and washed with saturated saline (35.0 mL), dried over anhydrous sodium sulfate, filtered, and concentrated under reduced pressure. The residue was separated and purified by column chromatography (PE: EA=1:1) to obtain the target compound (500 mg, yield 23.2%, yellow solid). LC- MS (ESI) *m*/*z* 331.1 [M+H]⁺. ¹H NMR (400 MHz, CDCl₃) δ 7.86 (d, *J=* 2.3 Hz, 1H), 6.21 (d, *J=* 2.2 Hz, 1H), 6.15 (s, 1H), 4.56 - 4.50 (m, 2H), 4.31 - 4.27 (m, 2H), 4.23 - 4.19 (m, 2H), 3.77 - 3.76 (m, 2H), 3.33 - 3.26 (m, 2H), 2.27 - 2.20 (m, 2H), 2.12 - 2.07 (m, 2H), 2.03 - 1.96 (m, 2H), 1.88 - 1.82 (m, 2H).

### Step 3: Synthesis of 4-(5-((1R,5S)-8-oxa-3-azabicyclo[3.2.1]octan-3-yl)-3-iodopyrazolo[1,5-a]pyrimidin-7-yl)tetrahydro-2H-pyran-4-ol

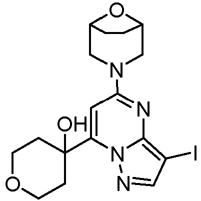

*N*-iodosuccinimide (272 mg, 1.21 mmol) was added to a solution of 4-(5-((1*R*,5*S*)-8-oxa-3-azabicyclo[3.2.1]octan-3-yl)pyrazolo[1,5-a]pyrimidin-7-yl)tetrahydro-2*H*-pyran-4-ol (400 mg, 1.21 mmol) in acetonitrile (10.0 mL), the reaction mixture was stirred at room temperature for 1 hour, and the reaction was stopped. Then water (35.0 mL) was added to dilute the solution, extracted with EA (30.0 mL×3), the organic phases were combined and washed with saturated saline (30.0 mL), dried over anhydrous sodium sulfate and filtered, and concentrated under reduced pressure. The residue was separated and purified by column chromatography (PE: EA=1:1) to obtain the target compound (400 mg, yield 72.4%, yellow solid). LC- MS (ESI) *m*/*z* 457.1 [M+H]⁺.

### Step 4: Synthesis of 4-(5-((1R,5S)-8-oxa-3-azabicyclo[3.2.1]octan-3-yl)-3-(1-(tetrahydro-2H-pyran-2-yl)-1H-pyrazol-5-yl)pyrazolo[1,5-a]pyrimidin-7-yl)tetrahydro-2H-pyran-4-ol

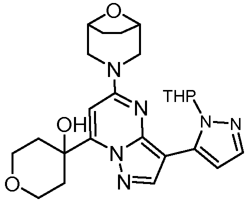

Under nitrogen protection, dichloro[1,1'-bis(ditertbutyl phosphine)ferrocene palladium (55.9 mg, 0.0877 mmol) and potassium phosphate (558 mg, 2.63 mmol) were added to a solution of 4-(5-((1*R*,5*S*)-8-oxa-3-azabicyclo[3.2.1]octan-3-yl)-3-iodopyrazolo[1,5-a]pyrimidin-7-yl)tetrahydro-2*H-*pyran-4-ol (400 mg, 0.877 mmol) and 1-(tetrahydro-2H-pyran-2-yl)-5-(4,4,5,5-tetramethyl-1,3,2-dioxybenzofuran-2-yl)-1*H*-pyrazole (366 mg, 1.31 mmol) in 1,4-dioxane (10.0 mL) and water (2.00 mL), the reaction mixture was stirred at 100°C for 16 hours, and the reaction was stopped. Then water (35.0 mL) was added to dilute the solution, then extracted with EA (35.0 mL×3), the organic phases were combined and washed with saturated saline (35.0 mL), dried over anhydrous sodium sulfate and filtered, and concentrated under reduced pressure. The residue was separated and purified by column chromatography (PE: EA=1:1) to obtain the target compound (150 mg, yield 35.6%, yellow solid). LC-MS (ESI) *m*/*z* 481.4 [M+H]⁺.

### Step 5: Synthesis of 4-(5-((1R,5S)-8-oxa-3-azabicyclo[3.2.1]octan-3-yl)-3-(1H-pyrazol-5-yl)pyrazolo[1,5-a]pyrimidin-7-yl)tetrahydro-2H-pyran-4-ol

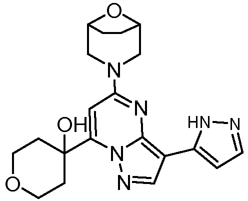

4-(5-(8-Oxa-3-azabicyclo[3.2.1]octan-3-yl)-3-(1-(tetrahydro-2*H*-pyran-2-yl)-1*H*-pyrazol-5-yl)pyrazolo[1,5-a]pyrimidin-7-yl)tetrahydro-2*H*-pyran-4-ol (150 mg, 0.312 mmol) and ethyl acetate hydrochloride (10.0 mL, 3M) were added to a reaction flask at room temperature, the reaction mixture was stirred at room temperature for 2 hours, and the reaction was stopped. The reaction was concentrated under reduced pressure, and the resulting residue was purified by reverse phase preparative chromatography (acetonitrile/water with 0.05% formic acid) to obtain the target compound (66.6 mg, yield 53.8%, white solid). LC- MS (ESI) *m*/*z* 397.2 [M+H]⁺. ¹H NMR (400 MHz, CDCl₃) δ 8.21 (s, 1H), 7.64 (s, 1H), 6.56 (s, 1H), 6.20 (s, 1H), 4.59 - 4.55 (m, 2H), 4.11 - 4.05 (m, 2H), 4.04 - 3.93 (m, 2H), 3.93 - 3.87 (m, 2H), 3.40 - 3.34 (m, 2H), 2.26 - 2.20 (m, 2H), 2.13 - 2.02 (m, 4H), 1.88 - 1.80 (m, 2H).

### Example 26: 3-(7-(1-methyl-1Hpyrazol-5-yl)-3-(1H-pyrazol-3-yl)pyrazolo[1,5-a]pyrimidin-5-yl)-8-oxa-3-azabicyclo[3.2.1]octane

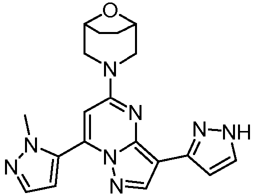

### Step 1: Synthesis of 5-chloro-7-(1-methyl-1H-pyrazol-5-yl)pyrazolo[1,5-a]pyrimidine

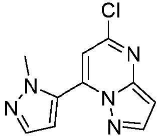

At room temperature, sodium carbonate (1.69 g, 16.0 mmol) and [1,1'- bis(diphenylphosphino) ferrocene]palladium dichloride (340 mg, 0.532 mmol) were added to a mixed solution of 5,7-dichloropyrazo[1,5-a]pyrimidine (1.00 g, 5.32 mmol) and 1-methyl-5-(4,4,5,5-tetramethyl-1,3,2-dioxaboran-2-yl)-1*H*-pyrazole (1.33 g, 6.38 mmol) in water (1.00 mL) and dioxane (4.00 mL) under nitrogen protection. The reaction was stopped after the reaction mixture was stirred at 90°C under nitrogen protection for 10 hours. After the reaction solution was cooled, water (20.0 mL) was added and then extracted with EA (30.0 mL×2), the organic phases were combined and washed with saturated saline (20.0mL), dried over anhydrous sodium sulfate, and concentrated under reduced pressure. The residue was separated and purified by normal chromatography column (EA: PE=1:1) to obtain crude product (370mg, yield 29.7%, yellow solid). LC-MS (ESI) *m*/*z* 233.9[M+H]⁺.

### Step 2: Synthesis of 3-(7-(1-methyl-1H-pyrazol-5-yl)pyrazolo[1,5-a]pyrimidin-5-yl)-8-oxa-3-azabicyclo[3.2.1]octane

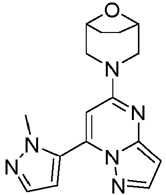

DIEA (613 mg, 4.75 mmol) was added to a solution of 5-chloro-7-(1-methyl-1*H*-pyrazol-5-yl)pyrazolo[1,5-a]pyrimidine (370 mg, 1.58 mmol) and 8-oxa-3-azabicyclo[3.2.1]octane hydrochloride (358 mg, 2.39 mmol) in NMP (15.0 mL) at room temperature. The reaction mixture was reacted at 140°C for 2 hours in microwave reactor under nitrogen protection, and the reaction was stopped. Then the reaction solution was diluted with EA (35.0 mL), and washed with saturated saline (30.0 mL×3), dried over anhydrous sodium sulfate and filtered, and concentrated under reduced pressure. The residue was separated and purified by column chromatography (PE: EA=1:1) to obtain the target compound (260 mg, yield 52.9%, yellow oil). LC-MS (ESI) *m*/*z* 311.2 [M+H]⁺. ¹H NMR (400 MHz, CDCl₃) δ 7.92 (d, J = 2.2 Hz, 1H), 7.61 (d, *J=* 1.9 Hz, 1H), 6.63 (d, *J=* 1.9 Hz, 1H), 6.30 (s, 1H), 6.23 (d, *J=* 2.2 Hz, 1H), 4.55 - 4.50 (m, 2H), 3.97 - 3.90 (m, 2H), 3.88 (s, 3H), 3.33 - 3.27 (m, 2H), 2.04 - 1.98 (m, 2H), 1.90 - 1.82 (m, 2H).

### Step 3: Synthesis of 3-(3-iodo-7-(1-methyl-1H-pyrazol-5-yl)pyrazolo[1,5-a]pyrimidin-5-yl)-8-oxa-3-azabicyclo[3.2.1]octane

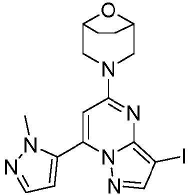

NIS (188 mg, 0.837 mmol) was added to a solution of 3-(7-(1-methyl-1*H*-pyrazol-5-yl)pyrazolo[1,5-a]pyrimidin-5-yl)-8-oxa-3-azabicyclo[3.2.1]octane (260 mg, 0.837 mmol) in acetonitrile (10.0 mL), the reaction mixture was stirred at room temperature for 3 hours, and the reaction was stopped. Then water (15.0 mL) was added, extracted with EA (20.0 mL×3), the organic phases were combined and washed with saturated saline (15.0 mL), dried over anhydrous sodium sulfate and filtered, and concentrated under reduced pressure. The residue was separated and purified by column chromatography (PE: EA=1:1) to obtain the target compound (300 mg, yield 82.1%, yellow solid). LC- MS (ESI) m/z 436.9 [M+H]⁺. ¹H NMR (400 MHz, CDCl₃) δ 7.90 (s, 1H), 7.62 (d, *J* = 1.9 Hz, 1H), 6.61 (d, *J* = 1.9 Hz, 1H), 6.30 (s, 1H), 4.57 - 4.52 (m, 2H), 4.11 - 3.89 (m, 2H), 3.85 (s, 3H), 3.37 - 3.30 (m, 2H), 2.05 - 1.99 (m, 2H), 1.90 - 1.82 (m, 2H).

### Step 4: Synthesis of 3-(7-(1-methyl-1H-pyrazol-5-yl)-3-(1-(tetrahydro-2H-pyran-2-yl)-1H-pyrazol-3-yl)pyrazolo[1,5-a]pyrimidin-5-yl)-8-oxa-3-azabicyclo[3.2.1]octane

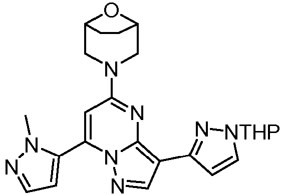

Pd(dtbpf)Cl₂ (43.8 mg, 0.0687 mmol) and potassium phosphate (437 mg, 0.2.06 mmol) were added to a solution of 3-(3-iodo-7-(1-methyl-1*H*-pyrazol-5-yl)pyrazolo[1,5-a]pyrimidin-5-yl)-8-oxa-3-azabicyclo[3.2.1]octane (300 mg, 0.687 mmol) and 1-(tetrahydro-2*H*-pyran-2-yl)-3-(4,4,5,5-tetramethyl-1,3,2-dioxaboran-2-yl)-1*H*-pyrazole (286 mg, 1.03 mmol) in 1,4-dioxane (10.0 mL) and water (2.00 mL) under nitrogen protection, the reaction mixture was stirred at 60°C for 16 hours and the reaction was stopped. Then water (30.0 mL) was added to dilute the solution, and then extracted with EA (30.0 mL×3), the organic phases were combined and washed with saturated saline (25.0 mL), dried over anhydrous sodium sulfate and filtered, and concentrated under reduced pressure. The residue was separated and purified by column chromatography (PE: EA=1:1) to obtain the target compound (150 mg, yield 47.3%, yellow solid). LC-MS (ESI) m/z 461.1 [M+H]⁺. ¹H NMR (400 MHz, CDCl₃) δ 8.16 (s, 1H), 7.68 (d, *J=* 1.5 Hz, 1H), 7.64 (d, *J=* 1.9 Hz, 1H), 6.71 - 6.63 (m, 2H), 6.38 (s, 1H), 5.51 - 5.39 (m, 1H), 4.60 - 4.51 (m, 2H), 4.09 - 3.95 (m, 2H), 3.93 (s, 3H), 3.72 - 3.61 (m, 1H), 3.39 - 3.30 (m, 2H), 2.72 - 2.59 (m, 1H), 2.16 - 2.09 (m, 1H), 2.02 - 1.96 (m, 2H), 1.88 - 1.77 (m, 3H), 1.64 - 1.55 (m, 2H).

### Step 6: Synthesis of 3-(7-(1-methyl-1H-pyrazol-5-yl)-3-(1H-pyrazol-3-yl)pyrazolo[1,5-a]pyrimidin-5-yl)-8-oxa-3-azabicyclo[3.2.1]octane

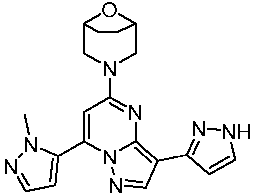

3 -(7-(1-Methyl-1*H*-pyrazol-5-yl)-3-(1-(tetrahydro-2*H*-pyran-2-yl)-1*H*-pyrazol-3-yl)pyrazolo[1,5-a]pyrimidin-5-yl)-8-oxa-3-azabicyclo[3.2.1]octane (150 mg, 0.325 mmol) and hydrochloric acid in EA solution (5.00 mL, 3M) were added to a reaction flask at room temperature, the reaction mixture was stirred at room temperature for 2 hours, and the reaction was stopped. The reaction was concentrated under reduced pressure and the resulting residue was purified by reverse phase preparative chromatography (acetonitrile/water with 0.05% formic acid) to obtain the target compound (82.68 mg, yield 67.4%, light yellow solid). LC- MS (ESI) m/z 377.2 [M+H]⁺. ¹H NMR (400 MHz, DMSO-*d₆*) δ 12.89 - 12.58 (m, 1H), 8.38 - 8.19 (m, 1H), 7.80 - 7.45 (m, 2H), 6.89 - 6.68 (m, 3H), 4.52 - 4.46 (m, 2H), 4.31 - 4.03 (m, 2H), 3.85 (s, 3H), 3.23 - 3.15 (m, 2H), 1.89 - 1.74 (m, 4H).

### Example 27: 3-(8-(1-methyl-1Hpyrazol-5-yl)-3-(1H-pyrazol-3-yl)imidazo[1,2-b]pyridazin-6-yl)-8-oxa-3-azabicyclo[3.2.1]octane

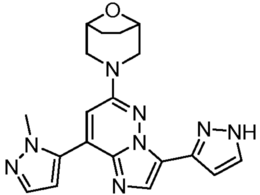

### Step 1: Synthesis of 8-bromo-6-chloro-3-iodoimidazo[1,2-b]pyridazine

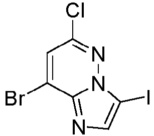

NIS (1.94 g, 8.60 mmol) was added to a solution of 8-bromo-6-chloroimidazolo[1,2-b]pyridazine (2.00 g, 8.60 mmol) in acetonitrile (30.0 mL), the reaction mixture was stirred at room temperature for 2 hours, and the reaction was stopped. Then water (45.0 mL) was added, extracted with EA (40.0 mL×3), the organic phases were combined and washed with saturated saline (40.0 mL), dried over anhydrous sodium sulfate and filtered, and concentrated under reduced pressure. The residue was separated and purified by column chromatography (PE: EA=5:1) to obtain the target compound (3.00 g, yield 97.3%, yellow solid). LC-MS (ESI) *m*/*z* 357.8 [M+H]⁺, 359.8 [M+2+H]⁺.

### Step 2: Synthesis of 8-bromo-6-chloro-3-(1-(tetrahydro-2H-pyran-2-yl)-1H-pyrazol-3-yl)imidazo[1,2-b]pyridazine

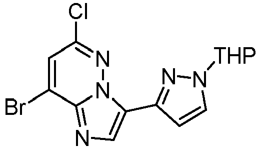

Under nitrogen protection, tetratriphenylphosphine palladium (644 mg, 0.558 mmol) and potassium phosphate (3.55 g, 16.7 mmol) were added to a solution of 8-bromo-6-chloro-3-iodoimidazo[1,2-b]pyridazine (2.00 g, 5.58 mmol) and 1-(tetrahydro-2H-pyran-2-yl)-3-(4,4,5,5-tetramethyl-1,3,2-dioxaboran-2-yl)-1*H*-pyrazole (1.55 g, 5.58 mmol) in 1,4-dioxane (20.0 mL) and water (4.00 mL).The reaction was stirred at room temperature for 16 hours and the reaction was stopped. Then water (45.0 mL) was added, extracted with EA (45.0 mL×3), the organic phases were combined and washed with saturated saline (45.0 mL), dried over anhydrous sodium sulfate and filtered, and concentrated under reduced pressure. The residue was separated and purified by column chromatography (PE: EA=3:1) to obtain the target compound (400 mg, yield 18.7%, yellow solid). LC-MS (ESI) m/z 382.0 [M+H]⁺, 384.0 [M+2+H]⁺. ¹H NMR (400 MHz, CDCl₃) δ 8.10 (s, 1H), 7.73 (s, 1H), 7.46 (s, 1H), 6.91 (s, 1H), 5.32-5.27 (m, 1H), 4.11- 4.06 (m, 1H), 3.68-3.62 (m, 1H), 2.65-2.56 (m, 1H), 2.02 - 1.97 (m, 2H), 1.77-1.70 (m, 1H), 1.63-1.57 (m, 2H).

### Step 3: Synthesis of 6-chloro-8-(1-methyl-1H-pyrazol-5-yl)-3-(1-(tetrahydro-2H-pyran-2-yl)-1H-pyrazol-3-yl)imidazo[1,2-b]pyridazine

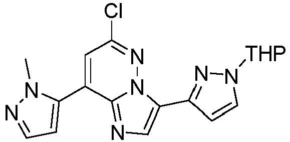

Under nitrogen protection, 1,1-bis(diphenylphosphino) ferrocene palladium chloride (76.5 mg, 0.105 mmol) and potassium phosphate (666 mg, 3.14 mmol) were added to a solution of 8-bromo-6-chloro-3-(1-(tetrahydro-2*H*-pyran-2-yl)-1*H*-pyrazol-3-yl)imidazo[1,2-b]pyridazine (400 mg, 1.05 mmol) and 1-methyl-5-(4,4,5,5-tetramethyl-1,3,2-dioxaboran-2-yl)-1*H*-pyrazole (217 mg, 1.05 mmol) in 1,4-dioxane (10.0 mL) and and water (2.00 mL), the reaction mixture was stirred at 60°C for 2 hours and the reaction was stopped. Then water (25.0 mL) was added, extracted with EA (25.0 mL×3), the organic phases were combined and washed with saturated saline (20.0 mL), dried over anhydrous sodium sulfate and filtered, and concentrated under reduced pressure. The residue was separated and purified by column chromatography (PE: EA=1:1) to obtain the target compound (150 mg, yield 37.4%, yellow solid). LC-MS (ESI) *m*/*z* 384.0 [M+H]⁺. ¹H NMR (400 MHz, CDCl₃) δ 8.10 (s, 1H), 7.73 (s, 1H), 7.46 (s, 1H), 6.91 (s, 1H), 5.32 - 5.27 (m, 1H), 4.11 - 4.06 (m, 1H), 3.68 - 3.62 (m, 1H), 2.65 - 2.56 (m, 1H), 2.02 - 1.97 (m, 2H), 1.77 - 1.70 (m, 1H), 1.63 - 1.57 (m, 2H).

### Step 4: Synthesis of 3-(8-(1-methyl-1H-pyrazol-5-yl)-3-(1-(tetrahydro-2H-pyran-2-yl)-1H-pyrazol-3-yl)imidazo[1,2-b]pyridazin-6-yl)-8-oxa-3-azabicyclo[3.2.1]octane

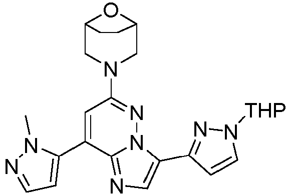

Under nitrogen protection, cesium carbonate (382 mg, 1.1 mL) and 8-oxa-3-azabicyclo[3.2.1] octane hydrochloride (66.3 mg, 0.443 mmol) were added to a solution of 6-chloro-8-(1-methyl-1*H-*pyrazol-5-yl)-3-(1-(tetrahydro-2*H*-pyran-2-yl)-1*H*-pyrazol-3-yl)imidazo[1,2-b]pyridazine (150 mg, 0.391 mmol) and (2-amino-2-[1,1'-biphenyl]-2-yl)(dicyclohexyl(2,6-diisopropoxy-2-[1,1'-biphenyl]-2-yl)-phosphoryl)palladium (II) chloride (39.1 mg, 0.0391 mmol) in toluene (6.00 mL), the reaction mixture was stirred at 110°C for 16 hours and the reaction was stopped. Then water (15.0 mL) was added, extracted with EA (15.0 mL×3), the organic phases were combined and washed with saturated saline (20.0 mL), dried over anhydrous sodium sulfate and filtered, and concentrated under reduced pressure. The residue obtained was separated and purified by column chromatography (PE: EA=1:1) to obtain the target compound (60.0 mg, yield 33.3%, yellow solid). LC-MS (ESI) *m*/*z* 461.2 [M+H]⁺.

### Step 5: Synthesis of 3-(8-(1-methyl-1H-pyrazol-5-yl)-3-(1H-pyrazol-3-yl)imidazo[1,2-b]pyridazin-6-yl)-8-oxa-3-azabicyclo[3.2.1]octane

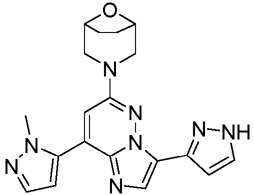

At room temperature, 3-(8-(1-methyl-1*H*-pyrazol-5-yl)-3-(1-(tetrahydro-2*H*-pyran-2-yl)-1*H-*pyrazol-3-yl)imidazo[1,2-b]pyridazin-6-yl)-8-oxa-3-azabicyclo[3.2.1]octane (60.0 mg, 0.130 mmol) and hydrochloric acid in EA solution (5.00 mL, 3M) were added to a reaction flask, the reaction mixture was stirred at room temperature for 2 hours, and the reaction was stopped. The reaction was concentrated under reduced pressure and the resulting residue was purified by reverse phase preparative chomatography (acetonitrile/water with 0.05% formic acid) to obtain the target compound (18.0 mg, yield 36.7%, white solid). LC-MS (ESI) *m*/*z* 377.2 [M+H]⁺. ¹H NMR (400 MHz, MeOD-*d₄*) δ 7.98 (s, 1H), 7.82 - 7.74 (m, 1H), 7.63 (d, *J=* 1.9 Hz, 1H), 7.24 - 7.18 (m, 1H), 7.17 (s, 1H), 6.71 (d, *J* = 1.9 Hz, 1H), 4.58 - 4.52 (m, 2H), 3.93 (s, 4H), 3.90 - 3.88 (m, 1H), 3.30 - 3.28 (m, 1H), 3.28 - 3.25 (m, 1H), 2.04 - 1.97 (m, 4H).

### Example 28: 3-(7-(8-oxa-3-azabicyclo[3.2.1]octan-3-yl)-3-(1H-pyrazol-5-yl)pyrazolo[1,5-a]pyrimidin-5-yl)-8-oxa-3-azabicyclo[3.2.1]octane

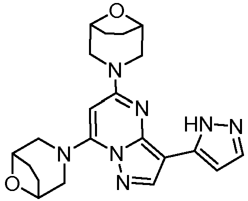

### Step 1: Synthesis of 3-(7-(8-oxa-3-azabicyclo[3.2.1]octan-3-yl)pyrazolo[1,5-a]pyrimidin-5-yl)-8-oxa-3-azabicyclo[3.2.1]octane

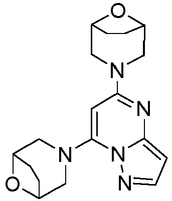

DIEA (2.06 g, 16.0 mmol) was added to a solution of 5,7-dichloropyrazo[1,5-a]pyrimidine (1.00 g, 5.32 mmol) and (1*R*,5*S)*-8-oxa-3-azabicyclo [3.2.1] octane hydrochloride (2.39 g, 16.0 mmol) in N-methylpyrrolidone (15.0 mL), the reaction mixture was stirred at 145°C for 16 hours, and the reaction was stopped. After the reaction mixture was cooled to room temperature, water (20.0 mL) was added to dilute the solution, extracted with EA (25.0 mL×3), the organic phases were combined, then washed with water (20.0 mL×3) and saturated saline (20.0 mL×3), dried over anhydrous sodium sulfate and filtered, and the filtrate was concentrated under reduced pressure. The residue was separated and purified by column chromatography (PE: EA=10:1-1:1) to obtain the target compound (550 mg, yield 30.3%, white solid). LC-MS (ESI) *m*/*z* 342.3 [M+H]⁺.

### Step 2: Synthesis of 3-(7-(8-oxa-3-azabicyclo[3.2.1]octan-3-yl)-3-iodopyrazolo[1,5-a]pyrimidin-5-yl)-8-oxa-3-azabicyclo[3 .2.1]octane

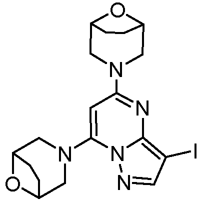

N-iodosuccinimide (399 mg, 1.77 mmol) was added to a solution of 3-(7-(8-oxa-3-azabicyclo[3.2.1]octan-3-yl)pyrazolo[1,5-a]pyrimidin-5-yl)-8-oxa-3-azabicyclo[3.2.1]octane (550 mg, 1.61 mmol) in acetonitrile (20.0 mL). The reaction was stopped after the reaction mixture was stirred at room temperature for 2 hours. The reaction mixture was concentrated under reduced pressure, and the residue obtained was purified by column chromatography (PE: EA=5:1-1:1) to obtain the target compound (500 mg, yield 66.4%, yellow solid). LC-MS (ESI) *m*/*z* 468.3 [M+H]⁺. ¹H NMR (400 MHz, DMSO-*d₆*) δ7.90 (s, 1H), 5.70 (s, 1H), 4.45 - 4.38 (m, 4H), 4.08 - 3.98 (m, 4H), 3.09 - 3.00 (m, 4H), 2.07 - 1.98 (m, 2H), 1.90 - 1.78 (m, 4H), 1.75 - 1.65 (m, 2H).

### Step 3: Synthesis of 3-(7-(8-oxa-3-azabicyclo[3.2.1]octan-3-yl)-3-(1-(tetrahydro-2H-pyran-2-yl)-1H-pyrazol-5-yl)pyrazolo[1,5-a]pyrimidin-5-yl)-8-oxa-3-azabicyclo[3.2.1]octane

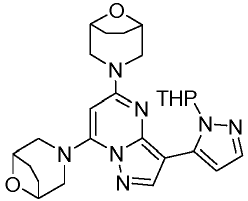

Potassium carbonate (177 mg, 1.28 mmol) and [1,1'-bis (di-tertbutylphosphine)ferrocene] palladium dichloride (42.0 mg, 0.0642 mmol) were added to a mixed solution of 3-(7-(8-oxa-3-azabicyclo[3.2.1]octan-3-yl)-3-iodopyrazolo[1,5-a]pyrimidin-5-yl)-8-oxa-3-azabicyclo[3.2.1]octane (300 mg, 0.642 mmol) and 1-(tetrahydro-2H-pyran-2-yl)-5-(4,4,5,5-tetramethyl-1,3,2-dioxybenzofuran-2-yl)-1*H*-pyrazole (268 mg, 0.963 mmol) in water (1.00 mL) and 1,4-dioxane (5.00 mL). The reaction was stopped after the reaction mixture was stirred at 60°C for 8 hours under nitrogen protection. After the reaction solution was cooled to room temperature, water (10.0 mL) was added,then extracted with EA (15.0 mL×3), the organic phase was washed with saturated saline (15.0 mL×2), dried over anhydrous sodium sulfate and filtered, and the filtrate was concentrated under reduced pressure. The residue was separated and purified by column chromatography (PE: EA=5:1-1:3) to obtain the target compound (150 mg, yield 47.5%, yellow solid). LC-MS (ESI) *m*/*z* 492.4 [M+H]⁺.

### Step 4: Synthesis of 3-(7-(8-oxa-3-azabicyclo[3.2.1]octan-3-yl)-3-(1H-pyrazol-5-yl)pyrazolo[1,5-a]pyrimidin-5-yl)-8-oxa-3-azabicyclo[3.2.1]octane

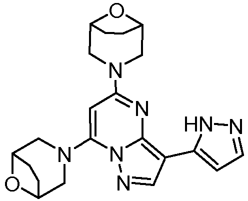

A solution of 3-(7-(8-oxa-3-azabicyclo[3.2.1]octan-3-yl)-3-(1-(tetrahydro-2*H*-pyran-2-yl)-1*H-*pyrazol-5-yl)pyrazolo[1,5-a]pyrimidin-5-yl)-8-oxa-3-azabicyclo[3.2.1]octane (120 mg, 0.244 mmol) in hydrogen chloride methanol (3.0 M, 3.00 mL) was stirred at room temperature for 1 hour and then the reaction was stopped. The reaction mixture was concentrated under reduced pressure, and the residue obtained was separated and purified by reverse phase preparative chromatography (acetonitrile/water with 0.05% formic acid) to obtain the target compound (33.96 mg, yield 34.1%, white solid). LC-MS (ESI) *m*/*z* 408.2 [M+H]⁺. ¹H NMR (400 MHz, DMSO-*d₆*) δ 12.64 (Brs, 1H), 8.23 (s, 1H), 7.55 (s, 1H), 6.69 (s, 1H), 5.72 (s, 1H), 4.49 - 4.41 (m, 4H), 4.16 - 4.04 (m, 4H), 3.09 (d, *J*= 10.9 Hz, 4H), 2.11 - 2.01 (m, 2H), 1.92 - 1.80 (m, 4H), 1.77 - 1.68 (m, 2H).

### Example 29: 1-((R)-4-(2-((1R,5S)-8-oxa-3-azabicyclo[3.2.1]octan-3-yl)-7-(1H-pyrazol-3-yl)imidazol[1,5-b]pyridazin-4-yl)-3-methylpiperazin-1-yl)ethan-1-one

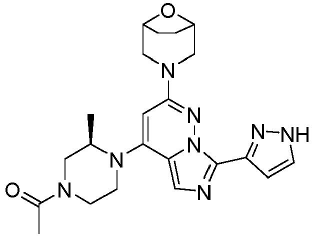

### Step 1: Synthesis of tert-butyl (R)-4-(3,6-dichloropyridazin-4-yl)-3-methylpiperazine-1-carboxylate

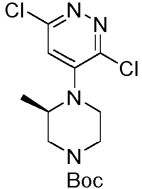

3,4,6-Trichloropyridazine (5.49 g, 29.9 mmol) and DIEA (9.68 g, 74.9 mmol) were added to a solution of tert-butyl (A)-3-methylpiperazine-1-carboxylate (5.00 g, 24.9 mmol) in N-methylpyrrolidone (35.0 mL), the reaction mixture was stirred at room temperature for 16 hours under nitrogen protection, and the reaction was stopped. Then water (60.0 mL) was added to dilute the solution, and then extracted with ethyl acetate (50.0 mL×3), the organic phase was washed with saturated saline (40.0 mL), dried over anhydrous sodium sulfate and filtered, and concentrated under reduced pressure. The residue was separated and purified by column chromatography (petroleum ether: ethyl acetate=5:1) to obtain the target compound (3.50 g, yield 40.4%, yellow solid). LC- MS (ESI) *m*/*z* 346.9 [M+H]⁺. ¹H NMR (400 MHz, CDCl₃) δ 6.83 (s, 1H), 4.21 - 4.16 (m, 1H), 4.14 - 4.08 (m, 1H), 3.83 (d, *J=* 13.1 Hz, 1H), 3.42 - 3.29 (m, 2H), 3.13 - 3.09 (m, 2H), 1.47 (s, 9H), 1.12 (d, *J* = 6.6 Hz, 3H).

### Step 2: Synthesis of (R)-3,6-dichloro-4-(2-methylpiperazin-1-yl)pyridazine

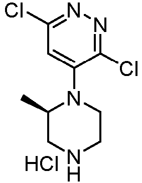

*tert*-Butyl (R)-4-(3,6-dichloropyridazin-4-yl)-3-methylpiperazin-1-carboxylate (3.50 g, 10.1 mmol) and hydrochloric acid/ethyl acetate (8.00 mL, 4M) were added to a reaction flask, the reaction mixture was stirred at room temperature for 5 hours, and the reaction was stopped. the reaction solution was concentrated under reduced pressure to obtain the target compound (2.30g, yield 80.5%, yellow solid). LC- MS (ESI) *m*/*z* 247.0 [M+H]⁺.

### Step 3: Synthesis of (R)-1-(4-(3,6-dichloropyridazin-4-yl)-3-methylpiperazin-1-yl)ethan-1-one

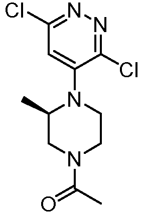

Acetyl chloride (0.955 g, 12.2 mmol) and triethylamine (2.46 g, 24.3 mmol) were added to a solution of (R)-3,6-dichloro-4-(2-methylpiperazin-1-yl)pyridazine hydrochloride (2.30 g, 8.11 mmol) in dichloromethane (20.0 mL), the reaction mixture was stirred at room temperature for 16 hours, and the reaction was stopped. Then water (50.0 mL) was added to dilute the solution, and then extracted with ethyl acetate (45.0 mL×3), the organic phase was washed with saturated saline (30.0 mL), dried over anhydrous sodium sulfate and filtered, and concentrated under reduced pressure. The residue was separated and purified by chromatographic plate (petroleum ether: ethyl acetate=5:1) to obtain the target compound (2.00 g, yield 85.2%, yellow solid). LC- MS (ESI) *m*/*z* 288.9 [M+H]⁺. ¹H NMR (400 MHz, CDCl₃) δ 6.85 (s, 1H), 4.75 - 4.49 (m, 1H), 4.36 - 4.15 (m, 2H), 3.47 - 3.30 (m, 2H), 3.23 - 2.94 (m, 2H), 2.17 - 2.10 (m, 3H), 1.19 - 1.12 (m, 3H).

### Step 4: Synthesis of 1-((3R)-4-(6-(8-oxa-3-azabicyclo[3.2.1]octan-3-yl)-3-chloropyridazin-4-yl)-3-methylpiperazin-1-yl)ethan-1-one

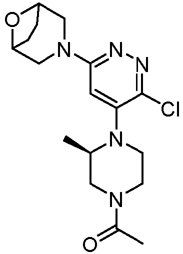

(1R,5S)-8-oxa-3-azabicyclo[3.2.1]octane hydrochloride (1.55 g, 10.4 mmol) and *N,N-*diisopropylethylamine (2.68 g, 20.7 mmol) were added to a solution of (R)-1-(4-(3,6-dichloropyridazin-4-yl)-3-methylpiperazin-1-yl)ethan-1-one (2.00 g, 6.92 mmol) in *N-*methylpyrrolidone (25.0 mL), the reaction mixture was stirred at 145°C for 16 hours, and the reaction was stopped. Then water (50.0 mL) was added to dilute the solution, and then extracted with ethyl acetate (45.0 mL×3), the organic phase was washed with saturated saline (40.0 mL), dried over anhydrous sodium sulfate and filtered, and concentrated under reduced pressure. The residue was separated and purified by column chromatography (PE: EA=1:1) to obtain the target compound (1.80 g, yield 71.1%, yellow solid). LC- MS (ESI) *m*/*z* 366.3 [M+H]⁺. ¹H NMR (400 MHz, CDCl₃) δ 6.15 - 6.08 (m, 1H), 4.54 - 4.44 (m, 3H), 3.97 - 3.82 (m, 2H), 3.78 - 3.64 (m, 2H), 3.58 - 3.47 (m, 2H), 3.25 - 3.18 (m, 2H), 2.20 - 2.13 (m, 2H), 2.13 - 2.10 (m, 2H), 2.02 - 1.97 (m, 2H), 1.89 - 1.81 (m, 3H), 1.06 - 1.01 (m, 3H).

### Step 5: Synthesis of 4-((R)-4-acetyl-2-methylpiperazin-1-yl)-6-(8-oxa-3-azabicyclo[3.2.1]octan-3-yl)pyridazine-3-carbonitrile

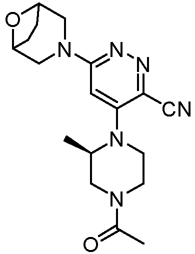

Bis(diphenylphosphino)cyclopentadienyl iron (546 mg, 0.984 mmol) and Pd₂(dba)₃ (450 mg, 0.492 mmol) were added to a solution of 1-((3R)-4-(6-(8-oxa-3-azabicyclo[3.2.1]octan-3-yl)-3-chloropyridazin-4-yl)-3-methylpiperazin-1-yl)ethan-1-one (1.8 g, 4.92 mmol) and zinc cyanide (1.16 g, 9.84 mmol) in DMF (30.0 mL). The reaction mixture was stirred at 135°C under nitrogen protection for 16 hours, and the reaction was stopped. Then the reaction solution was diluted with EA (40.0 mL), and washed with saturated saline (35 mL×3), dried over anhydrous sodium sulfate and filtered, and concentrated under reduced pressure. The residue was separated and purified by column chromatography (DCM: methanol=10:1) to obtain the target compound (1.05g, yield 59.9%, yellow solid). LC-MS (ESI) *m*/*z* 357.0 [M+H]⁺. ¹H NMR (400 MHz, CDCl₃) δ 5.95 - 5.84 (m, 1H), 4.74 - 4.61 (m, 1H), 4.53 (s, 2H), 4.30 - 4.18 (m, 1H), 4.07 - 3.83 (m, 2H), 3.66 (s, 1H), 3.54 - 3.41 (m, 1H), 3.36 - 3.17 (m, 4H), 3.03 - 2.89 (m, 1H), 2.18 - 2.10 (m, 3H), 2.03 - 1.98 (m, 2H), 1.87 - 1.79 (m, 2H), 1.19 - 1.12 (m, 3H).

### Step 6: Synthesis of 1-((3R)-4-(3-(aminomethyl)-6-(8-oxa-3-azabicyclo[3.2.1]octan-3-yl)pyridazin-4-yl)-3-methylpiperazin-1-yl)ethan-1-one

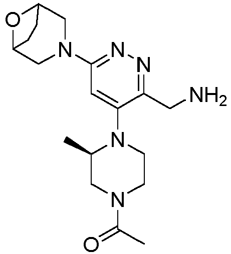

Raney Ni (543 mg, 9.26 mmol) and ammonia (1.00 mL) were added to solution of 4-((R)-4-acetyl-2-methylpiperazin-1-yl)-6-(8-oxa-3-azabicyclo[3 .2.1]octan-3-yl)pyridazine-3-carbonitrile (1.10 g, 3.09 mmol) in tetrahydrofuran (15.0 mL). The reaction mixture was reacted at room temperature for 5 hours under hydrogen atmosphere, and the reaction was stopped. Then the reaction solution was filtered and concentrated under reduced pressure, and the resulting residue was the target compound (660 mg, yield 59.3%, yellow oil). LC-MS (ESI) *m*/*z* 361.0 [M+H]⁺.

### Step 7: Synthesis of N-((4-((R)-4-acetyl-2-methylpiperazin-1-yl)-6-(8-oxa-3-azabicyclo[3.2.1]octan-3-yl)pyridazin-3-yl)methyl)-1H-pyrazole-5-carboxamide

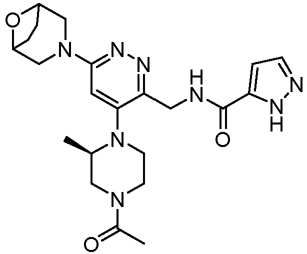

(0.456 mg, 1.20 mmol) and DIEA (387 mg, 3.00 mmol) were added to a solution of 1-((3R)-4-(3-(aminomethyl)-6-(8-oxa-3-azabicyclo[3.2.1]octan-3-yl)pyridazin-4-yl)-3-methylpiperazin-1-yl)ethan-1-one (360 mg, 0.999 mmol) and 1*H*-pyrazol-5-carboxylic acid (101 mg, 0.899 mmol) in the tetrahydrofuran (10.0 mL), the reaction mixture was stirred at room temperature for 1 hour, the reaction was stopped. Then water (30.0 mL) was added , extracted with EA (25.0 mL×3), the organic phases were combined and washed with saturated saline (20.0 mL), dried over anhydrous sodium sulfate and filtered, and concentrated under reduced pressure. The residue was separated and purified by column chromatography (DCM: methanol=10:1) to obtain the target compound (170 mg, yield 37.4%, yellow solid). LC- MS (ESI) *m*/*z* 455.0 [M+H]⁺.

### Step 8: Synthesis of 1-((R)-4-(2-((1R,5S)-8-oxa-3-azabicyclo[3.2.1]octan-3-yl)-7-(1H-pyrazol-3-yl)imidazo[1,5-b]pyridazin-4-yl)-3-methylpiperazin-1-yl)ethan-1-one

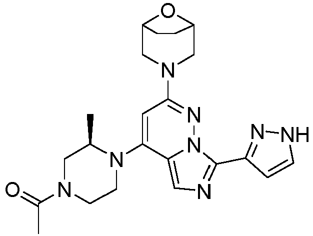

At room temperature, *N-*((4-((*R*)-4-acetyl-2-methylpiperazin-1-yl)-6-(8-oxa-3-azabicyclo[3.2.1]octan-3-yl)pyridazin-3-yl)methyl)-1*H*-pyrazole-5-carboxamide (170 mg, 0.374 mmol) and phosphorus oxychloride (6.00 mL) were added to a reaction flask. The reaction mixture was reacted at 100°C for 1 hour and the reaction was stopped. The reaction solution was concentrated under reduced pressure, and the residue was purified by reverse phase preparative chromatography (acetonitrile/water with 0.05% formic acid) to obtain the target compound (6.68 mg, yield 4.09%, white solid). LC- MS (ESI) *m*/*z* 437.3 [M+H]⁺. ¹H NMR (400 MHz, MeOD-d4) δ 7.83 - 7.51 (m, 2H), 7.13 (s, 1H), 5.80 (s, 1H), 4.55 - 4.48 (m, 3H), 3.87 - 3.80 (m, 2H), 3.74 - 3.42 (m, 4H), 3.30 - 3.05 (m, 4H), 2.24-2.12 (m, 3H), 2.04 - 1.93 (m, 4H), 1.26-1.13 (m, 3H).

### Example 30: 3-(4-(4,4-difluoro-2-methylpiperidin-1-yl)-7-(1H-pyrazol-3-yl)imidazo[1,5-b]pyridazin-2-yl)-8-oxa-3-azabicyclo[3.2.1]octane

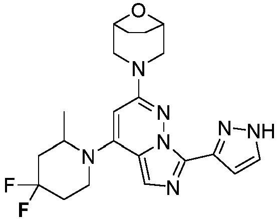

### Step 1: Synthesis of 1-(3,6-dichloropyridazin-4-yl)-2-methylpiperidin-4-one

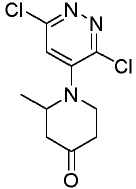

DIEA (6.09 g, 60.2 mmol) was added to a solution of 2-methylpiperidine-4-one hydrochloride (3.00 g, 20.1 mmol) and 3,4,6-trichloropyridazine (4.41 g, 24.1 mmol) in *N-*methylpyrrolidone (50.0 mL). The reaction mixture was stirred at 60°C under nitrogen protection for 16 hours, and the reaction was stopped. Then the reaction solution was diluted with ethyl acetate (100 mL), washed with saturated saline (60.0 mL×3), dried over anhydrous sodium sulfate and filtered, and concentrated under reduced pressure. The residue was separated and purified by column chromatography (PE: EA=5:1) to obtain the target compound (3.00 g, yield 57.5%, yellow solid). LC-MS (ESI) m/z 260.0 [M+H]⁺.

### Step 2: Synthesis of 3,6-dichloro-4-(4,4-difluoro-2-methylpiperidin-1-yl)pyridazine

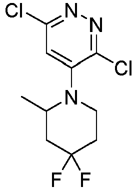

Diethylamino sulfur trifluoride (2.60 g, 16.2 mmol) was added to a solution of 1-(3,6-dichloropyridazin-4-yl)-2-methylpiperidin-4-one (2.8 g, 10.7 mmol) in dichloromethane (50.0 mL) under nitrogen protection at 0°C. The reaction mixture was stirred at 0°C for 1 hour under nitrogen protectionand then the reaction was stopped. Then the reaction solution was concentrated under reduced pressure, and the resulting residue was the target compound (1.80 g, yield 59.3%, yellow oil). LC-MS (ESI) *m*/*z* 281.9 [M+H]⁺.

### Step 3: Synthesis of 3-(6-chloro-5-(4,4-difluoro-2-methylpiperidin-1-yl)pyridazin-3-yl)-8-oxa-3-azabicyclo[3.2.1]octane

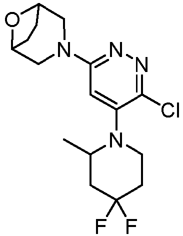

DIEA (2.47 g, 19.1 mmol) was added to a solution of 3,6-dichloro-4-(4,4-difluoro-2-methylpiperidin-1-yl)pyridazine (1.80 g, 6.38 mmol) and 8-oxa-3-azabicyclo[3.2.1]octane hydrochloride (1.43 g, 9.57 mmol) in *N*-methylpyrrolidone (50.0 mL). The reaction mixture was stirred at 145°C for 16 hours under nitrogen protection, and the reaction was stopped. Then the reaction solution was diluted with ethyl acetate (85.0 mL), and washed with saturated saline (55.0 mL×3), dried over anhydrous sodium sulfate and filtered, and concentrated under reduced pressure. The residue obtained was separated and purified by column chromatography (PE: EA=1:1) to obtain the target compound (1.80 g, yield 78.6%, yellow solid). LC-MS (ESI) *m*/*z* 359.0 [M+H]⁺. ¹H NMR (400 MHz, CDCl₃) δ 6.23 (s, 1H), 4.54 - 4.50 (m, 2H), 4.09 - 3.96 (m, 1H), 3.86 - 3.74 (m, 2H), 3.47 - 3.37 (m, 1H), 3.26 - 3.19 (m, 2H), 3.13 - 2.99 (m, 1H), 2.32 - 2.23 (m, 1H), 2.23 - 2.08 (m, 2H), 2.04 - 1.97 (m, 3H), 1.90 - 1.84 (m, 2H), 1.14 (d, *J* = 16.6, 6.7 Hz, 3H).

### Step 4: Synthesis of 6-(8-oxa-3-azabicyclo[3.2.1]octan-3-yl)-4-(4,4-difluoro-2-methylpiperidin-1-yl)pyridazine-3-carbonitrile

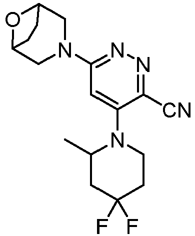

Bis(diphenylphosphino)cyclopentadienyl iron (556 mg, 1.00 mmol) and Pd₂(dba)₃ (459 mg, 0.501 mmol) were added to a solution of 3-(6-chloro-5-(4,4-difluoro-2-methylpiperidin-1-yl)pyridazin-3-yl)-8-oxa-3-azabicyclo[3.2.1]octane (1.8 g, 5.02 mmol) and zinc cyanide (1.18 g, 10.0 mmol) in *N*,*N*-dimethylformamide (30.0 mL), the reaction mixture was stirred at 140°C for 16 hours under nitrogen protection, and the reaction was stopped. Then the reaction solution was diluted with ethyl acetate (65.0 mL), and washed with saturated saline (40.0 mL×3), dried over anhydrous sodium sulfate and filtered, and concentrated under reduced pressure. The residue was separated and purified by column chromatography (PE: EA=1:1) to obtain the target compound (1.50g, yield 85.6%, yellow solid). LC-MS (ESI) *m*/*z* 350.1 [M+H]⁺. ¹H NMR (400 MHz, CDCl₃) δ 5.93 (s, 1H), 4.53 - 4.50 (m, 2H), 4.42 - 4.33 (m, 1H), 4.01 - 3.90 (m, 2H), 3.51 - 3.42 (m, 2H), 3.31 - 3.26 (m, 2H), 2.29 - 2.15 (m, 2H), 2.11 - 1.99 (m, 4H), 1.84 - 1.80 (m, 2H), 1.24 (dd, J= 6.9, 1.7 Hz, 3H).

### Step 5: Synthesis of (6-(8-oxa-3-azabicyclo[3.2.1]octan-3-yl)-4-(4,4-difluoro-2-methylpiperidin-1-yl)pyridazin-3-yl)methanamine

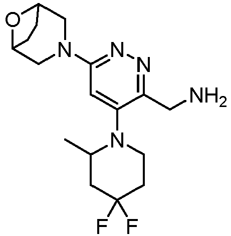

Raney nickel (1.26 g, 21.5 mmol) was added to a solution of 6-(8-oxa-3-azabicyclo[3.2.1]octan-3-yl)-4-(4,4-difluoro-2-methylpiperidin-1-yl)pyridazine-3-carbonitrile (1.50 g, 4.29 mmol) in tetrahydrofuran (30.0 mL). The reaction mixture was reacted at room temperature for 16 hours under hydrogen atmosphere, and the reaction was stopped. Then the reaction solution was filtered and concentrated under reduced pressure. The residue obtained was the target compound (1.20g, yield 79.1%, yellow oil). LC-MS (ESI) *m*/*z* 354.0 [M+H]⁺.

### Step 6: Synthesis of N-((6-(8-oxa-3-azabicyclo[3.2.1]octan-3-yl)-4-(4,4-difluoro-2-methylpiperidin-1-yl)pyridazin-3-yl)methyl)-1H-pyrazole-5-carboxamide

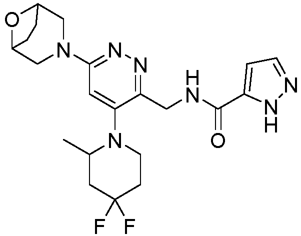

(1.55 g, 4.07 mmol) and DIEA (1.32 g, 10.2 mmol) were added to a solution of (6-(8-oxa-3-azabicyclo[3.2.1]octan-3-yl)-4-(4,4-difluoro-2-methylpiperidin-1-yl)pyridazin-3-yl)methanamine (1.20 g, 3.40 mmol) and 1*H*-pyrazole-5-carboxylic acid (342 mg, 3.06 mmol) in THF (30.0 mL). The reaction mixture was reacted at room temperature for 1 hour and the reaction was stopped. Then the reaction solution was filtered and concentrated under reduced pressure, and the residue was separated and purified by column chromatography (EA) to obtain the target compound (1.00 g, yield 65.8%, yellow solid). LC-MS (ESI) *m*/*z* 448.0 [M+H]⁺. ¹H NMR (400 MHz, CDCl₃) δ 8.76 (s, 1H), 7.59 (d, *J=* 2.1 Hz, 1H), 6.80 (d, *J=* 2.2 Hz, 1H), 6.44 (s, 1H), 4.87 - 4.72 (m, 2H), 4.55 - 4.50 (m, 2H), 3.86 - 3.77 (m, 2H), 3.49 (s, 2H), 3.38 - 3.31 (m, 1H), 3.26 - 3.21 (m, 2H), 2.35 - 2.22 (m, 2H), 2.03 - 1.99 (m, 2H), 1.90 - 1.84 (m, 2H), 1.50 - 1.47 (m, 2H), 1.02 (d, *J=* 6.2 Hz, 3H).

### Step 7: Synthesis of 3-(4-(4,4-difluoro-2-methylpiperidin-1-yl)-7-(1H-pyrazol-3-yl)imidazo[1,5-b]pyridazin-2-yl)-8-oxa-3-azabicyclo[3.2.1]octane

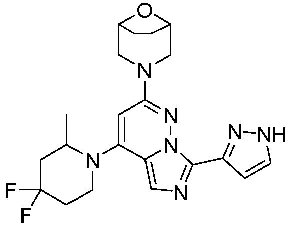

*N*-((6-(8-oxa-3-azabicyclo[3.2.1]octan-3-yl)-4-(4,4-difluoro-2-methylpiperidin-1-yl)pyridazin-3-yl)methyl)-1*H*-pyrazole-5-carboxamid (200 mg, 0.447 mmol), phosphorus oxychloride (685 mg, 4.47 mmol) and acetonitrile (5 mL) were added to a reaction flask at room temperature. The reaction mixture was reacted at 100°C for 1 hour and the reaction was stopped. The reaction solution was concentrated under reduced pressure, and the obtained residue was purified by reverse phase preparative chromatography (acetonitrile/water with 0.05% formic acid) to obtain the target compound (110 mg, yield 57.3%, white solid). LC- MS (ESI) *m*/*z* 430.2 [M+H]⁺. ¹H NMR (400 MHz, DMSO-*d*₆) δ 7.81 - 7.59 (m, 2H), 7.10 (s, 1H), 5.92 (s, 1H), 4.71 - 4.59 (m, 1H), 4.47 (s, 2H), 3.88 - 3.79 (m, 3H), 3.47 - 3.40 (m, 1H), 3.07 (d, *J* = 11.1 Hz, 2H), 2.44 - 2.28 (m, 1H), 2.22 - 2.04 (m, 3H), 1.89-1.81 (m, 4H), 1.21 (d, *J=* 5.6 Hz, 3H).

### Example 31 and 32: 3-(4-((R)-4,4-difluoro-2-methylpiperidin-1-yl)-7-(1H-pyrazol-3-yl)imidazo[1,5-b]pyridazin-2-yl)-8-oxa-3-azabicyclo[3.2.1]octane, and 3-(4-((S)-4,4-difluoro-2-methylpiperidin-1-yl)-7-(1H-pyrazol-3-yl)imidazo[1,5-b]pyridazin-2-yl)-8-oxa-3-azabicyclo[3.2.1]octane

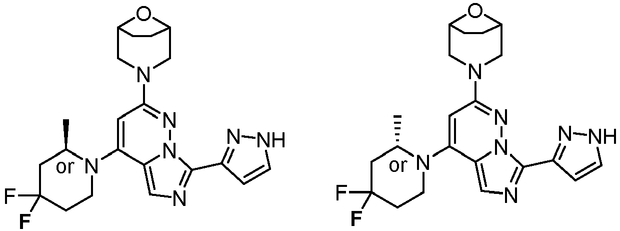

50 mg of 3-(4-(4,4-difluoro-2-methylpiperidin-1-yl)-7-(1*H*-pyrazol-3-yl)imidazo[1,5-b]pyridazin-2-yl)-8-oxa-3-azabicyclo[3.2.1]octane racemate was subjected to chiral resolution (Waters SFC 150 (room temperature, 100 bar, 214 nm) and 250 * 25 mm 10 mm DAICELCHIRALPAKIG (supercritical carbon dioxide: MEOH (+0.1% 7.0mol/l Ammonia in MEOH), 45:55, 3.2 min, 100 mL/min)), to obtain P1 (14.40 mg, e.e. 100.00%) eluted first (with a small retention time) and P2 (14.37 mg, e.e. 99.07%) eluted later (with a large retention time). LC-MS (ESI) *m*/*z* 430.2 [M+H]⁺.
LC- MS (ESI) m/z 430.2 [M+H]+. (P1): 1H NMR (400 MHz, MeOD-d4) δ 7.71 (s, 1H), 7.55 (s, 1H), 7.13 (s, 1H), 5.88 (s, 1H), 4.71 - 4.63 (m, 1H), 4.50 (s, 2H), 3.87 - 3.76 (m, 3H), 3.58 - 3.50 (m, 1H), 3.24 - 3.16 (m, 2H), 2.44 - 2.28 (m, 1H), 2.24 - 2.08 (m, 3H), 2.01 - 1.94 (m, 4H), 1.30 (d, J = 6.9 Hz, 3H);
(P2): ¹H NMR (400 MHz, MeOD-*d*₄) δ 7.70 (s, 1H), 7.54 (s, 1H), 7.13 (s, 1H), 5.88 (s, 1H), 4.71 - 4.63 (m, 1H), 4.50 (s, 2H), 3.86 - 3.76 (m, 3H), 3.58 - 3.46 (m, 1H), 3.22 - 3.17 (m, 2H), 2.44 - 2.27 (m, 1H), 2.25 - 2.08 (m, 3H), 2.02 - 1.94 (m, 4H), 1.30 (d, *J* = 7.3 Hz, 3H).

### Example 34: 1-((R)-4-(6-((1R,5S)-8-oxa-3-azabicyclo[3.2.1]octan-3-yl)-1-(1H-pyrazol-3-yl)-1H-pyrazolo[3,4-b]pyridin-4-yl)-3-methylpiperazin-1-yl)ethan-1-one

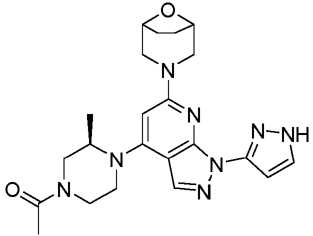

### Step 1: Synthesis of 1-((3R)-4-(6-((1R,5S)-8-oxa-3-azabicyclo[3.2.1]octan-3-yl)-1-(1-(tetrahydro-2H-pyran-2-yl)-1H-pyrazol-3-yl)-1H-pyrazolo[3,4-b]pyridin-4-yl)-3-methylpiperazin-1-yl)ethan-1-one

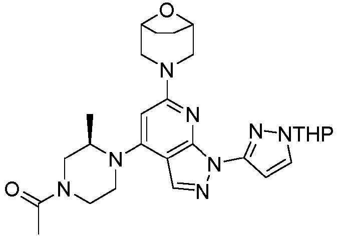

(1*R*,5*S*)-3-(4-iodo-1-(1-(tetrahydro-2*H*-pyran-2-yl-1*H*-pyrazol-3-yl)-1*H*-pyrazolo[3,4-b]pyridine-6-yl)-8-oxa-3-azabicyclo[3.2.1]octane (1g, 1.97 mmol), (*R*)-1-(3-methylpiperazin-1-yl) ethane-1-one (561 mg, 3.95 mmol), RuphosG2 (155 mg, 0.2 mmol), and cesium carbonate (1.93g, 5.91 mmol) were added to NMP (10 mL) in turn. The reaction solution was reacted for 1 hour at 150°C under microwave, and after the reaction was completed and is cooled, water (20 mL) was added, extracted with EA (20 mL×3), the organic phases were combined and washed with water and saturated saline, dried over anhydrous sodium sulfate and filtered, and the filtrate was concentrated under reduced pressure to obtain the crude product. The crude product was separated and purified by reverse phase column (MeOH: H₂O=5%:95% - 95%:5%) to obtain the target compound (60 mg, yield 6.0%). LC-MS (ESI) *m*/*z* 521.2 [M+H]⁺.

### Step 2: Synthesis of 1-((R)-4-(6-((1R,5S)-8-oxa-3-azabicyclo[3.2.1]octan-3-yl)-1-(1H-pyrazol-3-yl)-1H-pyrazolo[3,4-b]pyridin-4-yl)-3-methylpiperazin-1-yl)ethan-1-one

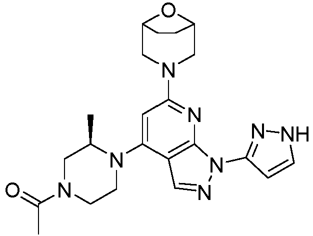

1-((3*R*)-4-(6-((1*R*,5*S*)-8-oxa-3-azabicyclo[3.2.1]octan-3-yl)-1-(1-(tetrahydro-2*H*-pyran-2-yl)-1*H*-pyrazol-3-yl)-1*H*-pyrazolo[3,4-b]pyridin-4-yl)-3-methylpiperazin-1-yl)ethan-1-one (60 mg, 0.12 mmol) was added to a solution of 6M hydrochloric acid in water/tetrahydrofuran (1 mL/2 mL), and stirred overnight at room temperature. After the reaction was completed, the solution was adjusted to pH>8 with saturated sodium bicarbonate aqueous solution, extracted with EA (20 mL×3), the organic layers were combined, and concentrated under reduced pressure to obtain the crude product. The crude product was washed with EA (2 mL), and the resulting solid was the target product (7 mg, yield 14.0%, green solid). LC-MS (ESI) *m*/*z* 437.3 [M+H]⁺. ¹H NMR (400 MHz, CDCl₃) δ 7.97 (s, 1H), 7.66 (s, 1H), 6.82 (s, 1H), 5.63 (s, 1H), 4.62 - 4.49 (m, 2H), 4.46 - 4.33 (m, 1H), 4.01 - 3.81 (m, 2H), 3.74 (s, 2H), 3.66 - 3.45 (m, 2H), 3.46 - 3.34 (m, 1H), 3.34 - 3.18 (m, 2H), 3.18 - 3.03 (m, 1H), 2.24 - 2.10 (m, 3H), 2.05 - 1.95 (m, 2H), 1.92 - 1.83 (m, 2H), 1.25 - 1.17 (m, 3H).

### Example 35: (4-(6-((1R,5S)-8-oxa-3-azabicyclo[3.2.1]octan-3-yl)-1-(1H-pyrazol-3-yl)-1H-pyrazolo[3,4-b]pyridin-4-yl)morpholin-3-yl)methanol

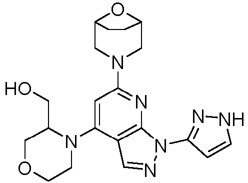

### Step 1: Synthesis of (4-(6-((1R,5S)-8-oxa-3-azabicyclo[3.2.1]octan-3-yl)-1-(1-(tetrahydro-2H-pyran-2-yl)-1H-pyrazol-3-yl)-1H-pyrazolo[3,4-b]pyridin-4-yl)morpholin-3-yl)methanol

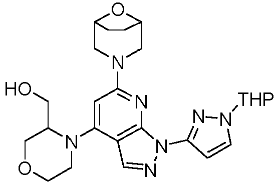

(1*R*,5*S*)-3-(4-iodo-1-(1-(tetrahydro-2*H*-pyran-2-yl)-1*H*-pyrazol-3-yl)-1*H*-pyrazol[3,4-b] pyridine-6-yl)-8-oxa-3-azabicyclo[3.2.1]octane (300 mg, 0.6 mmol), morpholin-3-yl methanol (144 mg, 1.2 mmol), RuphosPdG2 (48 mg, 0.06 mmol), and cesium carbonate (582 mg, 1.8 mmol) were successively added to NMP (12 mL), and the mixture was stirred under microwave at 150°C for 1 hour. After the reaction was completed, the reaction was extracted with EA (20 mL×3), the organic layers were combined, washed with water (20 mL×5) and saturated saline (30 mL), dried over anhydrous sodium sulfate and filtered, and the filtrate was concentrated under reduced pressure to obtain the crude product. The crude product was separated and purified by silica gel column (DCM: MeOH=30:1) to obtain the target compound (29 mg, yield 9.7%, white solid). LC-MS (ESI) m/z: 496.3 [M+H]⁺.

### Step 2: Synthesis of (4-(6-((1R,5S)-8-oxa-3-azabicycli[3.2.1]octan-3-yl)-1-(1H-pyrazol-3-yl)-1H-pyrazolo[3,4-b]pyridin-4-yl)morpholin-3-yl)methanol

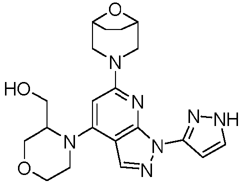

(4-(6-((1*R*,5*S*)-8-oxa-3-azabicyclo[3.2.1]octan-3-yl)-1-(1-(tetrahydro-2*H*-pyran-2-yl)-1*H-*pyrazol-3-yl)-1*H*-pyrazolo[3,4-b]pyridin-4-yl)morpholin-3-yl)methanol (29 mg, 0.06 mmol) was added to a solution of 6M hydrochloric acid in water/tetrahydrofuran (2 mL/4 mL), the mixture was stirred at 50°C overnight. After reaction was completed, the solution was adjusted to pH>8 with saturated sodium bicarbonate aqueous solution, extracted with EA (20 mL×3), the organic layers were combined, washed with saturated saline (30 mL), dried over anhydrous sodium sulfate and filtered. The filtrate was concentrated under reduced pressure to obtain crude product. The target product (10 mg, yield 40.5%, white solid) was obtained by silica gel column separation and purification (DCM: MeOH=15:1). LC-MS (ESI) *m*/*z:* 412.2. ¹H NMR (400 MHz, CDCl₃) 6 8.02 (s, 1H), 7.61 (d, *J=* 1.5 Hz, 1H), 6.80 (s, 1H), 5.69 (s, 1H), 4.57 - 4.47 (m, 2H), 4.21 (d, *J* = 11.7 Hz, 1H), 4.17 - 4.09 (m, 1H), 4.10 - 3.98 (m, 2H), 3.91 - 3.79 (m, 4H), 3.80 - 3.69 (m, 1H), 3.53 (d, *J=* 5.8 Hz, 2H), 3.28 - 3.18 (m, 2H), 2.06 - 1.91 (m, 3H), 1.91 - 1.79 (m, 2H).

### Example 36: 3-(7-(3,3-dimethylmorpholino)-3-(1H-pyrazol-5-yl)pyrazolo[1,5-a]pyrimidin-5-yl)-8-oxa-3-azabicyclo[3.2.1]octane

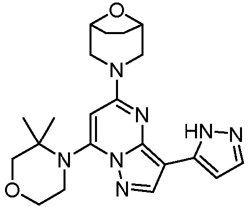

### Step 1: Synthesis of 4-(5-chloropyrazolo[1,5-a]pyrimidin-7-yl)-3,3-dimethylmorpholine

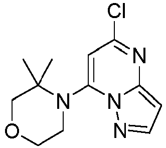

5,7-Dichloropyrazolo[1,5-a]pyrimidine (540 mg, 2.9 mmol), 3,3-dimethylmorpholine hydrochloride (1.0 g, 8.6 mmol) and DIEA (1.1 g, 8.6 mmol) were added to NMP (15 mL). The obtained mixture was reacted for 0.5 hour at 100°C in microwave under nitrogen protection. After the reaction was completed, water (15 mL) was added to the reaction mixture, extracted with EA (20 mL×3). The organic phase was combined and washed with water (30 mL×5) and saturated saline (30 mL×2), dried over anhydrous sodium sulfate and filtered. The filtrate was concentrated under reduced pressure to obtain crude product, which was separated and purified by silica gel column (PE: EA=7:3) to obtain the target compound (0.9 g, yield 116.3%, white solid). LC-MS (ESI) *m*/*z:* 267.0 [M+H]⁺.

### Step 2: Synthesis of 3-(7-(3,3-dimethylmorpholino)pyrazolo[1,5-a]pyrimidin-5-yl)-8-oxa-3-azabicyclo[3.2.1]octane

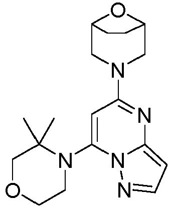

4-(5-Chloropyrazolo[1,5-a]pyrimidin-7-yl)-3,3-dimethylmorpholine (200 mg, 0.8 mmol), (1*R*,5*S*)-8-oxa-3-azabicyclo[3.2.1]octane hydrochloride (225 mg, 1.5 mmol) and DIEA (290 mg, 2.3 mmol) were successively added to NMP (10 mL) in turn. The reaction mixture was reacted for 2 hours at 140°C in microwave. After the reaction was completed, water (15 mL) was added to the reaction mixture, extracted with EA (20 mL×3), the organic phases were combined, washed with water (30 mL×5) and saturated saline (30 mL×2), dried over anhydrous sodium sulfate and filtered, and the filtrate was concentrated under reduced pressure to obtain crude product. The target product (202 mg, yield 78.6%, white solid) was obtained by silica gel column separation and purification (PE: EA=57%: 43%).

### Step 3: Synthesis of 3-(7-(3,3-dimethylmorpholino)-3-iodopyrazolo[1,5-a]pyrimidin-5-yl)-8-oxa-3-azabicyclo[3.2.1]octane

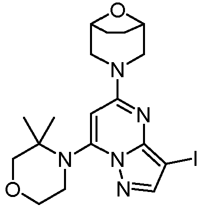

3-(7-(3,3-Dimethylmorpholino)pyrazolo[1,5-a]pyrimidin-5-yl)-8-oxa-3-azabicyclo[3.2.1]octane (202 mg, 0.6 mmol) and N-iodosuccinimide (135 mg, 0.6 mmol) were added to acetonitrile (5 mL), and stirred at room temperature for 0.5 h. After the reaction was completed, concentration under reduced pressure provided the crude product, which was separated and purified by silica gel column (PE: EA=1: 1) to obtain the target compound (224 mg, yield 79.7%, white solid).

### Step 4: Synthesis of 3-(7-(3,3-dimethylmorpholino)-3-(1-(tetrahydro-2H-pyran-2-yl)-1H-pyrazol-5-yl)pyrazolo[1,5-a]pyrimidin-5-yl)-8-oxa-3-azabicyclo[3.2.1]octane

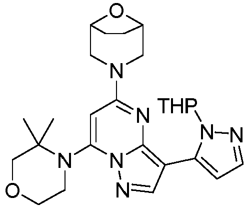

3 -(7-(3,3 -Dimethylmorpholino)-3 -iodopyrazolo[1, 5-a]pyrimidin-5-yl)-8-oxa-3 - azabicyclo[3.2.1]octane (172 mg, 0.4 mmol), 1-(tetrahydro-2H-pyran-2-yl)-5-(4,4,5,5-tetramethyl-1,3,2-dioxobenzofuran-2-yl)-1*H*-pyrazole (153 mg, 0.6 mmol), potassium phosphate (233 mg, 1.1 mmol) and [1,1 '- bis (diphenylphosphino)ferrocene] palladium dichloride (24 mg, 0.04 mmol) were successively added to dioxane/water (10 mL/2 mL). The reaction mixture was reacted for 1 hour at 80°C in an oil bath. After the reaction was completed, water (15 mL) was added in the reaction mixture, extracted with EA (20 mL×3), the organic phases were combined, washed with water (30 mL×5) and saturated saline (30 mL×2), dried over anhydrous sodium sulfate and filtered. The filtrate was concentrated under reduced pressure to obtain the crude product, which was separated and purified by silica gel column (PE: EA=1:4) to obtain the target compound (68 mg, yield 37.2%, white solid). LC-MS (ESI) *m*/*z:* 494.0 [M+H]⁺.

### Step 5: Synthesis of 3-(7-(3,3-dimethylmorpholino)-3-(1H-pyrazol-5-yl)pyrazolo[1,5-a]pyrimidin-5-yl)-8-oxa-3-azabicyclo[3.2.1]octane

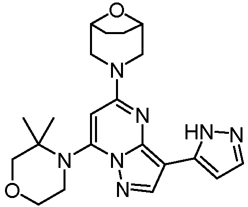

3 -(7-(3,3 -Dimethylmorpholino)-3 -(1 -(tetrahydro-2*H*-pyran-2-yl)-1*H*-pyrazol-5-yl)pyrazolo[1,5-a]pyrimidin-5-yl)-8-oxa-3-azabicyclo[3.2.1]octane (68 mg, 0.1 mmol) was added to a solution of 6M hydrochloric acid in water/tetrahydrofuran (2 mL/4 mL), stirred at 50°C for 1 hour. After the reaction was completed, the solution was adjusted to pH>8 with saturated sodium bicarbonate aqueous solution, extracted with EA (20 mL×3), the organic layers were combined, washed with saturated saline (30 mL), dried over anhydrous sodium sulfate and filtered, and the filtrate was concentrated under reduced pressure to obtain the crude product. The crude product was separated and purified by silica gel column (DCM/MeOH=30/1) to obtain the target compound (38 mg, yield 67.9%, white solid). LC-MS (ESI) *m*/*z:* 410.2 [M+H]⁺. ¹H NMR (400 MHz, DMSO-*d*₆) *δ* 8.21 (s, 1H), 7.57 (s, 1H), 6.70 (s, 1H), 5.97 (s, 1H), 4.54 - 4.40 (m, 2H), 4.05 (d, *J=* 11.3 Hz, 2H), 3.88 - 3.75 (m, 2H), 3.59 - 3.50 (m, 2H), 3.45 (s, 2H), 3.12 (d, *J=* 10.4 Hz, 2H), 1.90 - 1.69 (m, 4H), 1.32 (s, 6H).

### Activity Examples

Unless otherwise specified, the experimental materials, reagents, operations and methods used in the following activity examples can be obtained from commercial channels or easily known or prepared based on the piror art.

### Activity Example 1: ATR Kinase inhibition assay

This experiment adopted Homogeneous time-resolved fluorescence technology (HTRF^{®}) of Cisbio Company for assay.

Experiment method: buffers were prepared as follows:

| Name | Resource | Final concentration | Concentration of stock solution | Volume (uL) |
|---|---|---|---|---|
| HEPES (mM) | Gibco, Cat # 15630-080 | 25 | 1000 | 75 |
| Brij35 | Sigma, Cat # 9002-92-0 | 0.01% | 1% | 30 |
| BSA (mg/mL) | Sigma, Cat # B2064-50G | 1 | 100 | 30 |
| DTT (mM) | Sigma, Cat # D0632-10G | 5 | 1000 | 15 |
| Glycerol | Sigma, Cat # G5516-500ML | 1% | 50% | 60 |
| MnCl₂ (mM) | Sigma, Cat # 7773-01-5 | 10 | 1000 | 30 |

| | | | | |
|---|---|---|---|---|
| H₂O | | | | 2760 |
| Total (uL) | | | | 3000 |

Preparation of solutions of the test compounds: The example compounds shown in the table below were prepared into solutions using DMSO as solvent, the stock solution typically being 10 mM. The maximum starting concentration in this experiment was 3 µ M, DMSO 3-fold serial dilutions for 10 concentrations, the serially diluted solution was added to each well of the corresponding 384-well plate (geriner bio-one, Cat # 784075) to give final concentrations of 3000, 1000, 333, 111, 37, 12.3, 4.12, 1.37, 0.457, 0.152 nM, respectively, and the corresponding amount of DMSO was added to the additional wells to be used as negative or positive control wells, respectively.

The enzyme and substrate were separately dissolved and diluted in two tubes with the buffer prepared above at the following concentrations:

| **Name** | Source | Final concentration after adding in the plate | Concentration of prepared solution | Concentration of stock solution |
|---|---|---|---|---|
| ATR (A tube) | eurofins, Cat #14-953M | 30 nM | 60 nM | 410.26 nM |
| P53 (B tube) | eurofins, Cat #14-953M | 40 nM | 80 nM | 9720 nM |
| ATP (B tube) | Sigma, Cat # R0441 | 150 nM | 300 nM | 10000 nM |

5 µL of the ATR protein solution from tube A was added to the compound-containing wells and the negative control wells in the assay plates using an electric multichannel pipettor, and the same volume of the prepared buffer (medium only, no enzyme) was added to the positive control wells. The plates were centrifuged for one minute at 1000 rpm, and then placed in an incubator at 25°C for 15 minutes. 5 µL of the substrate mix solution from tube B was added to the positive control well, negative control well and the compound-containing well of the assay plates in the same manner, centrifuged and incubated at 25°C for 90 minutes.

During the reaction, after ATR phosphorylated the substrate p53, two antibodies were added. Among them, anti-phospho-p53-Eu, as an energy donor, can specifically bind to the phosphorylation site of p53, while anti-GST-d2, as an energy receptor, can specifically bind to the GST-tagged p53. Upon excitation by a laser with certain wavelength (the excitation wavelength in this experiment is 340nm), the energy donor can emit emission light of 615 nm, and when the energy donor and the energy receptor are close enough in space (that is, two antibodies are connected to p53 at the same time), energy transfer can occur between the energy donor and the energy receptor, so that the energy receptor can emit emission light of 665nm. The two emission lights were detected by a plate reader, and the ratio of the signals at 665 nm and 615 nm was calculated. The IC₅₀ of the samples tested can be obtained by plotting and calculation.

For detection, two antibodies were diluted in HTRF detection buffer (Cisbio, Cat #62SDBRDF, Lot #17A) as shown in the table below:

| **Test kit** | Source | Dilution factor | Concentration of stock solution |
|---|---|---|---|
| anti-phospho-p53-Eu | Cisbio, Cat # 61P08KAZ | 400 | 400 unit |
| anti-GST-d2 | Cisbio, Cat # 61GSTDLB | 200 | 200 unit |

10 µL per well of the detection solution was added to the positive control, negative control and compound wells of the assay plate using an electric multichannel pipette, centrifuged and incubated overnight at 4°C, and read with the plate reader Envision 2104.

Compound inhibition rate was calculated using interpolation. The mean values of the positive and negative controls were calculated respectively, and the following formula was used to calculate the inhibition rates of the compounds: Single well inhibition rate - 1 - (Value of single well signal - Mean of positive control signal) / (Mean of negative control signal - Mean of positive control signal) Compound inhibition curves were plotted using a four parameter Logistic equation curve: compound concentrations were converted to Base-10 logarithms and the concentrations and inhibition rates were introduced into XLfit software, the formula is shown below: Inhibition rate = Minimum response + (Compound concentration̂ Cure slope) * (Maximum response * Minimum response) / (Compound concentration̂ Curve slope + Half inhibition concentration̂ Curve slope) The IC50 value of each compound to enzyme activity was obtained.

**Table 1 ATR Kinase Inhibition Activity**

| **Compound No** | ATR **IC₅₀ (nM)** | **Compound No** | ATR **IC₅₀ (nM)** |
|---|---|---|---|
| 1 | 17.4 | 16 | 21.9 |
| 2 | 40.3 | 17 | 32.0 |
| 3 | 5.9 | 18 | 50.8 |
| 4 | 20.3 | 26 | 57.7 |
| 5 | 8.2 | 27 | 149.0 |
| 6 | 13.2 | 20 | 1.4 |
| 7 | 21.4 | 23 | 85.5 |
| 8 | 18.4 | 29 | 4.5 |
| 9 | 79.2 | 30 | 5.2 |
| 10 | 144.7 | 31 | 3.9 |
| 11 | 22.4 | 32 | 5.6 |
| 12 | 16.9 | 34 | 7.6 |
| 13 | 18.0 | 35 | 15.3 |
| 14 | 8.0 | 36 | 6.2 |
| 15 | 22.0 | | |

### Activity Example 2: Cell proliferation assay

The cell proliferation inhibition effect of the compound of the invention was tested using CellTiter-Glo^{™} cell viability assay kit. This kit uses luciferase as the detector. During the luminescence process, the luciferase requires the participation of ATP. CellTiter-Glo^{™} reagent was added to the cell culture medium, and the luminescence value was measured. The light signal is proportional to the amount of ATP present in the system, and ATP positively correlates with the number of viable cells present, thus the cell proliferation activity isi measured.

Cell culture and seeding: LOVO cells in logarithmic growth phase (human colon carcinoma cells) (ATCC CCL-229) were harvested and counted using a platelet counter. Trypan blue exclusion method was used to detect the cell viability to ensure that the cell viability was above 90%. 90 µL of cell suspension (RPMI1640 + 10% fetal bovine serum) was added to a 96-well transparent flat-bottom black-wall plate (Thermo, 165305) respectively, and the cell concentration was adjusted to 3000/well/90 µl. Cells in the 96-well plate were incubated overnight at 37°C, 5% CO₂ with 95% humidity (Thermo, Model 3100 Series).

Firstly, DMSO was used as the solvent to prepare 10mM mother solution of the test compounds, and then PBS was used to dilute 100 times to prepare a solution of 10 times of final concentration, the highest concentration was 100 µM. 10 µL solution of the test compounds was added to each well of the 96 well plate inoculated with cells, namely the solution was further diluted 10 times to reach the final concentration of 10 µM. Starting from 10 µM, the final concentration of the test compound was 3-fold serially diluted, with 9 concentrations in total with 3 triplicates for each concentration. The 96 well plates with the test compounds and cells were incubated at 37°C, 5% CO₂ and 95% humidity for 96 hours, and then CellTiter Glo analysis was performed.

CellTiter-Glo Reagents (CellTiter Glo ^{®} Luminescent Cell Viability Assay, Promega, G7572) were melted and the 96-well plates were equilibrated to room temperature for 30 minutes. An equal volume of CellTiter-Glo solution was added to each well, and the cells were lysed by shaking on the fixed orbit shaker for 5 minutes. The assay plates were left at room temperature for 20 minutes to stabilize the luminescence signal, and the luminescence signals were read using the SpectraMax multi-labeled microplate detector (MD, M3).

Data were analyzed using GraphPad Prism 7.0 software, fitted with non-linear S-curve regression to derive the dose-response curves, and IC₅₀ values were calculated therefrom. Cell survival rate (%) = (LumDrug to be tested - LumCulture medium control) / (LumCell control - Lum Culture medium control) × 100%

**Table 2 Results of cell proliferation experiment**

| **Compound No** | **LOVO IC₅₀(µM)** | **Compound No.** | **LOVO IC₅₀(µM)** |
|---|---|---|---|
| 1 | 0.304 | 16 | 0.310 |
| 2 | 0.479 | 19 | 0.620 |
| 3 | 0.067 | 20 | 0.039 |
| 4 | 0.140 | 26 | 1.268 |
| 5 | 0.384 | 29 | 0.488 |
| 6 | 0.097 | 31 | 0.158 |
| 8 | 0.243 | 32 | 0.156 |
| 9 | 0.592 | 34 | 0.424 |
| 11 | 2.642 | 35 | 0.522 |
| 14 | 0.419 | 36 | 0.148 |
| 15 | 0.186 | | |

### Activity Example 3: Metabolic stability of human and mouse liver microsomes

According to standard methods of in vitro metabolic stability studies in the art, eg., the methods described in Kerns, Edward H. and Di Li (2008). Drug-like Properties: Concepts, Structure Design and Methods: from ADME to Toxicity Optimization. San Diego: Academic Press; Di, Li et al., Optimization of a Higher Throughput Microsomal Stability Screening Assay for Profiling Drug Discovery Candidates, J. Biomol. Screen. 2003, 8(4), 453, the liver microsomal metabolic stability study of the compounds of the present disclosure was carried out analogously as follows.

| Species | Product information | Supplier | Abbreviation |
|---|---|---|---|
| Human | Cat No. 452117 | Corning | HLM |
| | Lot No. 38295 | | |
| CD-1 mice | Cat No. M1000 | Biopredic | MLM |
| | Lot No. 2010017 | | |

Liver microsome (protein concentration 0.56mg/mL) were added to 1 µM compound working solution (diluted to 100 µM with 100% acetonitrile from 10mM DMSO stock solution, organic phase content: 99% ACN, 1% DMSO), and after pre-incubation at 37°C for 10 min, the reaction was initiated by adding cofactor NADPH (prepared with magnesium chloride solution). After incubation for appropriate time (such as 5, 10, 20, 30 and 60 minutes), samples were collected, and an appropriate termination solution (glacial acetonitrile containing 200 ng/mL tolbutamide and 200 ng/mL labetalol (i.e. acetonitrile at 4°C)) was added to stop the reaction.

Sample treatment (n = 1): each appropriate sample was added, vortexed and centrifuged at high speed. The supernatants were collected, and detected the substrate by HPLC-MS/MS. The peak area at the 0 min was set as 100%. The peak areas at other time points were converted to percentages of the reamining amount. The natural logarithms of the percentage of remaining amount at each time point was plotted against the incubation time, and the slope (-k) was calculated. Then, Clint (µL/min/mg) and half-life (T1/2, min) of the compounds were calculated following the equation: Inherent clearance rate (Clint) = (k* Volume of incubation solution)/Mass of liver microsomes. The results are shown in Table 3.

**Table 3. Test results of metabolic stability of human and mouse liver microsomes**

| | Human liver microsomes | | | Mouse liver microsomes | | |
|---|---|---|---|---|---|---|
| Compound No. | T_{1/2} (min) | Remain % (T=60min) | CLint(mic) (µL/min/mg) | T _{1/2} (min) | Remain % (T=60min) | CLint(mic) (µL/min/mg) |
| 1 | 118.3 | 65.0 | 11.7 | 58.3 | 44.4 | 23.8 |
| 5 | >145 | 77.1 | <9.6 | 67.5 | 56.6 | 20.5 |
| 6 | >145 | 74.8 | <9.6 | 33.6 | 30.7 | 41.3 |
| BAY-1895344 | 109.3 | 71.5 | 12.7 | 25.1 | 17.0 | 55.1 |

### Activity Example 4: Pharmacokinetic (PK) assay of the compounds of the disclosure in mice

The PK of each compound was determined as follows: 6 CD-1 mice (obtained from Shanghai Lingchang Biotechnology Co., Ltd.) were divided into two groups, 3 mice in each group. One group was administered intravenously (IV) at a dose of 1 mg/kg, and the vehicle was 5%DMSO/95%(20%Captisol); one group was administered by oral gavage at a dose of 5 mg/kg, and the vehicle was 1%HPMC. Blood samples were collected from saphenous vein of lower leg in each group at 0, 0.083, 0.25, 0.5, 1, 2, 4, 6, 8 and 24 h after administration. Approximately 40 µL of the blood samples were collected in anticoagulant tubes containing EDTA-K2. Immediately after collection, the tubes were inverted at least 5 times to ensure adequate mixing, and then placed on ice. The blood samples collected at each time point were centrifuged at 4°C, 8000 rpm for 5 minutes to obtain plasma. Another 1.5 mL centrifuge tube was marked with the compound name, animal number, and time point, and the plasma was transferred to this tube. The plasma was stored at -80°C until analysis.

Compound concentrations in plasma were determined by UPLC-MS/MS method, and the pharmacokinetic parameters were calculated by Phoenix WinNolin 6.4 pharmacokinetic software on the obtained data.

The specific test results are as follows, showing that the compounds have good pharmacokinetic absorption and have the PK advantages.

**Table 4. In vivo PK results of Example compounds**

| Com. No. | IV dose 1mg/kg | | | | | Po dose 5mg/kg | | | |
|---|---|---|---|---|---|---|---|---|---|
| | AUC₀₋₂₄ₕ (ng/mL*hr) | C0 (ng/mL) | t_{1/2} (hr) | Vss (L/kg) | CL_obs (mL/min/kg) | AUC₀₋₂₄ₕ (ng/mL*hr) | Cmax (ng/mL) | t_{1/2} (hr) | F% |
| 1 | 925 | 823 | 1.51 | 1.68 | 18.3 | 3443 | 1082 | 2.57 | 75.6 |
| 3 | 1652 | 2865 | 7.56 | 1.03 | 10.0 | 6455 | 3153 | 3.99 | 77.5 |
| 5 | 2705 | 2179 | 3.45 | 0.75 | 6.15 | 9327 | 3020 | 2.58 | 69.0 |
| BAY-1895344 | 445 | 764 | 2.79 | 3.02 | 36.3 | 704 | 526 | 2.21 | 32.1 |

### Activity Example 5: Determination of equilibrium solubility of the compound of the disclosure in FaSSIF

According to the standard method for solubility determination familiar to those skilled in the art and conventional in the art, for example the method described in (Kerns, Edward H. and Di Li (2008) Drug-like Properties: Concepts, Structure Design and Methods: from ADME to Toxicity Optimization. San Diego: Academic Press), FaSSIF (pH6.5) (simulating intestinal fluids of a human in the state of hunger before meals) was used as the test system, and the solubility property of the compounds of the disclosure was investigated as follows.

The compound powder was weighed into a volumetric flask, 450 µL of pH 6.5 FaSSIF solution was added to obtain a supersaturated suspension, the samples were vortexed for at least 2 minutes, and the volumetric flasks were shaken on a vibrating screen at 800 rpm for 24 hours, and then centrifuged at 4000 rpm for 20 minutes. The compressed filtrate was added to a HPLC system, and the concentrations were calculated by a standard curve method. The results are shown in Table 5.

The composition of the fast simulated intestinal fluid FaSSIF is 0.056% (w/v) lecithin, 0.161% (w/v) sodium taurocholate, 0.39% (w/v) potassium phosphate, 0.77% (w/v) potassium chloride, deionized H₂O, pH 6.5.

**Table 5. Solubility in FaSSIF of the compounds of the disclosure**

| **Example** | **FaSSIF pH=6.5 (µg/mL)** |
|---|---|
| 1 | 1140.8 |
| 3 | 1144.7 |
| BAY-1895344 | 33.4 |

The above experimental results showed that, compared with the control, the Example compounds of the invention unexpectedly exhibited significantly higher solubility, thus having better druggability.

The structure of the control compound used in the above experiments is 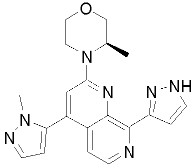 (purchased from Shanghai QianYan Technology Co., Ltd., CAS No. 1876467-74-1), or can be prepared and characterized according to the method of Example 111 in WO2016020320.

It will be appreciated by those skilled in the art that the foregoing descriptions are exemplary and explanatory in nature, and intended to illustrate the invention and its preferred embodiments. Through routine experimentation, an artisan will recognize apparent modifications and variations that may be made without departing from the spirit of the invention. All such modifications coming within the scope of the appended claims are intended to be included therein. Thus, the invention is intended to be defined not by the above description, but by the following claims and their equivalents.

All publications cited in this specification are herein incorporated by reference.

## Claims

1. A compound of formula (I), a pharmaceutically acceptable salt, or an isomer thereof: 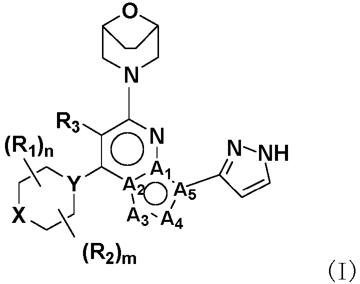 wherein
A₁, A₂ and A₅ are each independently C or N;
A₃ and A₄ are each independently CR₄, N or NR₅;
X is O, C(R₆)₂ or NR₇;
Y is N or CR₈;
R₁, R₂ and R₃ are each independently H, -OH, oxo, halogen, CN, -C₁₋₆ alkyl or -O-C₁₋₆ alkyl, wherein the -C₁₋₆ alkyl is optionally substituted with one or more halogen or hydroxyl; or R₁ and R₂ are linked together to form a C₁₋₃ alkylene bridge;
R₄ is H, oxo, halogen or -C₁₋₆ alkyl, wherein the -C₁₋₆ alkyl is optionally substituted with one or more halogen or hydroxyl;
R₅ is H or -C₁₋₆alkyl, wherein the -C₁₋₆alkyl is optionally substituted with one or more halogen; each R₆ is independently H, halogen, CN, -OH, -NH₂, -NH-C₁₋₆ alkyl, -N(C₁₋₆ alkyl)₂, -C₁₋₆ alkyl, -O-C₁₋₆ alkyl, -C(O)-C₁₋₆ alkyl, -C(O)-C₃₋₆ cycloalkyl, -SO₂-C₁₋₆ alkyl, -SO₂-C₃₋₆ cycloalkyl, -SO-C₁₋₆ alkyl, -SO-C₃₋₆ cycloalkyl, -C₆₋₁₀ aryl or -C₃₋₆ cycloalkyl, wherein the -C₁₋₆ alkyl, -C₆₋₁₀ aryl or -C₃₋₆ cycloalkyl is optionally substituted with one or more halogen, hydroxyl, -O-C₁₋₆ alkyl, -C₁₋₆ alkyl, or -C₁₋₆ alkyl substituted with halogen or hydroxyl;
R₇ is H, -C₁₋₆ alkyl, -C(O)-C₁₋₆ alkyl, -C(O)-C₃₋₆ cycloalkyl, -SO₂-C₁₋₆ alkyl, - SO₂-C₃₋₆ cycloalkyl, -SO-C₁₋₆ alkyl or -SO-C₃₋₆ cycloalkyl, wherein the -C₁₋₆ alkyl or -C₃₋₆ cycloalkyl is optionally substituted with one or more halogen, hydroxyl, -O-C₁₋₆ alkyl, -C₁₋₆ alkyl, or -C₁₋₆ alkyl substituted with halogen or hydroxyl;
R₈ is H, -OH or halogen;
n and m are each independently an integer of 0 to 4.

2. The compound of formula (I), a pharmaceutically acceptable salt, or an isomer thereof according to claim 1, wherein at least two of A₁, A₂, A₃, A₄ and A₅ are N or NRs, and the others are C or CR₄; preferably two of them are N or NR₅, and the others are C or CR₄.

3. The compound of formula (I), a pharmaceutically acceptable salt, or an isomer thereof according to claim 1 or 2, wherein the heteroaryl moiety containing A₁~A₅ and consisting of a six membered ring fused to a five membered ring has a structure selected from: 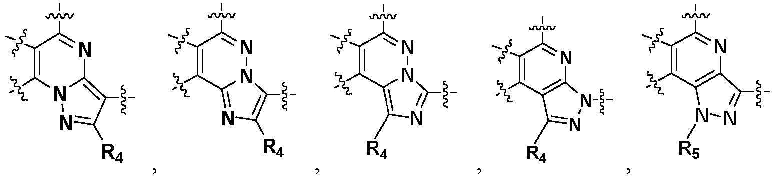 and 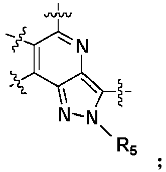 preferably 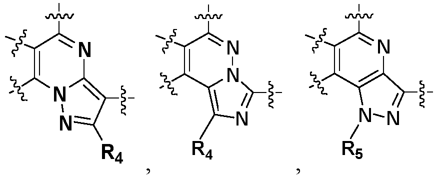 or

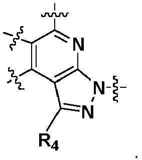

4. The compound of formula (I), a pharmaceutically acceptable salt, or an isomer thereof according to any one of claims 1-3, wherein R₄ is H, R₅ is H or -C₁₋₆ alkyl.

5. The compound of formula (I), a pharmaceutically acceptable salt, or an isomer thereof according to any one of claims 1-4, wherein the six-membered ring comprising X and Y 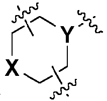 is selected from

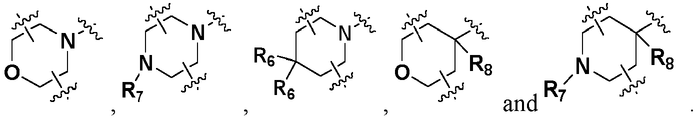

6. The compound of formula (I), a pharmaceutically acceptable salt, or an isomer thereof according to any one of claims 1-4, wherein X is selected from -O-, -NH-, -N(C₁₋₆ alkyl)-, -CH₂-, - C(halogen)₂.

7. The compound of formula (I), a pharmaceutically acceptable salt, or an isomer thereof according to anyone of claims 1-6, wherein Y is N or CRs, wherein R₈ is OH.

8. The compound of formula (I), a pharmaceutically acceptable salt, or an isomer thereof according to any one of claims 1-7, wherein
one of m and n is 0, the other is 1, R₁ or R₂ is each independently C₁₋₆ alkyl optionally substituted with one or more halogen, connected to an ortho position of Y or an ortho position of X; or
one of m and n is 0, the other is 2, R₁ or R₂ is each independently C₁₋₆ alkyl optionally substituted with one or more halogen, connected to an ortho position of Y, an ortho position of X or connected to an ortho-position of Y and an ortho-position of X respectively; or
m and n are both 1, R₁ and R₂ are each independently C₁₋₆ alkyl optionally substituted with one or more halogen, both are connected to ortho positions of Y, or both connected to ortho positions of X, or connected to an ortho position of Y and an ortho position of X respectively;
preferably one of m and n is 0, the other is 1, R₁ or R₂ is C₁₋₆ alkyl, and connected to an ortho position of Y or an ortho position of X.

9. The compound of formula (I), a pharmaceutically acceptable salt, or an isomer thereof according to any one of claims 1-7, wherein m=1 and n=1, R₁ and R₂ are connected to ortho-positions of Y respectively and form together a C₁₋₃ alkylene bridge, preferably a C₂ alkylene bridge; or R₁ and R₂ are connected to ortho-positions of X respectively and form together a C₁₋₃ alkylene bridge, preferably a C₂ alkylene bridge.

10. The compound of formula (I), a pharmaceutically acceptable salt, or an isomer thereof according to any one of claims 1-9, wherein R₃ is H.

11. The compound of formula (I), a pharmaceutically acceptable salt, or an isomer thereof according to claim 1, wherein the heteroaryl moiety containing A₁~A₅ and consisting of a six membered ring fused to a five membered ring is selected from: 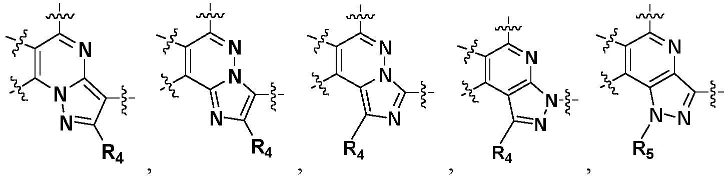 and 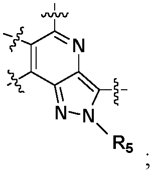
the six-membered ring comprising X and Y is selected from 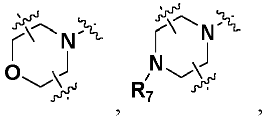 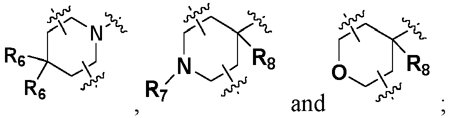
R₁ and R₂ are each independently H or -C₁₋₆ alkyl, wherein the -C₁₋₆ alkyl is optionally substituted with one or more halogen; or R₁ and R₂ are connected to form a C₁₋₃ alkylene bridge;
R₃ is H or halogen;
R₄ is H;
R₅ is H or -C₁₋₆ alkyl;
each R₆ is independently H or halogen, -C₁₋₆ alkyl or -O-C₁₋₆ alkyl, wherein the -C₁₋₆ alkyl is optionally substituted with one or more halogen;
R₇ is H or -C₁₋₆ alkyl, wherein the -C₁₋₆ alkyl is optionally substituted with one or more halogen;
R₈ is H, -OH or halogen;
n and m are each independently an integer of 0 to 2.

12. The compound of formula (I), a pharmaceutically acceptable salt, or an isomer thereof according to claim 11, wherein the heteroaryl moiety containing A₁~A₅ and consisting of a six membered ring fused to a five membered ring is selected from

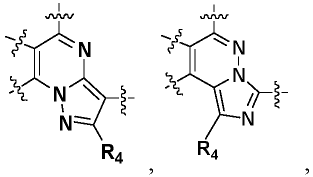

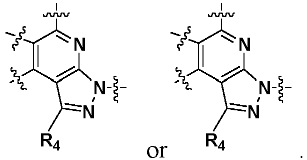

13. The compound of formula (I), a pharmaceutically acceptable salt, or an isomer thereof according to claims 11 or 12, wherein the six-membered ring comprising X and Y 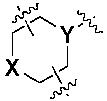 is

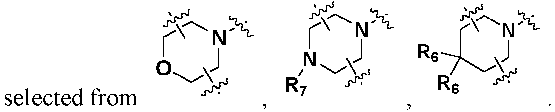

14. The compound of formula (I), a pharmaceutically acceptable salt, or an isomer thereof according to any one of claims 11-13, wherein R₁ and R₂ are each independently H or -C₁₋₆ alkyl.

15. The compound of formula (I), a pharmaceutically acceptable salt, or an isomer thereof according to any one of claims 11-14, wherein one of n and m is 0, the other is 1, R₁ or R₂ is connected to an ortho position of Y or an ortho position of X on the ring; or one of n and m is 0, the other is 2, R₁ or R₂ are simultaneously connected to an ortho-position of Y, an ortho-position of X, or connected to an ortho-position of Y and an ortho-position of X respectively, preferably simultaneously connected to an ortho-position of Y; or n and m are both 1, R₁ and R₂ are each independently connected to an ortho position of Y, or an ortho position of X, preferably both are connected to ortho-positions of X; or R₁ and R₂ simultaneously connected to ortho positions of Y or ortho positions of X are linked together to form a C₁₋₃ alkylene bridge, preferably a C₂ alkylene bridge.

16. The compound of formula (I), a pharmaceutically acceptable salt, or an isomer thereof according to any one of claims 11-15, wherein R₃ is H.

17. The compound of formula (I), a pharmaceutically acceptable salt, or an isomer thereof according to any one of claims 11-16, wherein R₅ is -C₁₋₆ alkyl, preferably -CH₃.

18. The compound of formula (I), a pharmaceutically acceptable salt, or an isomer thereof according to any one of claims 11-16, wherein each R₆ is independently H or halogen, preferably H or F.

19. The compound of formula (I), a pharmaceutically acceptable salt, or an isomer thereof thereof according to any one of claims 11-16, wherein R₇ is H or -C₁₋₆ alkyl, preferably -CH₃.

20. A compound, a pharmaceutically acceptable salt, or an isomer thereof, selected from

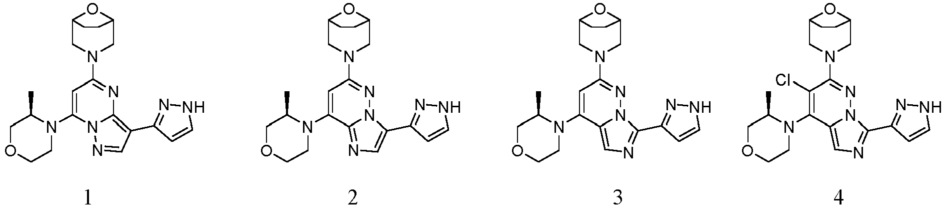

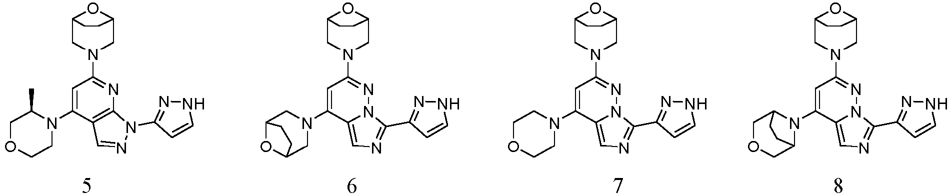

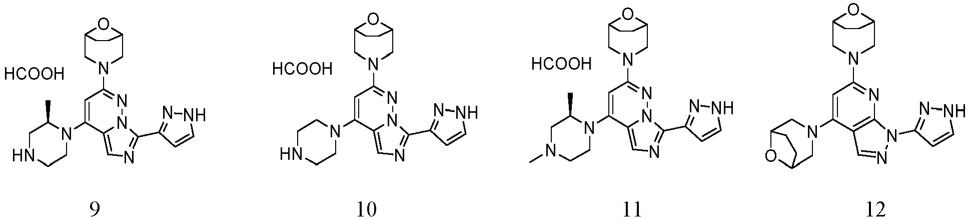

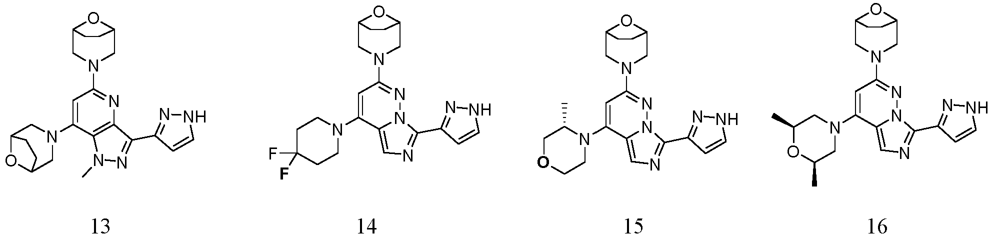

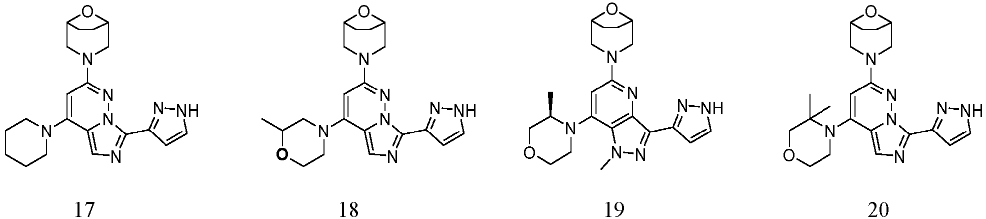

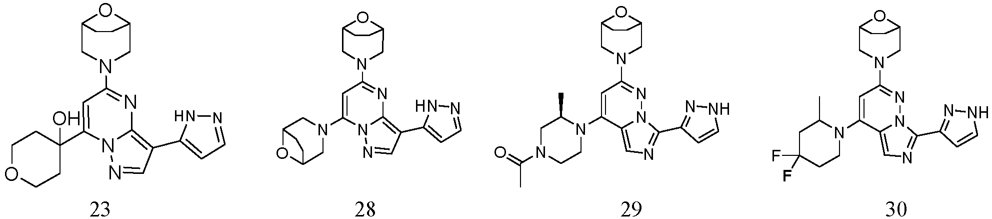

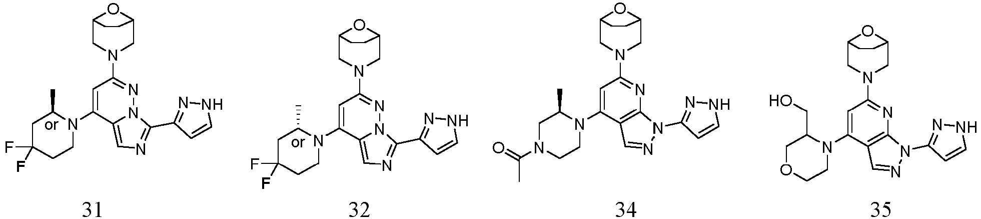

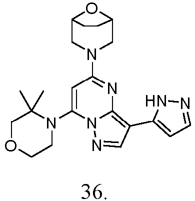

21. A pharmaceutical composition comprising a compound according to any one of claims 1 to 20, a pharmaceutically acceptable salt or an isomer thereof, and one or more pharmaceutically acceptable excipients.

22. The pharmaceutical composition according to claim 21, further comprising at least another active pharmaceutical ingredient.

23. Use of the compound, a pharmaceutically acceptable salt or an isomer thereof according to any one of claims 1 to 20 or the pharmaceutical composition according to any one of claims 21-22, in the prevention or treatment of ATR kinase-associated diseases.

24. Use of the compound, a pharmaceutically acceptable salt or an isomer thereof according to any one of claims 1 to 20 or the pharmaceutical composition according to any one of claims 21-22, in the manufacture of a medicament for the prevention or treatment of ATR kinase-associated diseases.

25. The use according to claim 23 or 24, wherein the ATR kinase-associated diseases are selected from the group consisting of hematological malignancies, e.g. leukemia (including chronic lymphocytic leukemia, acute lymphocytic leukemia, acute myelogenous leukemia, and chronic myelogenous leukemia), multiple myeloma, lymphoid malignancies (e.g. lymphoma, Hodgkin's disease, non-Hodgkin's lymphoma), myelodysplastic syndromes, and solid tumors such as carcinomas and sarcomas and their metastases, e.g. breast cancer, lung cancer (non-small cell lung cancer, small cell lung cancer, squamous cell carcinoma, bronchioloalveolar carcinoma), central nervous system tumor (e.g. glioma, dysembryonic dysplastic neuroepithelial tumor, glioblastoma multiforme, mixed Glioma, medulloblastoma, retinoblastoma, neuroblastoma, germ cell tumor and teratoma), gastrointestinal cancer (e.g. gastric cancer, esophageal cancer, liver cancer, bile duct cancer, colorectal cancer, carcinoma of small intestine, pancreatic cancer), skin cancer, melanoma, thyroid cancer, bone cancer, head and neck cancer, salivary gland cancer, prostate cancer, testicular cancer, ovarian cancer, cervical cancer, uterine cancer, endometrial cancer, vulvar cancer, bladder cancer, renal cancer, squamous cell carcinoma, sarcomas (e.g. osteosarcoma, chondrosarcoma, leiomyosarcoma, soft tissue sarcoma, Ewing's sarcoma, gastrointestinal tissue carcinoma, gastrointestinal stromal tumor, Kaposi's sarcoma), and pediatric cancer (e.g. rhabdomyosarcoma and neuroblastoma).

26. The use according to claim 25, wherein the ATR kinase-associated diseases are selected from the group consisting of lung cancer, prostate cancer, melanoma, ovarian cancer, breast cancer, endometrial cancer, renal cancer, gastric cancer, sarcoma, head and neck cancer, central nervous system tumors and their metastases, and acute myelogenous leukemia.
